# EUROPEAN PATENT APPLICATION

(11) **EP 4 394 383 A1**
(43) Date of publication of application: **03.07.2024**
(21) Application number: 22861472.3
(22) Date of filing: 26.08.2022
(51) Int. Cl.: G01N 33/543

(54) **CORE-SHELL TEMPLATE MOLECULE/PARTICLE FOR TECHNIQUE FOR HIGH-YIELD FORMATION OF PORES ON SUBSTRATE**

(30) Priority: 27.08.2021 JP 2021139397
(71) Applicant: National University Corporation Kobe University, Kobe-shi, Hyogo 657-8501 (JP)
(72) Inventor: TAKEUCHI, Toshifumi, Kobe-shi, Hyogo 657-8501 (JP); SUNAYAMA, Hirobumi, Kobe-shi, Hyogo 657-8501 (JP); TAKANO, Eri, Kobe-shi, Hyogo 657-8501 (JP); HORIKAWA, Ryo, Kobe-shi, Hyogo 657-8501 (JP)
(74) Representative: Weickmann & Weickmann PartmbB
(86) International application number: PCT/JP2022/032270
(87) International publication number: WO 2023/027184

(57) **Abstract**

There are provided a sensor for analysis that can easily and sensitively perform analysis and inspection, a substrate for producing a sensor for analysis, a particle used for producing the substrate, and related technologies. The present disclosure provides a particle including a particle core and a modifier integrated with the particle core by an intermolecular chemical bonding that is separable under conditions that do not damage the particle core, or a particle for producing a substrate for producing a sensor for analysis, in which the particle includes a particle core and a modifier integrated with the core by an intermolecular chemical bonding that is separable under conditions that do not damage the substrate.

## Description

### Technical Field

The present disclosure relates to a technique for detecting an object to be detected on a substrate with high sensitivity. More specifically, the present disclosure relates to a core-shell template molecule/particle in a measurement technique and an application thereof, a substrate for producing a sensor for analysis of an object to be detected and a method for producing the same, a sensor for analysis of an object to be detected and a method for producing the same, and a method for analyzing an object to be detected.

### Background Art

Small extracellular vesicles (sEVs) such as an exosome are one of vesicles released from a cell, and are lipid bilayer membrane vesicles having a diameter of 20 to 200 nm. The small extracellular vesicles contain proteins and nucleic acids such as miRNA and mRNA inside them, and also have proteins on surfaces thereof. Since the small extracellular vesicles are characterized by such substances, it is considered that it is possible to infer what kind of cell the secreted cell is by analyzing the characteristics of the small extracellular vesicles. In addition, small extracellular vesicles have been confirmed to exist in various body fluids, and can be collected relatively easily.

Small extracellular vesicles secreted from cancer cells contain substances derived from tumors. Therefore, it is expected that diagnosis of cancer can be performed by analyzing substances contained in small extracellular vesicles in a body fluid. Furthermore, since small extracellular vesicles are actively secreted by cells, it is expected that the small extracellular vesicles exhibit some characteristics even at an early stage of cancer.

### Summary of Invention

### Solution to Problem

As a result of intensive studies, the present inventors have developed and completed a sensor for analysis that can easily and sensitively perform analysis and inspection, a substrate for producing a sensor for analysis, a particle used for producing the substrate, and related technologies.

The present disclosure provides, for example, the following items.

### (Item A1)

A particle including:
a particle core; and
a modifier integrated with the particle core by an interaction that is separable under conditions that do not damage the particle core.

### (Item A1A)

A particle including:
a particle core; and
a modifier integrated with the particle core by an intermolecular chemical bonding that is separable under conditions that do not damage the particle core.

### (ItemA1B)

A particle for producing a substrate for producing a sensor for analysis, the particle including:
a particle core, and a modifier integrated with the core by an interaction that is separable under conditions that do not damage the substrate.

### (Item A1B-2)

A particle for producing a substrate for producing a sensor for analysis, the particle including:
a particle core, and a modifier integrated with the core by an intermolecular chemical bonding that is under conditions that do not damage the substrate.

### (Item A1C)

The particle according to any one of the preceding items, in which the modifier is not a monomer covalently bonded to the core.

### (Item A1D)

The particle according to any one of the preceding items, in which the modifier includes one or a plurality of modifiers.

### (Item A2)

The particle according to any one of the preceding items, in which the modifier contains a substituent capable of binding to a substrate.

### (Item A3)

The particle according to any one of the preceding items, in which the modifier contains at least one group selected from the group consisting of a reversible linking group and a polymerizable functional group.

### (Item A3-1)

The particle according to any one of the preceding items, in which the modifier contains a polymerizable functional group.

### (Item A3A)

The particle according to any one of the preceding items, in which the modifier includes at least one selected from the group consisting of a group for binding a specific binding molecule to a target substance, a group for binding a signal substance, a signal substance, and a reversible linking group.

### (ItemA3A-1)

The particle according to any one of the preceding items, in which the modifier contains a group for binding a specific binding molecule to a target substance and a group for binding a signal substance; a group for binding a specific binding molecule to a target substance and a signal substance; or a reversible linking group.

### (Item A4A)

The particle according to any one of the preceding items, in which the reversible linking group is a disulfide group, an imino binding group, an oxime group, a hydrazone binding group, a boronic acid cyclic ester bond, a carboxylic acid ester group, a carboxylic acid thioester group, or an avidin-biotin bond.

### (Item A4B)

The particle according to any one of the preceding items, in which the group for binding a specific binding molecule to a target substance is an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group.

### (Item A4C)

The particle according to any one of the preceding items, in which the group for binding a signal substance is an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group.

### (Item A4D)

The particle according to any one of the preceding items, in which the signal substance is a fluorescent molecule, a radioactive element-containing substance, a magnetic substance, or an enzyme.

### (Item A4)

The particle according to any one of the preceding items, in which the interaction that is separable under conditions that do not damage the particle core is a non-covalent bonding.

### (Item A4A)

The particle according to any one of the preceding items, in which the intermolecular chemical bonding that is separable under conditions that do not damage the particle core or particle core core is a non-covalent bonding.

### (Item A5)

The particle according to any one of the preceding items, in which the interaction is an electrostatic interaction.

### (Item A5A)

The particle according to any one of the preceding items, in which the intermolecular chemical bonding is an electrostatic interaction.

### (Item A6)

The particle according to any one of the preceding items, in which the particle core is formed of silica, polystyrene, polylactic acid, polymethyl methacrylate, a polylactic acid/glycolic acid copolymer, chitosan, iron oxide, gold, silver, platinum, aluminum oxide, copper oxide, titanium oxide, zinc oxide, zirconium oxide, cerium oxide, cobalt oxide, tin-doped indium oxide (ITO), antimony-doped tin oxide (ATO), graphene, graphene oxide, a carbon nanotube, nanodiamond, or hydroxyapatite, or any combination thereof.

### (Item A7)

The particle according to any one of the preceding items, in which the particle core is formed of silica or polystyrene.
<B) Template particle clinging to polymer for producing substrate for producing sensor for analysis = use>

### (Item B 1)

A kit for producing a substrate for producing a sensor for analysis, the kit including:
the particle according to any one of the preceding items;
a substrate; and
a raw material of a polymer matrix, if necessary.

### (Item B1A)

A kit for producing a substrate for producing a sensor for analysis, the kit including:
a substrate; and
a particle including a particle core and a modifier integrated with the core by an interaction that is separable under conditions that do not damage the substrate.

### (Item B1B)

A kit for producing a substrate for producing a sensor for analysis, the kit including:
a substrate; and
a particle including a particle core and a modifier integrated with the core by an intermolecular chemical bonding that is separable under conditions that do not damage the substrate.

### (Item B 1)

A kit for producing a substrate for producing a sensor for analysis, the kit including:
a substrate;
a particle including a particle core and a modifier integrated with the core by an interaction that is separable under conditions that do not damage the substrate; and
a raw material of a polymer matrix, if necessary.

### (Item B1C)

A kit for producing a substrate for producing a sensor for analysis, the kit including:
a substrate;
a particle including a particle core and a modifier integrated with the core by an intermolecular chemical bonding that is separable under conditions that do not damage the substrate; and
a raw material of a polymer matrix, if necessary.

### (Item B2)

Use of the particles according to any one of the preceding items for producing a substrate for producing a sensor for analysis.

### (Item B3)

A method for producing the particles according to any one of the preceding items, the method including:
A) a step of providing a particle core;
B) a step of providing a modifier capable of being integrated with the core by an interaction that is separable under conditions that do not damage a substrate for producing a sensor for analysis; and
C) a step of subjecting the particle core and the modifier to a condition under which the particle core and the modifier are integrated by an interaction that is separable under conditions that do not damage the substrate.

### (Item B3A)

A method for producing the particles according to any one of the preceding items, the method including:
A) a step of providing a particle core;
B) a step of providing a modifier capable of being integrated with the core by an intermolecular chemical bonding that is separable under conditions that do not damage a substrate for producing a sensor for analysis; and
C) a step of subjecting the particle core and the modifier to a condition under which the particle core and the modifier are integrated by an intermolecular chemical bonding that is separable under conditions that do not damage the substrate.

### <C) Method for producing measurement substrate using A).>

### (Item C1)

A method for producing a substrate for producing a sensor for analysis, the method including:
A) a step of providing the particles according to any one of the preceding items;
B) a step of disposing the particles on a substrate;
C) a step of providing a raw material of a polymer matrix to the substrate on which the particles are immobilized, if necessary;
D) a step of forming a substrate on which the polymer matrix is disposed by subjecting the substrate to a condition under which the polymer matrix is polymerized, if necessary; and
E) a step of forming a scaffold by subjecting the substrate to a condition under which the particles are dissociated from the substrate.

### (Item C2)

A method for producing a sensor for analysis, the method including:
A) a step of providing the particles according to any one of the preceding items;
B) a step of disposing the particles on a substrate;
C) a step of providing a raw material of a polymer matrix to the substrate on which the particles are disposed, if necessary;
D) a step of forming a substrate on which the polymer matrix is disposed by subjecting the substrate to a condition under which the polymer matrix is polymerized, if necessary;
E) a step of forming a scaffold by subjecting the substrate to a condition under which the particles are dissociated from the substrate; and
F) a step of binding a substance required for measurement to the scaffold.

### (Item C3)

The method according to any one of the preceding items, in which in the step E, the condition under which the particles are dissociated from the substrate is a condition under which a reversible linking group is cleaved and a reversible binding group is produced.

### (Item C4)

The method according to any one of the preceding items, further including, after the step E, a step of converting the reversible binding group in the scaffold into a group for binding a specific binding molecule to a target substance or a group for binding a signal substance.

### <D) Substrate formed of C) and sensor>

### (Item DX)

A substrate for producing a sensor for analysis produced by any one of the preceding items.

### (Item DX2)

A sensor for analysis produced by any one of the preceding items.

### (Item D 1)

A substrate for producing a sensor for analysis, the substrate including:
A) a substrate;
B) a polymer matrix disposed on the substrate, the polymer matrix having a scaffold that at least partially fits into a molecule to be detected, if necessary; and
C) a binding group disposed on the scaffold.

### (Item D2)

A sensor for analysis including:
A) a substrate;
B) a polymer matrix disposed on the substrate, the polymer matrix having a scaffold that at least partially fits into a molecule to be detected, if necessary;
C) a group for binding a signal substance or a signal substance disposed on the scaffold; and
D) a group for binding a specific binding molecule that binds to a molecule to be detected.

### (Item D3)

The substrate or sensor according to any one of the preceding items, in which a cleanliness of the polymer matrix is 95% or more.

### (Item D4)

The substrate or sensor according to any one of the preceding items, in which a standard value of the scaffold in the substrate is 80% or more.

### (Item D5)

The substrate or sensor according to any one of the preceding items, in which a substituent derived from one or a plurality of modifiers integrated with the core by an interaction that is separable under conditions that do not damage the substrate is bonded onto the substrate.

### (Item D5)

The substrate or sensor according to any one of the preceding items, in which a substituent derived from one or a plurality of modifiers integrated with the core by an intermolecular chemical bonding that is separable under conditions that do not damage the substrate is bonded onto the substrate.

### (Item D5-1)

The substrate or sensor according to any one of the preceding items, in which the substituent derived from a modifier is an amino group, an acidic group, a coordinate binding group, a carbonyl group, an aldehyde group, a ketone group, a boronyl group, a cis-diol group, a thiol group, a biotin group, a desthiobiotin group, a peptide ligand, or a reversible linking group.

### (Item D5A)

The substrate or sensor according to any one of the preceding items, in which the amino group is a primary to quaternary amino group, an aliphatic amino group, an aromatic amino group, a hydrazide, a guanidine, or an amidine.

### (Item D5B)

The substrate or sensor according to any one of the preceding items, in which the acidic group is a carboxy group, a sulfonic acid group, or a phosphate group.

### (Item D5C)

The substrate or sensor of any one of the preceding items, in which the coordinate binding group is an NTA group or a His-tag.

### (Item D5D)

The substrate or sensor according to any one of the preceding items, in which the peptide ligand is a glutathione group or an RGD group.

### (Item D5E)

The substrate or sensor according to any one of the preceding items, in which the reversible linking group is a disulfide, pyridyl disulfide, imine, oxime, hydrazone, boronic acid cyclic ester, carboxylic acid ester, carboxylic acid thioester, or silyl group.

### (Item D6)

A sensor for analysis including:
A) a substrate;
B) a polymer matrix disposed on the substrate, the polymer matrix having a scaffold that at least partially fits into a molecule to be detected, if necessary;
C) a substituent derived from a modifier integrated with the core by an interaction that is separable under conditions that do not damage the substrate, the modifier being disposed on the scaffold;
D) a group for binding a signal substance or a signal substance disposed on the scaffold; and
E) a group for binding a specific binding molecule that binds to a molecule to be detected.

### (Item D6A)

A sensor for analysis including:
A) a substrate;
B) a polymer matrix disposed on the substrate, the polymer matrix having a scaffold that at least partially fits into a molecule to be detected, if necessary;
C) a substituent derived from a modifier integrated with the core by an intermolecular chemical bonding that is separable under conditions that do not damage the substrate, the modifier being disposed on the scaffold;
D) a group for binding a signal substance or a signal substance disposed on the scaffold; and
E) a group for binding a specific binding molecule that binds to a molecule to be detected.

### <E) Direct substrate>

### (Item E1)

A substrate for producing a sensor for direct analysis, including:
A) a substrate;
B) the particles according to any one of the preceding items;
C) a polymer matrix on which the particles are disposed, the polymer matrix being disposed on the substrate, if necessary; and
D) a blocking agent present on the outermost layer, if necessary.

### (Item E2)

A sensor for direct analysis including:
A) a substrate;
B) the particles according to any one of the preceding items;
C) a polymer matrix on which the particles are disposed, the polymer matrix being disposed on the substrate, if necessary; and
D) a group for binding a specific binding molecule and a group for binding a signal substance or a signal substance present on the particle, if necessary.

### (Item E3)

A method for producing a substrate for producing a sensor for direct analysis, the method including:
A) a step of providing the particles according to any one of the preceding items;
B) a step of disposing the particles on a substrate;
C) a step of providing a raw material of a polymer matrix to the substrate on which the particles are immobilized, if necessary; and
D) a step of forming a substrate on which the polymer matrix is disposed by subjecting the substrate to a condition under which the polymer matrix is polymerized, if necessary.

### (Item E4)

A method for producing a sensor for direct analysis, the method including:
A) a step of providing the particles according to any one of the preceding items;
B) a step of disposing the particles on a substrate;
C) a step of providing a raw material of a polymer matrix to the substrate on which the particles are immobilized, if necessary;
D) a step of forming a substrate on which the polymer matrix is disposed by subjecting the substrate to a condition under which the polymer matrix is polymerized, if necessary; and
F) a step of binding a substance required for measurement to the particles, if necessary.

### (Item 1)

A particle (C) including:
(A) a particle core; and
(B) a modifier,

in which the particle core includes (A1) a particle core core and (A2) a functional group-containing moiety present on the particle core,
the modifier includes (B-1) a first functional group capable of binding to the particle core and capable of forming a chemical bond between molecules, and (B-2) a second functional group that imparts properties desired in the sensor for analysis, where the functional group of (B-1) and the binding group of (B-2) are the same as or different from each other, and
the particle core and the modifier are integrated with the particle core by a chemical bonding between the molecules of the functional group-containing moiety and the first functional group.

### (Item 1A)

The particle according to any one of the preceding items, in which the (A1) particle core core is an inorganic material or an organic material.

### (Item 1AA)

The particle according to any one of the preceding items, in which the (A1) particle core core is a metal (gold, silver, platinum, tin-doped indium oxide (ITO), antimony-doped tin oxide (ATO), or the like), an oxide of a metal (iron oxide, aluminum oxide, copper oxide, titanium oxide, zinc oxide, zirconium oxide, cerium oxide, cobalt oxide, or the like), graphene, graphene oxide, a carbon nanotube, nanodiamond, a nitride, a fluoride, a sulfide, a boride, and a complex compound thereof, hydroxyapatite, silicon dioxide (silica), cured latex, dextran, chitosan, polylactic acid, poly(meth)acrylic acid, polymethyl methacrylate, a poly(lactic-co-glycolic acid) copolymer (PLGA), polystyrene, or polyethyleneimine.

### (Item 1B)

The particle according to any one of the preceding items, in which the (A2) functional group-containing moiety is an acidic group, a basic group, an aromatic group, an alkyl group, a polyhistidine (His) tag, a metal complex, biotin, an electron-deficient unsaturated carbon group such as a maleimide group or an acrylic acid ester group, an iodoacetamide group, a thiol group, a pyridyl disulfide group, an alkene (vinyl sulfone)/alkyne group for radical addition based click chemistry, a thioester group for a native chemical ligation method, an amino group, a carbonyl group, an aldehyde group, a hydroxyl group, a phenolic hydroxyl group, a carboxylic acid active ester group, a boronyl group, a cis-diol group, (sugar group), a (thio)lactone group, an alkyne group or a derivative thereof, or an azide group.

### (Item 1C)

The particle according to any one of the preceding items, in which the (B-1) the first functional group is a basic group, an acidic group, an aromatic group, an alkyl group, a polyhistidine (His) tag, a metal complex, biotin, an electron-deficient unsaturated carbon group such as a maleimide group or an acrylic acid ester group, an iodoacetamide group, a thiol group, a pyridyl disulfide group, an alkene (vinyl sulfone)/alkyne group for radical addition based click chemistry, a thioester group for a native chemical ligation method, an amino group, a carbonyl group, an aldehyde group, a hydroxyl group, a phenolic hydroxyl group, a carboxylic acid active ester group, a boronyl group, a cis-diol group, (sugar group), a (thio)lactone group, an alkyne group or a derivative thereof, or an azide group.

### (Item 1D)

The particle according to Item 1, in which the (B-2) second functional group is an amino group, a carboxy group, a carboxylic acid active ester group, a metal complex (Ni-NTA group or the like), a thiol group, a pyridyl disulfide group, a disulfide group, a maleimide group, a halogenated acetamide group, an azide group, an alkyne group (or a derivative group thereof), biotin (or a structural analog thereof), avidin (Avidin, Neutravidin, or Streptavidin), a cis-diol group, a boronyl group, or a (thio)lactone group.

### (Item 1E)

The particle according to any one of the preceding items, in which the (A2) functional group-containing moiety and the (B-1) first functional group are a combination in which left and right in the following Table are the same as each other.

**[Table 1A-1]**

| **A-2 (functional group on particle core)** | **B-1** |
|---|---|
| Acidic group (carboxy group, sulfo group, phosphate group, silanol group, or the like) | Basic group (primary amino group, secondary amino group, tertiary amino group, quaternary ammonium group, pyridyl group, guanidine group, amidino group, imidazole group, aminooxy group, or the like) |
| Basic group (primary amino group, secondary amino group, tertiary amino group, quaternary ammonium group, pyridyl group, guanidine group, amidino group, imidazole group, aminooxy group, or the like) | Acidic group (carboxy group, sulfo group, phosphate group, silanol group, or the like) |
| Aromatic group (substituted or unsubstituted aryl group or arylene group, or the like) | Aromatic group (substituted or unsubstituted aryl group or arylene group, or the like) |
| Alkyl group (C = 6 or higher linear or branched structure) | Alkyl group (C = 6 or higher linear or branched structure) |
| Polyhistidine (His) tag (6 × His, 8 × His, 10 × His, or the like) | Metal complex (Ni-NTA (nickel-nitrilotriacetic acid-derived group) or the like) |
| Metal complex (Ni-NTA (nickel-nitrilotriacetic acid-derived group) or the like) | Polyhistidine (His) tag (6 × His, 8 × His, 10 × His, or the like) |
| Biotin (biotin, desthiobiotin, and iminobiotin derivative) | Avidin (avidin, Neutravidin, Streptavidin) |
| Avidin (avidin, Neutravidin, Streptavidin) | Biotin (biotin, desthiobiotin, and iminobiotin derivative) |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Amino group (primary amino group, secondary amino group) | Maleimide group |
| Maleimide group | Amino group (primary amino group, secondary amino group) |
| Amino group (primary amino group, secondary amino group, aminooxy group) | Carbonyl group/aldehyde group |
| Carbonyl group/aldehyde group | Amino group (primary amino group, secondary amino group, aminooxy group) |
| Amino group (primary amino group, secondary amino group) hydroxyl group, phenolic hydroxyl group, thiol group | Carboxylic acid active ester group |
| Carboxylic acid active ester group | Amino group (primary amino group, secondary amino group) hydroxyl group, phenolic hydroxyl group, thiol group |
| Boronyl group | cis-diol group (sugar group) |
| cis-diol group (sugar group) | Boronyl group |
| Amino group | (Thio)lactone group |
| (Thio)lactone group | Amino group |
| Alkyne group and derivative thereof | Azide group |
| Azide group | Alkyne group and derivative thereof |

### (Item 1F)

The particle according to any one of the preceding items, in which the (A2) functional group-containing moiety and the (B-1) first functional group are a combination in which left and right in the following Table are the same as each other.

**[Table 1A-2]**

| **A-2 (functional group on particle core)** | **B-1** |
|---|---|
| Acidic group | Basic group |
| Basic group | Acidic group |
| Aromatic group | Aromatic group |
| Alkyl group | Alkyl group |
| Polyhistidine (His) tag | Metal complex |
| Metal complex | Polyhistidine (His) tag |
| Biotin | Avidin |
| Avidin | Biotin |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Amino group | Maleimide group |
| Maleimide group | Amino group |
| Amino group | Carbonyl group/aldehyde group |
| Carbonyl group/aldehyde group | Amino group |
| Amino group, hydroxyl group, phenolic hydroxyl group, thiol group | Carboxylic acid active ester group |
| Carboxylic acid active ester group | Amino group, hydroxyl group, phenolic hydroxyl group, thiol group |
| Boronyl group | cis-diol group (sugar group) |
| cis-diol group (sugar group) | Boronyl group |
| Amino group | (Thio)lactone group |
| (Thio)lactone group | Amino group |
| Alkyne group and derivative thereof | Azide group |
| Azide group | Alkyne group and derivative thereof |

### (Item 1G)

The particle according to any one of the preceding items, in which the (A2) functional group-containing moiety and the (B-1) first functional group are a combination in which left and right in the following Table are the same as each other.

**[Table 1A-3]**

| **A-2 (functional group on particle core)** | **B-1** |
|---|---|
| Carboxy group, sulfo group, phosphate group, silanol group | Primary amino group, secondary amino group, tertiary amino group, quaternary ammonium group, pyridyl group, guanidine group, amidino group, imidazole group, amino oxy group |
| Primary amino group, secondary amino group, tertiary amino group, quaternary ammonium group, pyridyl group, guanidine group, amidino group, imidazole group, aminooxy group | Carboxy group, sulfo group, phosphate group, silanol group |
| Aromatic group (substituted or unsubstituted aryl group or arylene group, or the like) | Substituted or unsubstituted aryl group or arylene group |
| Alkyl group (C = 6 or higher linear or branched structure) | Alkyl group (C = 6 or higher linear or branched structure) |
| 6 × His, 8 × His, 10 × His | Ni-NTA (nickel-nitrilotriacetic acid-derived group) |
| Ni-NTA (nickel-nitrilotriacetic acid-derived group) | 6 × His, 8 × His, 10 × His, or the like |
| Biotin, desthiobiotin, and iminobiotin derivative | Avidin, Neutravidin, Streptavidin |
| Avidin, Neutravidin, Streptavidin | Biotin, desthiobiotin, and iminoblotin derivative |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Primary amino group, secondary amino group | Maleimide group |
| Maleimide group | Primary amino group, secondary amino group |
| Primary amino group, secondary amino group, aminooxy group | Carbonyl group/aldehyde group |
| Carbonyl group/aldehyde group | Primary amino group, secondary amino group, aminooxy group |
| Primary amino group, secondary amino group, hydroxyl group, phenolic hydroxyl group, thiol group | Carboxylic acid active ester group |
| Carboxylic acid active ester group | Primary amino group, secondary amino group, hydroxyl group, phenolic hydroxyl group, thiol group |
| Boronyl group | cis-diol group (sugar group) |
| cis-diol group (sugar group) | Boronyl group |
| Amino group | (Thio)lactone group |
| (Thio)lactone group | Amino group |
| Alkyne group and derivative thereof | Azide group |
| Azide group | Alkyne group and derivative thereof |

### (Item 1H)

The particle according to Item 1, in which the (A2) functional group-containing moiety is an acidic group, and the (B-1) first functional group is a basic group.

### (Item 2)

The particle according to any one of the preceding items, in which the particle is used for producing a substrate for producing a sensor for analysis.

### (Item 3)

The particle according to any one of the preceding items, in which the modifier includes one or a plurality of modifiers.

### (Item 4)

The particle according to any one of the preceding items, in which the modifier contains a substituent capable of binding to a substrate.

### (Item 5)

The particle according to any one of the preceding items, in which the modifier contains at least one group selected from the group consisting of a reversible linking group and a polymerizable functional group.

### (Item 6)

The particle according to any one of the preceding items, in which the modifier contains a polymerizable functional group.

### (Item 7)

The particle according to any one of the preceding items, in which the modifier further includes at least one selected from the group consisting of a group for binding a signal substance, a signal substance, and a reversible linking group.

### (Item 8)

The particle according to any one of the preceding items, in which the modifier contains a group for binding a specific binding molecule to a target substance and a group for binding a signal substance; a group for binding a specific binding molecule to a target substance and a signal substance; or a reversible linking group.

### (Item 9)

The particle according to any one of the preceding items, in which the reversible linking group is a disulfide group, an imino binding group, an oxime group, a hydrazone binding group, a boronic acid cyclic ester bond, a carboxylic acid ester group, a carboxylic acid thioester group, or an avidin-biotin bond.

### (Item 10)

The particle according to any one of the preceding items, in which the group for binding a specific binding molecule to a target substance is an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group.

### (Item 11)

The particle according to any one of the preceding items, in which the group for binding a signal substance is an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group.

### (Item 12)

The particle according to any one of the preceding items, in which the signal substance is a fluorescent molecule, a radioactive element-containing substance, a magnetic substance, or an enzyme.

### (Item 13)

The particle according to any one of the preceding items, in which the chemical bonding between molecules is a non-covalent bonding.

### (Item 14)

The particle according to any one of the preceding items, in which the non-covalent bonding is an electrostatic interaction.

### (Item 15)

The particle according to any one of the preceding items, in which the particle core is formed of silica, polystyrene, polylactic acid, polymethyl methacrylate, a polylactic acid/glycolic acid copolymer, chitosan, iron oxide, gold, silver, platinum, aluminum oxide, copper oxide, titanium oxide, zinc oxide, zirconium oxide, cerium oxide, cobalt oxide, tin-doped indium oxide (ITO), antimony-doped tin oxide (ATO), graphene, graphene oxide, a carbon nanotube, nanodiamond, or hydroxyapatite, or any combination thereof.

### (Item 16)

The particle according to any one of the preceding items, in which the particle core is formed of silica or polystyrene.

### (Item 17)

A kit for producing a substrate for producing a sensor for analysis, the kit including:
the particles according to any one of the preceding items; and
a substrate.

### (Item 18)

The kit for producing a substrate for producing a sensor for analysis according to any one of the preceding items, further including a raw material of a polymer matrix.

### (Item 19)

Use of the particles according to any one of the preceding items for producing a substrate for producing a sensor for analysis.

### (Item 20)

A method for producing the particles according to any one of the preceding items, the method including:
A) a step of providing a particle core;
B) a step of providing a modifier capable of being integrated with the core by a separable intermolecular chemical bonding; and
C) a step of subjecting the particle core and the modifier to a condition under which the particle core and the modifier are integrated by a separable intermolecular chemical bonding.

### (Item 21)

A method for producing a substrate for producing a sensor for analysis, the method including:
A) a step of providing the particles according to any one of the preceding items;
B) a step of disposing the particles on a substrate; and
C) a step of forming a scaffold by subjecting the substrate to a condition under which the particles are dissociated from the substrate.

### (Item 22)

The method according to any one of the preceding items, further including a step of providing a raw material of a polymer matrix to the substrate on which the particles are immobilized.

### (Item 23)

The method according to any one of the preceding items, further including a step of forming a substrate on which the polymer matrix is disposed by subjecting the substrate to a condition under which the polymer matrix is polymerized.

### (Item 24)

A method for producing a sensor for analysis, the method including:
A) a step of providing the particles according to any one of the preceding items;
B) a step of disposing the particles on a substrate;
C) a step of forming a scaffold by subjecting the substrate to a condition under which the particles are dissociated from the substrate; and
D) a step of binding a substance required for measurement to the scaffold.

### (Item 25)

The method according to any one of the preceding items, further including a step of providing a raw material of a polymer matrix to the substrate on which the particles are disposed.

### (Item 26)

The method according to any one of the preceding items, further including a step of forming a substrate on which the polymer matrix is disposed by subjecting the substrate to a condition under which the polymer matrix is polymerized.

### (Item 27)

The method according to any one of the preceding items, in which in the step C, the condition under which the particles are dissociated from the substrate is a condition under which a reversible linking group is cleaved and a reversible binding group is produced.

### (Item 28)

The method according to any one of the preceding items, further including, after the step C, a step of converting the reversible binding group in the scaffold into a group for binding a specific binding molecule to a target substance or a group for binding a signal substance.

### (Item 29)

A substrate for producing a sensor for analysis, the substrate including:
A) a substrate;
B) a polymer matrix disposed on the substrate, the polymer matrix having a scaffold that at least partially fits into a molecule to be detected; and
C) a binding group disposed on the scaffold.

### (Item 30)

A sensor for analysis including:
A) a substrate;
B) a polymer matrix disposed on the substrate, the polymer matrix having a scaffold that at least partially fits into a molecule to be detected; and
C) a group for binding a specific binding molecule that binds to a molecule to be detected.

### (Item 31)

The substrate according to any one of the preceding items or the sensor according to any one of the preceding items, in which a cleanliness of the polymer matrix is 95% or more.

### (Item 32)

The substrate according to any one of the preceding items or the sensor according to any one of the preceding items, in which a standard value of the scaffold in the substrate is 80% or more.

### (Item 33)

The substrate according to any one of the preceding items or the sensor according to any one of the preceding items, in which a substituent derived from one or a plurality of modifiers integrated with the core by a separable intermolecular chemical bonding is bonded onto the substrate.

### (Item 34)

The substrate according to any one of the preceding items or the sensor according to any one of the preceding items, in which the substituent derived from a modifier is an amino group, an acidic group, a coordinate binding group, a carbonyl group, an aldehyde group, a ketone group, a boronyl group, a cis-diol group, a thiol group, a biotin group, a desthiobiotin group, a peptide ligand, or a reversible linking group.

### (Item 35)

The substrate according to any one of the preceding items or the sensor according to any one of the preceding items, in which the amino group is a primary to quaternary amino group, an aliphatic amino group, an aromatic amino group, a hydrazide, a guanidine, or an amidine.

### (Item 36)

The substrate according to any one of the preceding items or the sensor according to any one of the preceding items, in which the acidic group is a carboxy group, a sulfonic acid group, or a phosphate group.

### (Item 37)

The substrate according to any one of the preceding items or the sensor according to any one of the preceding items, in which the coordinate binding group is an NTA group or a His-tag.

### (Item 38)

The substrate according to any one of the preceding items or the sensor according to any one of the preceding items, in which the peptide ligand is a glutathione group or an RGD group.

### (Item 39)

The substrate according to any one of the preceding items or the sensor according to any one of the preceding items, in which the reversible linking group is a disulfide, pyridyl disulfide, imine, oxime, hydrazone, boronic acid cyclic ester, carboxylic acid ester, carboxylic acid thioester, or silyl group.

### (Item 40)

A sensor for analysis including:
A) a substrate;
B) a polymer matrix disposed on the substrate, the polymer matrix having a scaffold that at least partially fits into a molecule to be detected;
C) a substituent derived from a modifier integrated with the core by a separable intermolecular chemical bonding, the modifier being disposed on the scaffold;
D) a group for binding a signal substance or a signal substance disposed on the scaffold; and
E) a group for binding a specific binding molecule that binds to a molecule to be detected.

### (Item 41)

A substrate for producing a sensor for direct substrate analysis, the substrate including:
A) a substrate; and
B) the particles according to any one of the preceding items.

### (Item 42)

The substrate for producing a sensor for direct substrate analysis according to any one of items, further including a polymer matrix on which the particles are disposed, the polymer matrix being disposed on the substrate.

### (Item 43)

The substrate for producing a sensor for direct substrate analysis according to any one of the preceding items, further including a blocking agent present on the outermost layer.

### (Item 44)

A sensor for direct substrate analysis, the sensor including:
A) a substrate; and
B) the particles according to any one of the preceding items.

### (Item 45)

The sensor for direct substrate analysis according to any one of items, further including a polymer matrix on which the particles are disposed, the polymer matrix being disposed on the substrate.

### (Item 46)

The sensor for direct substrate analysis according to any one of the preceding items, further including a blocking agent present on the outermost layer.

### (Item 47)

The sensor for direct substrate analysis according to any one of the preceding items, further including a group for binding a specific binding molecule and a group for binding a signal substance or a signal substance present on the particle.

### (Item 48)

A method for producing a substrate for producing a sensor for direct analysis, the method including:
A) a step of providing the particles according to any one of the preceding items; and
B) a step of disposing the particles on a substrate.

### (Item 49)

The method according to any one of the preceding items, further including a step of providing a raw material of a polymer matrix to the substrate on which the particles are immobilized.

### (Item 50)

The method according to any one of the preceding items, further including a step of forming a substrate on which the polymer matrix is disposed by subjecting the substrate to a condition under which the polymer matrix is polymerized.

### (Item 51)

A method for producing a sensor for direct analysis, the method including:
A) a step of providing the particles according to any one of the preceding items; and
B) a step of disposing the particles on a substrate.

### (Item 52)

The method according to any one of the preceding items, further including a step of providing a raw material of a polymer matrix to the substrate on which the particles are immobilized.

### (Item 53)

The method according to any one of the preceding items, further including a step of forming a substrate on which the polymer matrix is disposed by subjecting the substrate to a condition under which the polymer matrix is polymerized.

### (Item 54)

The method according to any one of the preceding items, further including a step of binding a substance required for measurement to the particle.

As used herein, it is intended that the one or more features may be provided in further combination in addition to the specified combination. Still further embodiments and advantages of the present disclosure will be appreciated by those skilled in the art upon reading and understanding the following detailed description, if necessary. Advantageous Effects of Invention

By utilizing the technique related to the present disclosure, in a technique for producing a scaffold (for example, a concave portion or a convex portion) for extracellular vesicle detection using a molecular imprinting technique, a formation rate of the scaffold (for example, a concave portion or a convex portion) with respect to an input template material is dramatically improved, and an increase in sensitivity due to an increase in the number of scaffolds (for example, a concave portion or a convex portion) per unit area is expected. In addition, since the removal of the template substance is remarkably easy as compared with the conventional method, and the fluctuation between the substrates is suppressed, there is also a merit in terms of process. The method of the present disclosure is positioned as an important basic technique for producing a sensor substrate having excellent reproducibility and high sensitivity.

### Brief Description of Drawings

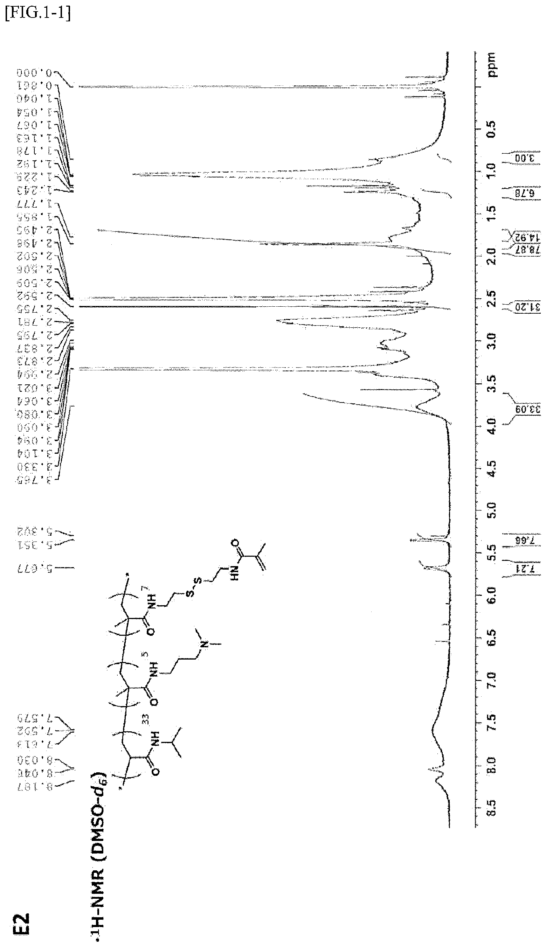

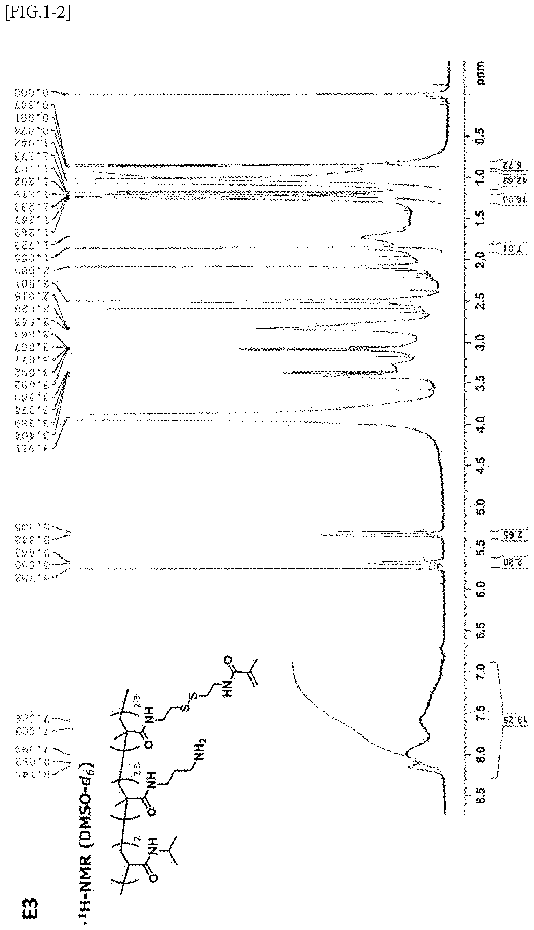

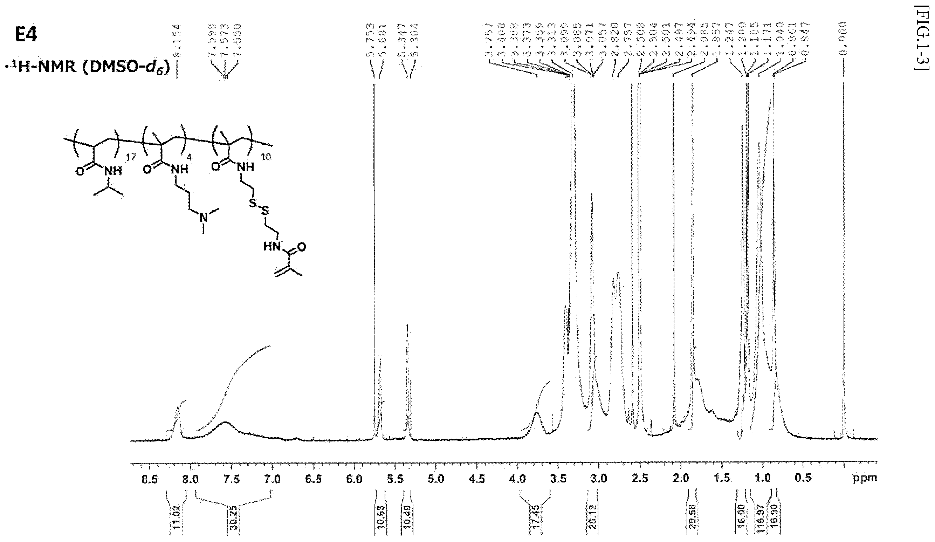

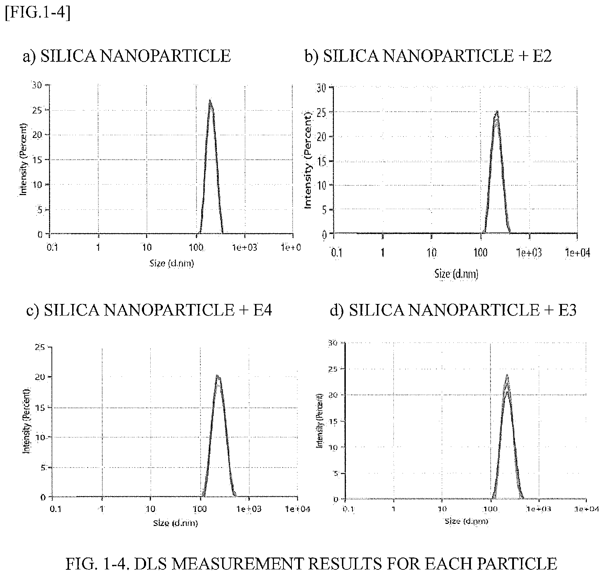

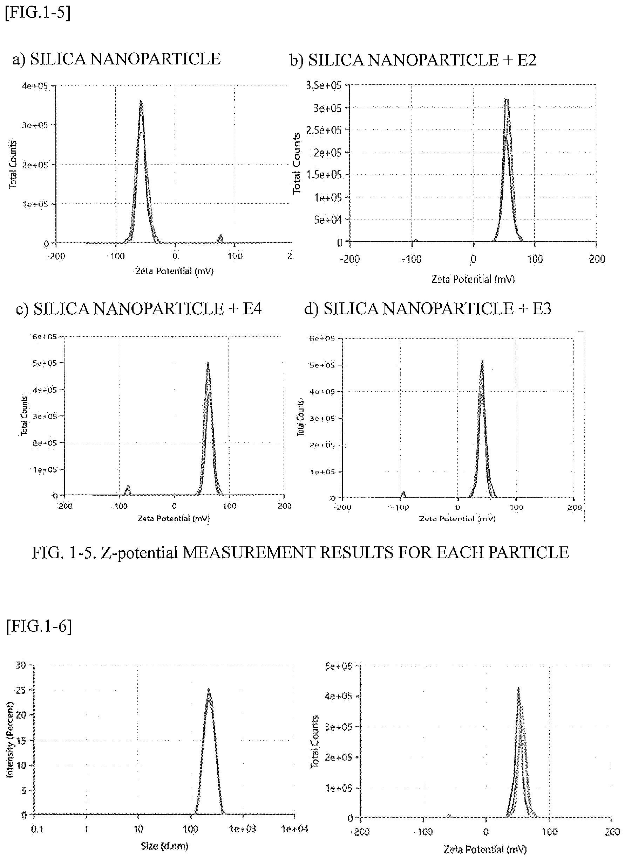

FIG. 1-1 illustrates an NMR chart of a polymer E2.
FIG. 1-2 illustrates an NMR chart of a polymer E3.
FIG. 1-3 illustrates an NMR chart of a polymer E4.
FIG. 1-4 illustrates DLS measurement results of particles in which silica particles and polymers E2 to E4 are integrated.
FIG. 1-5 illustrates Z-potential measurement results of particles in which silica particles and polymers E2 to E4 are integrated.
FIG. 1-6 illustrates DLS measurement results and Z-potential measurement results of particles in which silica particles and a polymer (E2-0) are integrated.
FIG. 2 illustrates images of a particle-immobilized substrate of Example 2 observed with a fluorescence microscope.
FIG. 3 illustrates a hybrid cell count analysis result using a fluorescence observation result.
FIG. 4 illustrates a result of an adsorption experiment of the sensor for analysis with hepatitis B virus.
FIG. 5 illustrates a result of an adsorption experiment of the sensor for analysis with a nanobody or a full body.
FIG. 6-1 illustrates images of a particle-immobilized substrate of Example 7 observed with a fluorescence microscope.
FIG. 6-2 illustrates images after polymerization of a polymer layer on a substrate of Example 7 observed with a fluorescence microscope.
FIG. 6-3 illustrates images after removal of silica nanoparticles on the substrate of Example 7 observed with a fluorescence microscope.
FIG. 6-4 illustrates images of silica nanoparticles and fluorescent cationic polymers on the substrate of Example 7 observed with a fluorescence microscope.
FIG. 6-5 illustrates a result of exosome concentration-dependent fluorescence intensity change using a direct sensing chip.
FIG. 7-1 illustrates images of a particle-immobilized substrate of Example 8 observed with a fluorescence microscope.
FIG. 7-2 illustrates images after an ammonium hydrogen fluoride treatment observed with a fluorescence microscope.
FIG. 7-3 illustrates film thickness measurement results of the substrate after the ammonium hydrogen fluoride treatment.
FIG. 7-4 illustrates an exosome detection result using a sensor for analysis of Example 8.
FIG. 8 illustrates a result of exosome concentration-dependent fluorescence intensity change using a sensor for analysis of Example 9.
FIG. 9 illustrates a fluorescence microscopic image of a substrate on which a complex of a His-Tag polymer and silica nanoparticles is immobilized.
FIG. 10 illustrates a fluorescence microscopic image and an SEM image of a substrate on which a complex of cationic silica nanoparticles and an anionic polymer Ex8 is immobilized.
FIG. 11 illustrates a fluorescence microscopic image and an SEM image of a substrate on which particles using biodegradable nanoparticles are immobilized.
FIG. 12-1 illustrates a fluorescence microscopic image and an SEM image of a substrate on which a complex of silica nanoparticles and a cationic polymer E2P20 containing an aromatic component is immobilized.
FIG. 12-2 illustrates a fluorescence microscopic image of a substrate on which a complex of polystyrene nanoparticles and a cationic polymer E2P20 containing an aromatic component is immobilized.
FIG. 13 illustrates a result of surface Z-potential measurement by DLS.
FIG. 14 illustrates fluorescence microscopic images after immobilization and polymerization in Example 16.
FIG. 15 illustrates fluorescence microscopic images after core particle removal in Example 16.
FIG. 16 illustrates a fluorescence microscopic image and an SEM image of a substrate on which silica nanoparticles introduced with a His-tag and a thiol group are immobilized.
FIG. 17 illustrates a change in fluorescence intensity of a substrate surface of a sensor produced without using particles.

### Description of Embodiments

Hereinafter, the present disclosure will be described in more detail.

Throughout the present disclosure, the expression of singular forms is to be understood as including the concept of plural forms unless otherwise stated. Therefore, the singular expressions (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present disclosure have the same meaning as commonly understood by those skilled in the art to which the present disclosure pertains. In the case of conflict, the present disclosure (including definitions) will control.

### (Definitions)

First, terms and general techniques used in the present specification will be described.

As used herein, the "particle core" refers to a basic substance having a structure serving as a template for forming a scaffold (for example, a concave portion or a convex portion) provided on a substrate in a sensor for analysis, and is not particularly limited as long as it can be used as a template in molecular imprinting, and includes inorganic particles and organic particles. A case where the size is in a range of nm may be referred to as a nanoparticle core, but it is understood that the nanoparticle core is not strictly distinguished as used herein, and the nanoparticle core does not exclude a nanoparticle core having a larger or smaller core structure unless the discussion is specifically intended to limit the size. The particle core includes (A1) a particle core core and (A2) a functional group-containing moiety present on the particle core.

As used herein, the term "particle core core" refers to a core constituting the particle core, and the functional group-containing moiety constitutes the particle core. Examples of the particle core core include section A1 in Table 1A. Specifically, examples of the particle core core include a metal (gold, silver, platinum, tin-doped indium oxide (ITO), antimony-doped tin oxide (ATO), or the like), an oxide of a metal (iron oxide, aluminum oxide, copper oxide, titanium oxide, zinc oxide, zirconium oxide, cerium oxide, cobalt oxide, or the like), graphene, graphene oxide, a carbon nanotube, nanodiamond, a nitride, a fluoride, a sulfide, a boride, a complex compound thereof, and hydroxyapatite, and preferably include silicon dioxide (silica). In addition, examples of the organic particle include, but are not limited to, a latex cured product, dextran, chitosan, polylactic acid, poly(meth)acrylic acid, polymethyl methacrylate, a poly(lactic-co-glycolic acid) copolymer (PLGA), polystyrene, and polyethyleneimine.

As used herein, the term "functional group-containing moiety" present on the particle core core is a moiety containing a functional group present on the particle core core, and the functional group-containing moiety is modified to impart desired properties to the particle core core. Examples of the functional group-containing moiety are described in A2 in Table 1A, and preferred combinations of the functional group-containing moiety and the first functional group may be the functional groups listed on the left and right in Table 1A.

A size of the particle core can vary depending on a target intended in the final sensor for analysis, and examples thereof include, but are not limited to, about 1 nm to about 100 µm, about 1 nm to about 20 nm, about 20 nm to about 500 nm, about 50 nm to about 200 nm, about 100 nm to about 500 nm, about 1 µm to about 10 µm, and about 10 µm to about 100 µm.

As used herein, the term "first functional group capable of binding to the particle core and capable of forming a chemical bond between molecules" refers to any functional group that imparts the ability to bind to the particle core via a functional group-containing moiety. Examples of the first functional group are described in B1 in Table 1A, and preferred combinations of the functional group-containing moieties may be the functional groups listed on the left and right in Table 1A. As used herein, the intermolecular chemical bonding may be referred to as an interaction, and these terms are used in the same meaning unless otherwise mentioned.

As used herein, the term "second functional group that imparts a desired property in the sensor for analysis" refers to any functional group that imparts a desired property in the sensor for analysis, and refers to a group that imparts a desired property in the sensor for analysis, and includes, for example, a signal group and a binding group capable of binding to a target substance. Examples of the second functional group are shown in B2 in Table 1A-4.

Specific examples of the functional group present on the particle core include, but are not limited to, groups shown in Table 1A-4.

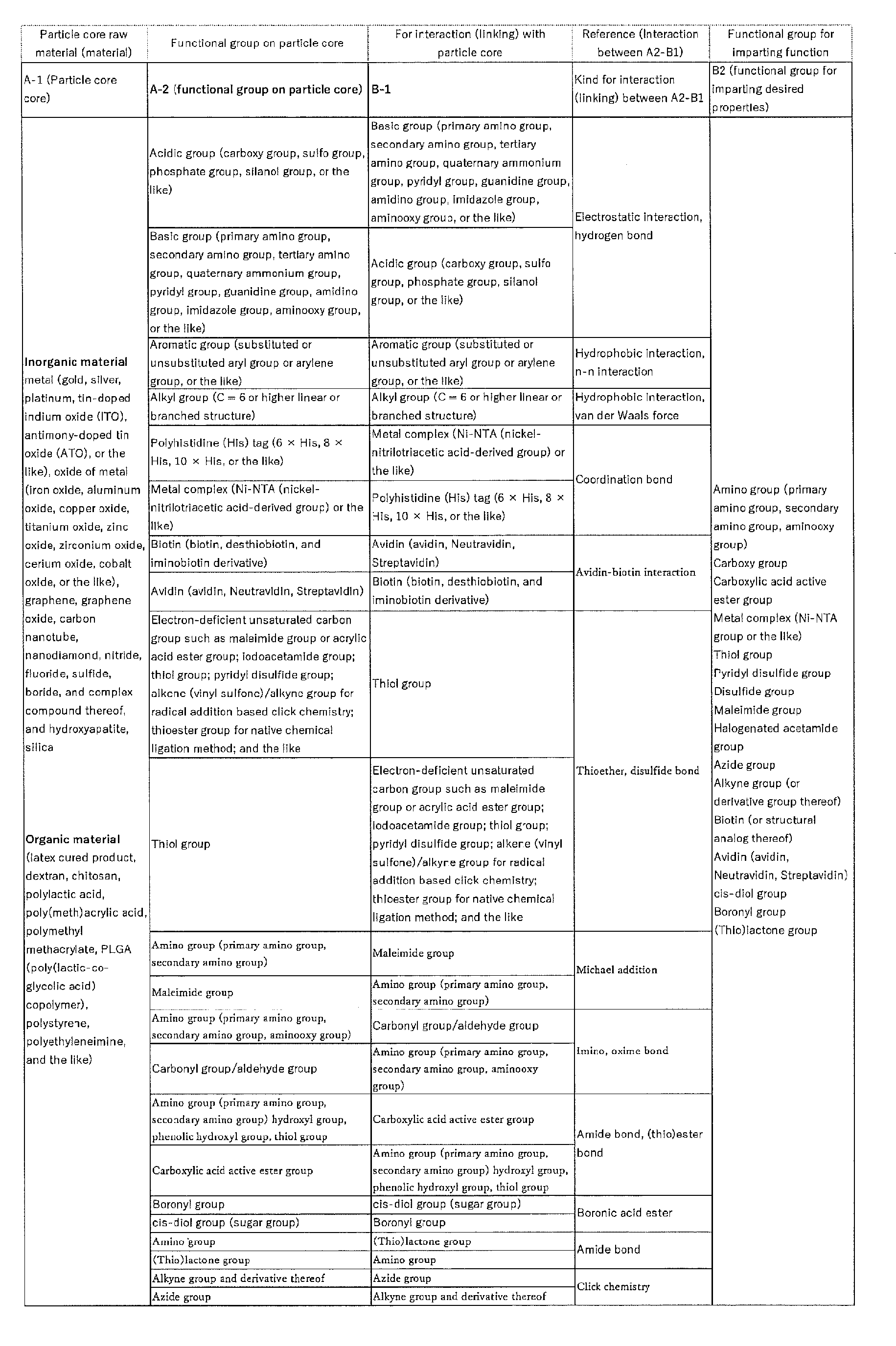

As used herein, the term "modifier" is a substance for directly or indirectly imparting an intended function in a sensor for analysis or a substrate for producing a sensor for analysis, which contains various functional groups or has a structure that can contain various functional groups in the structure or contains a signal group in order to impart such a function. As used herein, the modifier is advantageously a substance capable of a chemical bonding between the particle core and molecules, and preferably a substance capable of integrating with the particle core by an intermolecular chemical bonding that is separable under conditions that do not damage the substrate and capable of integrating with the particle core by an intermolecular chemical bonding that is separable under conditions that do not damage the particle core and/or capable of a chemical bonding between the particle core and molecules. The modifier includes (B0) a modifier core and (B-1) a first functional group capable of binding to the particle core of the present disclosure, but capable of intermolecular chemical bonding and/or (B2) a second functional group that imparts the properties desired in the sensor for analysis. Specific examples of the first and second functional groups include, but are not limited to, groups shown in Table 3A. The following are representative modifiers and are described in more detail elsewhere herein. Examples of the representative modifier include, but are not limited to, a polyisopropylacrylamide copolymer, a poly(meth)acrylamide copolymer, a polylactic acid derivative, a poly(meth)acrylic acid copolymer, a poly(meth)acrylic acid methyl copolymer, a polyhydroxyethyl (meth)acrylate copolymer, a chitosan derivative, a polylysine derivative, and combinations thereof. In addition, one or a plurality of modifiers may also be integrated with the particle core.

As used herein, the phrase "separable under conditions that do not damage the particle core" refers to any condition that when referring to a modifier, an intermolecular chemical bonding between the modifier and the particle core may be separated, but the particle core is not substantially damaged. In this case, typically, a condition under which the particle core is not substantially damaged can be said to be a condition under which the particle core core is not substantially damaged. Here, it is understood here that the particle core is not substantially damaged when there is no substantial hindrance to the detection in the sensor for analysis used. Preferably, the particle core is disposed on the substrate, and the intermolecular chemical bonding between the particle core and the substrate is cleaved without damaging the particle core, and the particle core and the substrate can be separated. Examples of the intermolecular chemical bonding that is separable under conditions that do not damage the nanoparticles include, but are not limited to, a non-covalent bonding such as an electrostatic interaction, and a covalent bonding such as a disulfide bonding.

As used herein, the phrase "separable under conditions that do not damage the substrate" refers to any condition that when referring to a modifier, an intermolecular chemical bonding between the modifier and the particle core may be separated, but the substrate of the sensor for analysis for which the modifier is intended is not substantially damaged. It is understood here that the substrate is not substantially damaged when there is no substantial hindrance to the detection in the sensor for analysis used. Preferably, the particle core is disposed on the substrate, and the intermolecular chemical bonding between the particle core and the substrate is cleaved without damaging the substrate, and the particle core and the substrate can be separated. Examples of the intermolecular chemical bonding that is separable under conditions that do not damage the substrate include, but are not limited to, a non-covalent bonding such as an electrostatic interaction, and a covalent bonding such as a disulfide bond. Those skilled in the art will appreciate that the same conditions may be used in the present specification as "separable under conditions that do not damage the particle core" and "separable under conditions that do not damage the substrate".

As used herein, the term "integration" generally refers to that substances are put together, and when referring to a modifier and a particle core, the substances may be integrated by any action as long as the substances are in a state of not being dissociated other than a case of being subjected to a condition such as "separable under conditions that do not damage the particle core" and/or "separable under conditions that do not damage the substrate" and/or have an intermolecular chemical bonding in an environment in which a sensor for analysis or a substrate for producing a sensor for analysis is produced. Examples of such an action include a covalent bonding or a non-covalent bonding.

As used herein, the term "non-covalent bonding" refers to any bonding or interaction in which substances are chemically bonded to each other by an intermolecular chemical bonding that is not a covalent bonding. Examples thereof include, but are not limited to, a hydrogen bonding, an electrostatic interaction, and a hydrophobic interaction.

As used herein, the term "group" refers to a monovalent group unless otherwise specified. Examples of the non-monovalent group include an alkylene group (divalent). In addition, as used as used herein, the term "group" may be omitted.

As used herein, the term "reversible linking group" refers to a direct bond or a divalent or higher valent group in which cleavage and bonding are reversible. Specific examples thereof include, but are not limited to, groups shown in 1-3 of Table 2A below.

**[Table 2A-1]**

| 1 - 1 | 1 - 2 | 1 - 3 | 1 - 4 |
|---|---|---|---|
| Binding functional group | Corresponding binding functional group | Reversible linking group | Mode of bonding of reversible moiety |
| (Group for binding specific binding molecule to target molecule) | (Binding group) | (Reversible linking group) | |
| (Group for binding signal substance) | (Binding group) | | |
| Thiol group, disulfide group, and pyridyl disulfide group | Thiol group, disulfide group, and pyridyl disulfide group | Disulfide group | Covalent bonding |
| Boronyl group | Sugar group | Boronic acid cyclic ester | Covalent bonding |
| Boronyl group | cis-diol group | Boronic acid cyclic ester | Covalent bonding |
| Carbonyl group/aldehyde group | Amino group | Imino binding group | Covalent bonding |
| Amino group | Carbonyl group/aldehyde group | | Covalent bonding |
| Carboxyl group | Hyd roxyl group | Carbonic acid ester group | Covalent bonding |
| Hydroxyl group | Carboxyl group/carbonic acid activated ester group | | |
| Carboxyl group | Thiol group | Carbonic acid thioester group | Covalent bonding |
| Thiol group | Carboxyl group/carbonic acid activated ester group | | |
| Aminooxy group | Carboxyl group/aldehyde group | Oxime group | Covalent bonding |
| Carbonyl group/aldehyde group | Aminooxy group | | |
| Carboxyl group/aldehyde group | Hyd roxyl group | Acetal group | Covalent bonding |
| Hydroxyl group | Carboxyl group/aldehyde group | | |

**[Table 2A-2]**

| | | | |
|---|---|---|---|
| Metal complex (Ni-NTA (nickel-nitrilotriacetic acid-derived group) or the like) | Polyhistidine (His) tag (6 ' His, 8 ' His, 10 His, or the like) | Coordination bond | Non-covalent bonding |
| Basic group (amino group, cyclic secondary amino group (for example, pyrrolidyl group), piperidi group, pyridyl group, imidazole group, guanidine group, or the like) | Acidic group (carboxyl group/carbonic acid activated ester group) | Hydrogen bond | Non-covalent bonding |
| Hydroxyl group | | | |
| Acidic group | Basic group | | Non-covalent bonding |
| (Carboxyl group or the like) | (Amino group, cyclic secondary amino group (for example, pyrrolidyl group or piperidi group), pyridyl group, imidazole group, guanidine group, or the like), hydroxyl group | | |
| Aromatic group (phenyl group of aminophenyl group or the like, naphthyl group of aminonaphthyl group or the like, pyridyl group, or the like) | Aromatic group (phenyl group of aminophenyl group or the like, naphthyl group of aminonaphthyl group or the like, pyridyl group, or the like) | Hydrophobic bond | Non-covalent bonding |
| Hydrazide | Carbonyl group/aldehyde group | Hydrazone binding group | Covalent bonding |
| Carbonyl group/aldehyde group | Hydrazide | | |
| Avidin (neutravidin, streptavidin) | Biotin (biocytin, desbiotin) | Avidin-biotin bond | Non-covalent bonding |
| Biotin (biocytin, desbiotin) | Avidin (neutravidin, streptavidin) | | |
| Glutathione (GHS) | Glutathione-S-transferase (GST) | Glutathione (GHS)-glutathione-s-trans | Non-covalent bonding |

**[Table 2A-3]**

| | | | |
|---|---|---|---|
| Glutathione-S-transferase (GST) | Glutathione (GHS) | Ferase (GST) bond | |
| Phos-tag | Phosphate ion | Coordination bond | Non-covalent bonding |
| Phosphate ion | Phos-tag | | |

As used herein, the term "... functional group capable of forming a chemical bond between molecules" refers to a functional group capable of bonding between different molecules. Specific examples thereof include, but are not limited to, groups shown in Table 3A.

**[Table 3A]**

| Kind of intermolecular chemical bonding | Functional group on particle core | Functional group capable of forming bond between particle core and molecule |
|---|---|---|
| | | B-1 |
| Electrostatic Interaction hydrogen bond | Acidic group (carboxy group, sulfo group, phosphate group, silanol group, or the like) | Basic group (primary amino group, secondary amino group, tertiary amino group, quaternary ammonium group, pyridyl group, guanidine group, amidino group, imidazole group, aminooxy group, or the like) |
| Electrostatic interaction hydrogen bond | Basic group (primary amino group, secondary amino group, tertiary amino group, quaternary ammonium group, pyridyl group, guanidine group, amidino group, imidazole group, aminooxy group, or the like) | Acidic group (carboxy group, sulfo group, phosphate group, silanol group, or the like) |
| Hydrophobic interaction pai-pai interaction | Aromatic group (substituted or unsubstituted aryl group or arylene group, or the like) | Aromatic group (substituted or unsubstituted aryl group or arylene group, or the like) |
| Hydrophobic interaction force between molecules | Alkyl group (C = 6 or higher linear or branched structure) | Alkyl group (C = 6 or higher linear or branched structure) |
| Coordination bond | Polyhistidine (His) tag (6 × His, 8 × His, 10 × His, or the like) | Metal complex (Ni-NTA (nickel-nitrllotriacetic acid-derived group) or the like) |
| Coordination bond | Metal complex (Ni-NTA (nickel-nitrilotriacetic acid-derived group) or the like) | Polyhistidine (His) tag (6 × His, 8 × His, 10 × His, or the like) |
| Avidin-biotin Interaction | Biotin (biotin, desthiobiotin, and iminobiotin derivative) | Avidin (avidin, Neutravidin, Streptavidin) |
| Avidin-biotin interaction | Avidin (avidin, Neutravidin, Streptavidin) | Biotin (biotin, desthiobiotin, and iminoblotin derivative) |
| Thioether disulfide bond | Electron-deficient unsaturated carbon group such as maleimlde group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |
| Thioether disulfide bond michael addition | Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Michael addition | Amino group (primary amino group, secondary amino group) | Maleimide group |
| | Maleimide group | Amino group (primary amino group, secondary amino group) |
| Imine bond oxime bond | Amino group (primary amino group, secondary amino group, aminooxy group) | Carbonyl group/aldehyde group |
| | Carbonyl group/aldehyde group | Amino group (primary amino group, secondary amino group, aminooxy group) |
| Amide bond ester bond thioester bond | Amino group (primary amino group, secondary amino group) hydroxyl group, phenolic hydroxyl group, thiol group | Carboxylic acid active ester group |
| | Carboxylic acid active ester group | Amino group (primary amino group, secondary amino group) hydroxyl group, phenolic hydroxyl group, thiol group |
| Boronic acid ester | Boronyl group | cis-diol group (sugar group) |
| | cis-diol group (sugar group) | Boronyl group |
| Amide bond | Amino group | Lactone group, Thiolactone group |
| | Lactone group, Thiolactone group | Amino group |
| Click chemistry | Alkyne group and derivative thereof | Azide group |
| | Azide group | Alkyne group and derivative thereof |

As used herein, the term "polymerizable functional group" refers to a functional group that can be polymerized with another polymerizable functional group to form a polymer. Examples thereof include, but are not limited to, a vinyl group, an allyl group, an epoxy group, a (meth)acryloyl group, and a (meth)acrylamide group.

As used herein, the term "substituent bondable to a substrate" refers to a group that can be arranged on a substrate by being covalently bonded or non-covalently bonded to a substituent on a substrate. Examples thereof include, but are not limited to, groups shown in Table 2A.

As used herein, the term "substrate" refers to a substance serving as a base of a sensor for analysis. A material of the substrate may be, for example, a material selected from the group consisting of a metal, a metal oxide, glass, paper (cellulose), silicon dioxide, silicon, a resin, and a combination thereof. Examples of the metal include, but are not limited to, gold, silver, copper, aluminum, titanium, tungsten, and molybdenum. Examples of the resin include, but are not limited to, poly(meth)acrylate, polystyrene, an acrylonitrile-butadiene-styrene copolymer (ABS), polycarbonate, polyester, polyethylene, polypropylene, nylon, polyurethane, a silicone resin, a fluororesin, a methylpentene resin, a phenol resin, a melamine resin, an epoxy resin, and a vinyl chloride resin.

As used herein, the term "polymer matrix" refers to a polymer that forms a matrix, and usually refers to a substance formed by polymerization of monomers. Advantageously, the matrix may have any shape or structure as long as it has a shape and structure suitable for the sensor when disposed in the sensor for analysis or the substrate for a sensor for analysis of the present disclosure. The shape or structure of the matrix is, for example, a thin film shape or a spherical shape (particle shape). As a preferred configuration of the matrix, a matrix having high biocompatibility is a main component in order to suppress adsorption of substances other than the target as much as possible.

As used herein, the term "raw material of a polymer matrix" refers to a raw material capable of forming a polymer matrix by a reaction. In general, in order to form a polymer matrix, it is sufficient to contain at least one monomer and at least one suitable polymerization initiator, but in addition to these, for example, an additional monomer (which may be partially polymerized), an additional polymerization initiator, a crosslinking agent, a RAFT agent, a catalyst, a reducing agent, a solvent, and the like can be contained. Examples of the monomer include, but are not limited to, styrene, N-isopropylacrylamide, and 2-methacryloyloxyethyl phosphorylcholine. Examples of the polymerization initiator include, but are not limited to, 2,2'-azobis(isobutyronitrile) (AIBN) and ethyl α-bromoisobutyrate. Examples of the crosslinking agent include, but are not limited to, a melamine compound, a guanamine compound, a glycoluril compound, N,N'-methylenebisacrylamide, and (tri, tetra, penta, hexa, or poly)ethylene glycol dimethacrylate. Examples of the RAFT agent include, but are not limited thereto, benzyl benzodithioate and 2-cyano-2-[(dodecylsulfanylthiocarbonyl)sulfanyl]propane. Examples of the catalyst include CuBr₂, but are not limited thereto. Examples of the reducing agent include ascorbic acid, but are not limited thereto. The solvent is selected from those generally known as solvents without particular limitation, and examples thereof include pure water, a buffer solution, MeOH, EtOH, DMA, and DMF, but are not limited thereto.

As used herein, the term "sensor for analysis" refers to a sensor capable of specifically recognizing and analyzing a target substance.

As used herein, the term "substrate for producing a sensor for analysis" refers to any substrate for producing a sensor for analysis, and any shape and material can be used as long as they are appropriate for the sensor for analysis. Preferably, the substrate for producing a sensor for analysis is a substrate having a scaffold (for example, a concave portion or a convex portion), and it is advantageous that the substrate for producing a sensor for analysis is configured so that a user can easily customize an object to be detected to a sensor capable of detecting the objected to be targeted with higher sensitivity by modifying the group for binding a specific binding molecule and the group for binding a signal substance in the scaffold (for example, a concave portion or a convex portion). As used herein, the term "substrate for producing a sensor for analysis" is also referred to as a "measurement substrate".

As used herein, the term "molecular imprinted polymer" refers to any polymer used in a molecular imprinting technique (see Takeuchi T. et. al. Chromatography, 2016, 37 (2), 43-64), and preferably refers to a substance recognition material having a binding space for a target substance obtained by forming a complex of a target substance or a derivative thereof and a functional monomer with a covalent bonding and/or a non-covalent bonding, performing polymerization with a crosslinking agent, and then removing the target substance.

As used herein, the phrase "... at least partially fits into a molecule" refers to a shape and/or structure that substantially enables detection or analysis of a molecule to be targeted when used in the sensor for analysis.

As used herein, the term "scaffold" refers to a portion for binding and stabilizing a substance required for measurement, which is a concave portion or a convex portion, and can immobilize or stabilize a binding group (a reversible binding group, a group for binding a specific binding molecule, a group for binding a signal substance, or the like) when used in the sensor for analysis of the present disclosure.

As used herein, the term "concave portion" refers to a void or a pore formed so as to capture a target when used in the sensor for analysis of the present disclosure, and preferably refers to a pore portion formed on a polymer matrix formed in the sensor for analysis.

As used herein, the term "outermost layer" refers to the outermost layer in the sensor or the substrate for producing a sensor of the present disclosure, and may refer to the substrate or the polymer matrix, or such an additional substance when coated with such an additional substance.

As used herein, the term "direct-type substrate" refers to a core particle or a substrate including a core particle to which a modifier clings, the modifier being formed to capture a target when used in the sensor for analysis of the present disclosure, and is preferably a substrate formed on a polymer matrix formed in the sensor for analysis and including a core particle with a modifier or a core particle. The direct-type substrate may include, but is not limited to, a substrate formed in a convex shape, or a substrate in which a polymer matrix is formed using a cube-like core to a height of the core or more.

As used herein, the term "condition under which a polymer matrix is polymerized" or "condition under which a raw material of a polymer matrix" refers to a condition under which a polymer matrix is polymerized when a raw material of the polymer matrix is present.

As used herein, the term "functional monomer" refers to a functional group capable of imparting some function to a polymer to be produced or a sensor or a substrate using the polymer when polymerized, and examples thereof include monomers having a basic group, an acidic group, a substituted or unsubstituted aromatic group, a boronyl group, as an intermolecular chemical bonding group. Examples of the basic group include, but are not limited thereto, a substituted or unsubstituted amino group, a substituted or unsubstituted nitrogen-containing aromatic group, an amidino group, and a guanidino group. Examples of the acidic group include, but are not limited thereto, a carboxyl group, a sulfo group, and a phosphate group. Examples of the substituted or unsubstituted aromatic group include, but are not limited to, a substituted or unsubstituted aryl group and a substituted or unsubstituted arylene group.

As used herein, the phrase "condition under which the particles are dissociated from the substrate" refers to a condition under which the particles disposed on the substrate can be separated from the substrate by breaking the intermolecular chemical bonding between the substrate and the particles. Examples thereof include heating, cooling, pH preparation (an acid treatment or an alkali treatment), washing with a surfactant containing solution, ultrasonic irradiation, light irradiation, shaking, and a reduction treatment.

As used herein, the term "substance required for measurement" refers to a substance capable of capturing and detecting a target substance. Examples thereof include a capturing agent and a label. The capturing agent refers to any agent for capturing an object to be measured, and examples thereof include an antibody, an antibody fragment, an antibody mimetic, a nucleic acid aptamer (DNA, RNA, a peptide nucleic acid, or an artificial nucleic acid), a phospholipid recognition protein, and a lectin.

As used herein, the term "signal substance" refers to a substance that can detect a target substance. Examples thereof include a fluorescent molecule, a radioactive element-containing substance, and a magnetic substance. From the viewpoint of ease of detection and the like, the signal substance is preferably a fluorescent substance. For example, a fluorescent substance/fluorescent substance pair or a fluorescent substance/extinction substance pair, such that the binding of the target substance can be detected by inhibition (or promotion) of Forster resonance energy transfer (FRET). In addition, a heterologous enzyme that forms an enzyme cascade, such that it is possible to detect a target substance by detecting enzyme cascade inhibition.

As used herein, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. As used herein, when a plurality of (two or more) markers or factors or means for capturing the markers are labeled by a fluorescence method, the markers are labeled with fluorescent substances having different maximum fluorescence emission wavelengths. A difference in maximum fluorescence emission wavelength is preferably 10 nm or more. In the case of labeling a ligand, any fluorescent substance can be used as long as it does not affect the function, and examples of the fluorescent substance include Alexa^{™} Fluor, BODIPY, ATTO, a quantum dot (QDot), and a fluorescent protein (GFP, YFP, mCherry, or the like). Alexa^{™} Fluor is a water-soluble fluorescent dye obtained by modifying coumarin, rhodamine, fluorescein, cyanine, or the like, is a series corresponding to a wide range of fluorescence wavelengths, and is significantly stable, bright, and less pH sensitive as compared with other fluorescent dyes having corresponding wavelengths. Examples of a combination of fluorescent dyes having a maximum fluorescence wavelength of 10 nm or more include a combination of Alexa^{™} 555 and Alexa^{™} 633 and a combination of Alexa^{™} 488 and Alexa^{™} 555. In the case of labeling a nucleic acid, any fluorescent dye can be used as long as it can bind to a base moiety thereof, but it is preferable to use a cyanine dye (for example, Cy3 or Cy5 of CyDyeTM series, or the like), a rhodamine 6G reagent, 2-acetylaminofluorene (AAF), an iodine derivative of AAF (AAIF), or the like. Examples of the fluorescent substance having a difference in maximum fluorescence emission wavelength of 10 nm or more include a combination of Cy5 and a rhodamine 6G reagent, a combination of Cy3 and fluorescein, and a combination of a rhodamine 6G reagent and fluorescein. In the present disclosure, such a label can be utilized to modify the object to be targeted so that the object can be detected by detection means used. Such modifications are known in the art and those skilled in the art can carry out such methods as appropriate for the label and depending on the object to be targeted.

As used herein, the term "condition under which a reversible linking group is cleaved and a reversible binding group is produced" refers to a condition under which a reversible linking group is cleaved and then the same reversible linking group or different reversible linking group is bonded to produce a new reversible linking group.

As used herein, the term "group for binding a specific binding molecule to a target substance" refers to a group capable of binding a specific binding molecule capable of specifically binding to a target substance. Examples of the group for binding a specific binding molecule to a target substance include binding functional groups listed in 2-1 of Table 2A.

**[Table 2A-1]**

| 2 - 1 | 2 - 2 |
|---|---|
| Binding functional group | Corresponding binding functional group |
| (Group for binding specific binding molecule to target molecule) | (Binding group) |
| (Binding group of group for binding signal substance) | (Binding group) |
| Amino group (monovalent amino group, divalent amino group, or the like) | Carbonic acid activated ester group (activated ester group using N-hydroxysuccinimide, nitrophenol, or pentafluorophenol; or carbamic acid activated ester group such as NHS carbamate); carboxyl group; aldehyde group; isocyanate group, isothiocyanate group; epoxy group; maleimide group; and the like |
| Amino group | Carboxyl group |
| Carboxyl group | Amino group |
| Sugar group (cis-diol group) | Boronyl group |
| Boronyl group | Sugar group (cis-diol group) |

**[Table 2A-2]**

| | |
|---|---|
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |
| Carbonyl group/aldehyde group | Amino group |
| Amino group | Carbonyl group/aldehyde group |
| Aminooxy group | Carbonyl group/aldehyde group |
| Carbonyl group/aldehyde group | Aminooxy group |
| Carbonyl group/aldehyde group | Hydroxyl group |
| Hydroxyl group | Carbonyl group/aldehyde group |
| Hydroxyl group and phenolic hydroxyl group | Carbonic acid activated ester group |
| Metal complex (Ni-NTA (nickel-nitrilotriacetic acid-derived group) or the like) | Polyhistidine (His) tag (6 × His, 8 × His, 10 × His, or the like) |
| Hydrazide group | Carbonyl group/aldehyde group |
| Carbonyl group/aldehyde group | Hydrazide |
| Avidin (neutravidin, streptavidin) | Biotin (biocytin, desbiotin) |
| Biotin (biocytin, desbiotin) | Avidin (neutravidin, streptavidin) |
| Glutathione (GSH) | Glutathione-S-transferase |
| Glutathione-S-transferase | Glutathione (GSH) |

As used herein, the term "group for binding a signal substance" refers to a group capable of modifying a signal substance. Examples of the signal substance include a fluorescent molecule, a radioactive element-containing substance, and a magnetic substance. From the viewpoint of ease of detection and the like, the signal substance is preferably a fluorescent substance. Examples of the group for binding a signal substance include binding functional groups listed in 2-1 of Table 3A.

**[Table 3A-1]**

| 2 - 1 | 2 - 2 |
|---|---|
| Binding functional group | Corresponding binding functional group |
| (Group for binding specific binding molecule to target molecule) | (Binding group) |
| (Binding group of group for binding signal substance) | (Binding group) |
| Amino group (monovalent amino group, divalent amino group, or the like) | Carbonic acid activated ester group (activated ester group using N-hydroxysuccinimide, nitrophenol, or pentafluorophenol; or carbamic acid activated ester group such as NHS carbamate); carboxyl group; aldehyde group; isocyanate group, isothiocyanate group; epoxy group; maleimide group; and the like |
| Amino group | Carboxyl group |
| Carboxyl group | Amino group |
| Sugar group (cis-diol group) | Boronyl group |
| Boronyl group | Sugar group (cis-diol group) |
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |

**[Table 3A-2]**

| | |
|---|---|
| Carbonyl group/aldehyde group | Amino group |
| Amino group | Carbonyl group/aldehyde group |
| Aminooxy group | Carbonyl group/aldehyde group |
| Carbonyl group/aldehyde group | Aminooxy group |
| Carbonyl group/aldehyde group | Hydroxyl group |
| Hydroxyl group | Carbonyl group/aldehyde group |
| Hydroxyl group and phenolic hydroxyl group | Carbonic acid activated ester group |
| Metal complex (Ni-NTA (nickel-nitrilotriacetic acid-derived group) or the like) | Polyhistidine (His) tag (6 × His, 8 × His, 10 × His, or the like) |
| Hydrazide group | Carbonyl group/aldehyde group |
| Carbonyl group/aldehyde group | Hydrazide |
| Avidin (neutravidin, streptavidin) | Biotin (biocytin, desbiotin) |
| Biotin (biocytin, desbiotin) | Avidin (neutravidin, streptavidin) |
| Glutathione (GSH) | Glutathione-S-transferase |
| Glutathione-S-transferase | Glutathione (GSH) |

As used herein, the term "property desired in a sensor for analysis" includes a property capable of binding to a target substance and/or binding to a signal substance when used in a sensor for analysis.

As used herein, the term "cleanliness" refers to a proportion at which particles disposed on a substrate are removed after cleaning. Examples of the method for measuring the cleanliness include a method of observing whether silica is removed after cleaning and a method of observing residual silica with a scanning electron microscope using silica nanoparticles fluorescently labeled (FITC). The cleanliness is preferably 95% or more.

As used herein, the term "standard value" refers to a proportion of a scaffold (for example, a concave portion or a convex portion) into which a fluorescent molecule is introduced before removing a template particle or after removing a template particle with respect to the number of template particles immobilized on a substrate. The standard value is preferably 80% or more.

As used herein, the term "substituent derived from a modifier" refers to a substituent present on a modifier or a group having a structure in which a substituent derived from a modifier can react and change. Examples thereof include representative substituents an amino group (primary to quaternary, aliphatic, aromatic, pyridine, hydrazide, guanidine, or amidine), an acidic group (a carboxy group, a sulfonic acid group, or a phosphate group), a coordinate binding group (an NTA group, a His-tag, or the like), a carbonyl group (an aldehyde group or a ketone group), a boronyl group, a cis-diol group (catechol-containing, sugar chain structure), a thiol group, a biotin group, a desthiobiotin group, a peptide ligand (a glutathione group or an RGD group), and a group reversible binding group (a disulfide, pyridyl disulfide, imine, oxime, hydrazone, boronic acid cyclic ester, carboxylic acid ester, carboxylic acid thioester, or silyl group). In addition, a substituent derived from one or a plurality of modifiers may also be included.

As used herein, the term "substrate for producing a sensor for direct analysis" refers to a substrate that includes particles and, if necessary, a polymer matrix present on the substrate and can modify a substance required for measurement on the disposed particles.

As used herein, the term "blocking agent" refers to a substance that prevents non-specific adsorption other than the polymer matrix. Examples of the blocking agent include, but are not limited to, a protein such as bovine serum albumin (BSA) and a non-protein such as a protein free blocking buffer.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description in the present disclosure. It is also understood that the following embodiments of the present disclosure may be used alone or in combination.

### <Modifier integrated particle>

In one aspect, the present disclosure provides a particle including a particle core; and a modifier integrated with the particle core by an intermolecular chemical bonding that is separable under conditions that do not damage the particle core.

In one aspect, the present disclosure provides a particle (C) including (A) a particle core and (B) a modifier, in which the particle core includes (A1) a particle core core and (A2) a functional group-containing moiety present on the particle core, the modifier includes (B-1) a first functional group capable of binding to the particle core and capable of forming a chemical bond between molecules, and (B-2) a second functional group that imparts properties desired in the sensor for analysis, where the functional group of (B-1) and the binding group of (B-2) are the same as or different from each other, and the particle core and the modifier are integrated with the particle core by a chemical bonding between the molecules by the separable functional groups.

In another aspect, there is provided a particle for producing a substrate for producing a sensor for analysis, and the particle includes a particle core, and a modifier integrated with the core by an intermolecular chemical bonding that is separable under conditions that do not damage the substrate.

In another aspect, there is provided a particle for producing a substrate for producing a sensor for analysis, and the particle includes a particle core, and a modifier integrated with the core by an intermolecular chemical bonding.

In embodiments of the particle of the present disclosure, the modifier can be any substance that is integrated with the particle core by a non-covalent intermolecular chemical bonding and/or can be any polymer that can be integrated with the particle core.

In one embodiment of the particle of the disclosure, the modifier is not a monomer that can be covalently bound to the particle core.

In one embodiment, the intermolecular chemical bonding that is separable under conditions that do not damage the particle core includes a non-covalent bonding or a partial covalent bonding. Whether or not the covalent bonding is preferable in the present disclosure can be determined by checking whether or not there may be at least one condition under which the covalent bonding can be separated when the particle core and the modifier are tried to be separated under conditions that do not damage the particle core. That is, when at least one condition that does not damage the particle core is found, the covalent bonding is included in the covalent bonding employed in the combination of the particle core and the modifier.

In one embodiment, the intermolecular chemical bonding that is separable under conditions that do not damage the substrate (for example, a substrate for producing a sensor for analysis) includes a non-covalent bonding or a partial covalent bonding. Whether or not the covalent bonding is preferable in the present disclosure can be determined by checking whether or not there may be at least one condition under which the covalent bonding can be separated when the particle core and the modifier are tried to be separated under conditions that do not damage the intended substrate. That is, when at least one condition that does not damage the intended substrate is found, the covalent bonding is included in the covalent bonding employed in the combination of the particle core and the modifier.

It is advantageous that the template substance such as the particle of the present disclosure can introduce various functional groups depending on the scaffold (for example, a concave portion or a convex portion) to be formed. Conventionally, it is required to introduce these functional groups with a specific strong covalent bonding, and it takes time and labor to prepare a template substance. The present disclosure provides a core-shell template molecule in which a required functional group is integrated in advance as a modifier, and the modifier is bound to a substance serving as a core by an intermolecular chemical bonding that is separable under conditions that do not damage the substrate for measurement to form a shell. The core-shell template molecule is stably immobilized on the substrate when forming a polymer matrix around the core-shell template molecule, but after the formation of the polymer matrix, the template substance that could not be removed conventionally without cleaning under conditions strong enough to dissolve the template substance can be removed by cleaning for several minutes.

The particle core used in the present disclosure is not particularly limited as long as it can be used as a template in molecular imprinting, and examples thereof include an artificially produced inorganic particle and organic particle. Examples of the inorganic particle include a metal (gold, silver, platinum, tin-doped indium oxide (ITO), antimony-doped tin oxide (ATO), or the like), an oxide of a metal (iron oxide, aluminum oxide, copper oxide, titanium oxide, zinc oxide, zirconium oxide, cerium oxide, cobalt oxide, or the like), graphene, graphene oxide, a carbon nanotube, nanodiamond, a nitride, a fluoride, a sulfide, a boride, a complex compound thereof, and hydroxyapatite, and preferably include silicon dioxide (silica). In addition, examples of the organic particle include a latex cured product, dextran, chitosan, polylactic acid, poly(meth)acrylic acid, polymethyl methacrylate, a poly(lactic-co-glycolic acid) (PLGA) copolymer, polystyrene, and polyethyleneimine.

The modifier used in the present disclosure is a chemically bondable substance between a particle core and a molecule, and examples thereof include a substance capable of containing various functional groups in a structure. Examples of the functional group include representative substituents an amino group (primary to quaternary, aliphatic, aromatic, pyridine, hydrazide, guanidine, or amidine), an acidic group (a carboxy group, a sulfonic acid group, or a phosphate group), a coordinate binding group (an NTA group, a His-tag, or the like), a carbonyl group (an aldehyde group or a ketone group), a boronyl group, a cis-diol group (catechol-containing, sugar chain structure), a thiol group, a biotin group, a desthiobiotin group, a peptide ligand (a glutathione group or an RGD group), and a group reversible binding group (a disulfide, pyridyl disulfide, imine, oxime, hydrazone, boronic acid cyclic ester, carboxylic acid ester, carboxylic acid thioester, or a silyl group).

The synthesis of the modifier can be performed as follows: RAFT polymerization was performed using a monomer, a RAFT agent, a polymerization initiator, and a solvent to synthesize a polymer. A modifier is synthesized by condensation of primary amino groups in the polymer with methacrylic acid. Alternatively, the synthesis of the modifier can also be performed by the following method: the modifier is synthesized by modifying a polymerizable functional group in a compound having a reversible linking group and a group capable of chemically bonding between the particle core and the molecule, such as cystamine.

In the particle of the present disclosure, the modifier is an integrated particle. The particle core of the present disclosure and a particle including a modifier integrated with the particle core by an intermolecular chemical bonding that is separable under conditions that do not damage the particle core can be provided by integrating the modifier with the particle core under a condition that allows an intermolecular chemical bonding. For example, the integration can be implemented as follows.
1) A dispersion of a particle core and a modifier are mixed, stirred and reacted to purify the particle of the present disclosure.
2) A dispersion of a particle core and a modifier are mixed, stirred, reacted, and centrifuged to remove reactants in the solution and purify the particle of the present disclosure.
3) The particle core is placed on a substrate and a modifier is added thereto. or
4) The modifier is immobilized (adsorbed) on the substrate, the particle core is added thereto, and the modifier is further added.

Binding by an intermolecular chemical bonding that is separable under conditions that do not damage the particle core is possible by integrating the modulator and the particle core by a non-covalent bonding, or by a covalent bonding of a modifier that is a polymer.

In one embodiment, the modifier contains a substituent capable of binding to the substrate. In one embodiment, a substituent capable of binding to a substrate can form a chemical bond between a functional group present on the substrate and a molecule by a covalent bonding or a non-covalent bonding. In one embodiment, the non-covalent bonding may be an electrostatic interaction. In one embodiment, the substituent capable of binding to the substrate may be a cationic functional group such as a primary amine, a secondary amine, a tertiary amine, or a quaternary amine.

In one embodiment, the modifier has a polymerizable functional group. In one embodiment, the polymerizable functional group may be a vinyl group, an allyl group, an epoxy group, a (meth)acryloyl group, or a (meth)acrylamide group, and preferably a (meth)acryloyl group.

In one embodiment, the modifier contains a group for binding a specific binding molecule to a target substance and a group for binding a signal substance; a group for binding a specific binding molecule to a target substance and a signal substance; or a reversible linking group.

In one embodiment, the group for binding a specific binding molecule to a target substance may be an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group, and may be preferably a biotin group, a nickel-nitrilotriacetic acid derived group, a thiol group, or a pyridyl disulfide group. In one embodiment, the group for binding a signal substance may be an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group, and may be preferably a biotin group, a nickel-nitrilotriacetic acid derived group, or a thiol or pyridyl disulfide group. In one embodiment, the signal substance may be a fluorescent molecule, a radioactive element-containing substance, a magnetic substance, or an enzyme, and may be preferably a fluorescent molecule. In one embodiment, the reversible linking group may be a disulfide bond, a boronic acid cis-diol ester, a boronic acid cyclic ester bond, an imino binding group, a carboxylic acid ester group, a carboxylic acid thioester group, an oxime group, a hydrazone binding group, an avidin-biotin bond, an acetal group, a coordination bond, a hydrogen bond, or a hydrophobic bond, and may be preferably a disulfide bond or a coordination bond.

In one embodiment, the particle core is an inorganic particle or an organic particle. In one embodiment, the inorganic particle may be a metal (gold, silver, platinum, tin-doped indium oxide (ITO), antimony-doped tin oxide (ATO), or the like), an oxide of a metal (iron oxide, aluminum oxide, copper oxide, titanium oxide, zinc oxide, zirconium oxide, cerium oxide, cobalt oxide, or the like), graphene, graphene oxide, a carbon nanotube, nanodiamond, a nitride, a fluoride, a sulfide, a boride, a complex compound thereof, or hydroxyapatite, and may be preferably silicon dioxide (silica). In one embodiment, the organic particle may be a latex cured product, dextran, chitosan, polylactic acid, poly(meth)acrylic acid, polymethyl methacrylate, a poly(lactic-co-glycolic acid) copolymer (PLGA), polystyrene, or polyethyleneimine.

In one embodiment, the particle core can be integrated with the modifier by mixing.

### <Substrate for producing sensor for analysis>

In one aspect, the present disclosure provides a kit for producing a substrate for producing a sensor for analysis, the kit including: a particle including the particle core of the present disclosure and a modifier integrated with the particle core by an intermolecular chemical bonding that is separable under conditions that do not damage the particle core; a substrate; and a raw material for a polymer matrix.

Here, as the particle, any embodiment described in the section of the modifier-integrated particle can be employed. As the substrate, any substance may be used as long as it is a raw material suitable for the intended analysis. A material of the substrate may be, for example, a material selected from the group consisting of a metal, glass, silicon dioxide, silicon, a resin, and a combination thereof. Examples of the metal include, but are not limited to, gold, silver, copper, aluminum, tungsten, and molybdenum. Examples of the resin include, but are not limited to, poly(meth)acrylate, polystyrene, an acrylonitrile-butadiene-styrene copolymer (ABS), polycarbonate, polyester, polyethylene, polypropylene, nylon, polyurethane, a silicone resin, a fluororesin, a methylpentene resin, a phenol resin, a melamine resin, an epoxy resin, and a vinyl chloride resin.

The raw material of the polymer matrix may be any material that is suitable for the intended analysis. In general, the material of the polymer matrix is sufficient to contain at least one monomer and at least one suitable polymerization initiator, but in addition to these, for example, an additional monomer (which may be partially polymerized), an additional polymerization initiator, a crosslinking agent, a RAFT agent, a catalyst, a reducing agent, a solvent, and the like can be contained. Examples of the monomer include, but are not limited to, styrene, N-isopropylacrylamide, and 2-methacryloyloxyethyl phosphorylcholine. Examples of the polymerization initiator include, but are not limited to, 2,2'-azobis(isobutyronitrile) (AIBN) and ethyl α-bromoisobutyrate. Examples of the crosslinking agent include, but are not limited to, a melamine compound, a guanamine compound, a glycoluril compound, N,N'-methylenebisacrylamide, and (tri, tetra, penta, hexa, or poly)ethylene glycol dimethacrylate. Examples of the RAFT agent include, but are not limited thereto, benzyl benzodithioate and 2-cyano-2-[(dodecylsulfanylthiocarbonyl)sulfanyl]propane. Examples of the catalyst include CuBr₂, but are not limited thereto. Examples of the reducing agent include ascorbic acid, but are not limited thereto. The solvent is selected from those generally known as solvents without particular limitation, and examples thereof include pure water, a buffer solution, MeOH, EtOH, DMA, and DMF, but are not limited thereto.

In one embodiment, the particle including a particle core and a modifier integrated with the particle core by an intermolecular chemical bonding that is separable under conditions that do not damage the particle core of the present disclosure can be used for producing a substrate for producing a sensor for analysis.

In one embodiment, the present disclosure provides a method for producing a particle of the present disclosure, the particle including a particle core; and a modifier integrated with the particle core by an intermolecular chemical bonding that is separable under conditions that do not damage the particle core. The method includes: A) a step of providing a particle core; B) a step of providing a modifier integrated with the particle core by an intermolecular chemical bonding that is separable under conditions that do not damage the particle core; and C) a step of subjecting the particle core and the modifier to a condition under which the particle core and the modifier are integrated. The condition for integration here may be advantageously an intermolecular chemical bonding that allows the particle core and the modifier to be separated under conditions do not damage the substrate. When the particles are used for a sensor for analysis or a substrate for producing a sensor for analysis, the conditions of integration may be advantageously an intermolecular chemical bonding capable of separating the substrate and the modifier under conditions that do not damage the substrate. It is understood that the particle core and the modifier and the conditions under which the particle core and the modifier are integrated can adopt any embodiment described elsewhere herein, for example, in the sections such as <Particle integrated with modifier> and <Production of particles for producing substrate for producing sensor for analysis>.

In one embodiment, the present disclosure provides a method for producing a particle of the present disclosure, the particle including a particle core; and a modifier integrated with the particle core by an intermolecular chemical bonding that is separable. The method includes: A) a step of providing a particle core; B) a step of providing a modifier integrated with the particle core by an intermolecular chemical bonding that is separable under; and C) a step of subjecting the particle core and the modifier to a condition under which the particle core and the modifier are integrated. The condition for integration here may be advantageously an intermolecular chemical bonding between the particle core and the modifier, and the substrate. When the particles are used for a sensor for analysis or a substrate for producing a sensor for analysis, the conditions of integration may be advantageously an intermolecular chemical bonding capable of separating the substrate and the modifier. It is understood that the particle core and the modifier and the conditions under which the particle core and the modifier are integrated can adopt any embodiment described elsewhere herein, for example, in the sections such as <Particle integrated with modifier> and <Production of particles for producing substrate for producing sensor for analysis>.

### <Substrate for producing sensor for analysis and sensor for analysis production>

In another aspect, the present disclosure provides a method for producing a substrate for producing a sensor for analysis. The method includes A) a step of providing a particle of the present disclosure including a particle core and a modifier integrated with the particle core by an intermolecular chemical bonding that is separable under conditions that do not damage the particle core; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a scaffold (for example, a concave portion or a convex portion) by subjecting the substrate to a condition under which the particles are dissociated from the substrate. It is understood that the particle core, the modifier, and the integration can adopt any embodiment described elsewhere herein, for example, in the sections such as <Particle integrated with modifier> and <Production of particles for producing substrate for producing sensor for analysis>.

In another aspect, the present disclosure provides a method for producing a substrate for producing a sensor for analysis. The method includes A) a step of providing a particle of the present disclosure including a particle core and a modifier integrated with the particle core by an intermolecular chemical bonding; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a scaffold (for example, a concave portion or a convex portion) by subjecting the substrate to a condition under which the particles are dissociated from the substrate. It is understood that the particle core, the modifier, and the integration can adopt any embodiment described elsewhere herein, for example, in the sections such as

### <Particle integrated with modifier> and <Production of particles for producing substrate for producing sensor for analysis>.

The step of providing a particle including a particle core and a modifier integrated with the particle core by an intermolecular chemical bonding that is separable under conditions that do not damage the particle core of the present disclosure can be performed by any method, and for example, a dispersion of the particle core and a modifier are mixed, stirred, and reacted, and then, the particle of the present disclosure is purified. It is understood that the particle of the present disclosure can adopt any embodiment described elsewhere herein, for example, in the sections such as <Particle integrated with modifier> and <Production of particles for producing substrate for producing sensor for analysis>.

The step of providing a particle including a particle core and a modifier integrated with the particle core by an intermolecular chemical bonding of the present disclosure can be performed by any method, and for example, a dispersion of the particle core and a modifier are mixed, stirred, and reacted, and then, the particle of the present disclosure is purified. It is understood that the particle of the present disclosure can adopt any embodiment described elsewhere herein, for example, in the sections such as <Particle integrated with modifier> and <Production of particles for producing substrate for producing sensor for analysis>.

In the present disclosure, the step of disposing of the particles on the substrate can be performed by any method. For example, the particles of the present disclosure are dispersed in a solution, dropped on the substrate, and disposed by being allowed to stand.

In the present disclosure, the step of providing of the raw material of the polymer matrix to the substrate on which the particles are immobilized can be performed by any method, for example, by adding a polymerizable monomer and providing a polymerizable functional group and a polymerizable monomer derived from the modifier as a substrate.

In the present disclosure, the step of forming of the substrate on which the polymer matrix is disposed by subjecting the substrate to a condition under which the polymer matrix is polymerized can be performed by any method. For example, a polymerizable monomer is added, a polymerizable functional group and a polymerizable monomer derived from the modifier are used as a substrate, and a polymer for a part of the surface of the particle of the present disclosure is synthesized using a polymerization initiating group as a polymerizable initiator. As a result, a substrate surface having a scaffold (for example, a concave portion or a convex portion) can be formed.

In the present disclosure, the step of forming of the scaffold (for example, a concave portion or a convex portion) by subjecting the substrate to a condition under which the particles are dissociated from the substrate can be performed by any step, and for example, the particles of the present disclosure can be removed by cleaving a reversible linking group.

In another aspect, the present disclosure provides a method for producing a sensor for analysis. The method includes A) a step of providing a particle of the present disclosure including a particle core and a modifier integrated with the particle core by an intermolecular chemical bonding that is separable under conditions that do not damage the particle core; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; E) a step of forming a scaffold (for example, a concave portion or a convex portion) by subjecting the substrate to a condition under which the particles are dissociated from the substrate; and F) a step of binding a substance required for measurement to the scaffold (for example, a concave portion or a convex portion). It is understood that the method can adopt any embodiment described elsewhere herein, for example, in the sections such as <Particle integrated with modifier> and <Production of particles for producing substrate for producing sensor for analysis>.

In another aspect, the present disclosure provides a method for producing a sensor for analysis. The method includes A) a step of providing a particle of the present disclosure including a particle core and a modifier integrated with the particle core by an intermolecular chemical bonding; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; E) a step of forming a scaffold (for example, a concave portion or a convex portion) by subjecting the substrate to a condition under which the particles are dissociated from the substrate; and F) a step of binding a substance required for measurement to the scaffold (for example, a concave portion or a convex portion). It is understood that the method can adopt any embodiment described elsewhere herein, for example, in the sections such as <Particle integrated with modifier> and <Production of particles for producing substrate for producing sensor for analysis>.

In one embodiment, in the step E, the condition under which the particles are dissociated from the substrate is a condition under which a reversible linking group is cleaved and a reversible binding group is produced.

In another embodiment, the method of the present disclosure further includes, after the step E, a step of converting the reversible binding group in the scaffold (for example, a concave portion or a convex portion) into a group for binding a specific binding molecule to a target substance or a group for binding a signal substance.

In one embodiment, the step of binding of the substance required for measurement to the scaffold (for example, a concave portion or a convex portion) can be performed by any method, for example, by modifying a cleaved reversible linking group produced in the scaffold (for example, a concave portion or a convex portion) by reduction with a group for binding a specific binding molecule or a group for binding a signal substance.

### <Substrate for producing sensor for analysis>

In another aspect, the present disclosure provides a substrate for producing a sensor for analysis produced by a method including A) a step of providing a particle of the present disclosure including a particle core and a modifier integrated with the particle core by an intermolecular chemical bonding that is separable under conditions that do not damage the particle core; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a scaffold (for example, a concave portion or a convex portion) by subjecting the substrate to a condition under which the particles are dissociated from the substrate. It is understood that the substrate can adopt any embodiment described elsewhere herein, for example, in the sections such as <Particle integrated with modifier>, <Production of particles for producing sensor for analysis>, <Production of substrate for producing sensor for analysis and sensor for analysis>.

In another aspect, the present disclosure provides a substrate for producing a sensor for analysis produced by a method including A) a step of providing a particle of the present disclosure including a particle core and a modifier integrated with the particle core by an intermolecular chemical bonding; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a scaffold (for example, a concave portion or a convex portion) by subjecting the substrate to a condition under which the particles are dissociated from the substrate. It is understood that the substrate can adopt any embodiment described elsewhere herein, for example, in the sections such as <Particle integrated with modifier>, <Production of particles for producing sensor for analysis>, <Production of substrate for producing sensor for analysis and sensor for analysis>.

In one embodiment, the present disclosure provides a substrate for producing a sensor for analysis, the substrate including: A) a substrate; B) a polymer matrix disposed on the substrate, the polymer matrix having a scaffold (for example, a concave portion or a convex portion) that at least partially fits into a molecule to be detected, if necessary; and C) a binding group disposed on the scaffold (for example, a concave portion or a convex portion).

In another aspect, the present disclosure provides a sensor for analysis, the sensor including: A) a substrate; B) a polymer matrix disposed on the substrate, the polymer matrix having a scaffold (for example, a concave portion or a convex portion) that at least partially fits into a molecule to be detected, if necessary; C) a group for binding a signal substance or a signal substance disposed on the scaffold (for example, a concave portion or a convex portion), if necessary; and D) a group for binding a specific binding molecule that binds to a molecule to be detected. It is understood that the sensor can adopt any embodiment described elsewhere herein, for example, in the sections such as <Particle integrated with modifier>, <Production of particles for producing sensor for analysis>, <Production of substrate for producing sensor for analysis and sensor for analysis>.

In one embodiment, the polymer matrix used in the present disclosure preferably has a cleanliness of 95% or more (an extent that cannot be achieved with hydrofluoric acid). In a case where the silica particles are insufficiently removed by hydrofluoric acid, it can be evaluated that the cleanliness is insufficient by performing elemental analysis (EDX) of the substrate surface. Alternatively, in addition to the cleanliness, it may be possible to distinguish by the production method in the yield of functionalization of the scaffold (for example, a concave portion or a convex portion). Examples of a preferred value of the cleanliness include 95% or more, 96% or more, 97% or more, 98% or more, and 99% or more.

In one embodiment, the present disclosure provides a measurement substrate having a standard value of the scaffold (for example, a concave portion or a convex portion) in the substrate of the present disclosure of 80% or more. As used herein, the term "standard value" refers to a proportion of the number of scaffolds (for example, concave portions or convex portions) into which a fluorescent molecule (or a fluorescent molecule-modified avidin) is introduced after a template molecule is removed using the number of template particles immobilized on the substrate as a parameter. Examples of a preferred value of the standard value include 80% or more, 85% or more, 90% or more, and 95% or more.

In one embodiment, the present disclosure provides a measurement substrate in which a substituent derived from a modifier is bonded onto the substrate. In one embodiment, a measurement substrate is provided in which the substituent derived from the modifier is an amino group (primary to quaternary, aliphatic, aromatic, pyridine, hydrazide, guanidine, or amidine), an acidic group (a carboxy group, a sulfonic acid group, or a phosphate group), a coordinate binding group (an NTA group, a His-tag, or the like), a carbonyl group (an aldehyde group or a ketone group), a boronyl group, a cis-diol group (catechol-containing, sugar chain structure), a thiol group, a biotin group, a desthiobiotin group, a peptide ligand (a glutathione group or an RGD group), a reversible binding group (a disulfide, pyridyl disulfide, imine, oxime, hydrazone, or boronic acid cyclic ester), or a covalent bonding that can be chemically cleaved relatively easily (carboxylic acid ester, carboxylic acid thioester, or a silyl group).

### <E) Direct substrate>

In another aspect, the present disclosure provides a substrate for producing a sensor for direct analysis. The substrate for producing a sensor for direct analysis includes A) a substrate, B) the particle of the present disclosure, and C) a polymer matrix on which the particles are disposed, the polymer matrix being disposed on the substrate. The direct substrate can surprisingly be used as a sensor as it is. In addition, it is understood that the substrate can adopt any embodiment described elsewhere herein, for example, in the sections such as <Particle integrated with modifier>, <Production of particles for producing sensor for analysis>, <Production of substrate for producing sensor for analysis and sensor for analysis>.

In another aspect, the present disclosure provides a sensor for direct analysis. This sensor for direct analysis includes: A) a substrate; B) a particle including a particle core of the present disclosure and a modifier integrated with the particle core by an intermolecular chemical bonding that is separable under conditions that do not damage the particle core; C) a polymer matrix on which the particles are disposed, the polymer matrix being disposed on the substrate, if necessary; D) a blocking agent present on the outermost layer, if necessary; and E) a substance required for measurement present on the particle, if necessary. It is understood that the sensor can adopt any embodiment described elsewhere herein, for example, in the sections such as <Particle integrated with modifier>, <Production of particles for producing sensor for analysis>, <Production of substrate for producing sensor for analysis and sensor for analysis>.

In another aspect, the present disclosure provides a sensor for direct analysis. This sensor for direct analysis includes: A) a substrate; B) a particle including a particle core of the present disclosure and a modifier integrated with the particle core by an intermolecular chemical bonding; C) a polymer matrix on which the particles are disposed, the polymer matrix being disposed on the substrate, if necessary; D) a blocking agent present on the outermost layer, if necessary; and E) a substance required for measurement present on the particle, if necessary. It is understood that the sensor can adopt any embodiment described elsewhere herein, for example, in the sections such as <Particle integrated with modifier>, <Production of particles for producing sensor for analysis>, <Production of substrate for producing sensor for analysis and sensor for analysis>.

In one embodiment, the blocking agent may be a protein such as bovine serum albumin (BSA) or a non-protein such as a protein free blocking buffer, and preferably a protein free blocking buffer.

In one embodiment, the substance required for measurement is a substance having a group for binding a specific binding molecule, a group for binding a signal substance, or a signal substance. In one embodiment, the group for binding a specific binding molecule may be an amino group, a carboxyl group, a sugar group (a cis-diol group), a boronyl group, a thiol group, a maleimide group, a carbonyl group, an aldehyde group, an aminooxy group, or a hydroxyl group, and preferably a sugar group (a cis-diol group) or a thiol group. In one embodiment, the group for binding a signal substance is an amino group, a carboxyl group, a sugar group (a cis-diol group), a boronyl group, a thiol group, a maleimide group, a carbonyl group, an aldehyde group, an aminooxy group, or a hydroxyl group, and may be preferably a sugar group (a cis-diol group) or a thiol group. In one embodiment, the signal substance is a fluorescent molecule, a radioactive element-containing substance, a magnetic substance, or an enzyme, and may be preferably a fluorescent molecule.

In one embodiment, the present disclosure provides a method for producing a substrate for producing a sensor for direct analysis. The method includes A) a step of providing a particle of the present disclosure including a particle core and a modifier integrated with the particle core by an intermolecular chemical bonding that is separable under conditions that do not damage the particle core; B) a step of disposing the particles on a substrate; C) a step of providing a raw material of a polymer matrix to the substrate on which the particles are immobilized, if necessary; and D) a step of forming a substrate on which the polymer matrix is disposed by subjecting the substrate to a condition under which the polymer matrix is polymerized, if necessary.

In one embodiment, the present disclosure provides a method for producing a sensor for direct analysis. The method includes A) a step of providing a particle of the present disclosure including a particle core and a modifier integrated with the particle core by an intermolecular chemical bonding that is separable under conditions that do not damage the particle core; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and F) a step of binding a substance required for measurement to the particle, if necessary.

In one embodiment, the present disclosure provides a method for producing a sensor for direct analysis. The method includes A) a step of providing a particle of the present disclosure including a particle core and a modifier integrated with the particle core by an intermolecular chemical bonding; B) a step of disposing the particles on a substrate; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and F) a step of binding a substance required for measurement to the particle, if necessary.

### (Production method)

The step for producing the sensor for analysis can be performed as follows. A dispersion of a particle core and a modifier are mixed, stirred and reacted to purify the particle of the present disclosure. The particles of the present disclosure are dispersed in a solution, dropped on a substrate, and disposed by being allowed to stand. A polymerizable monomer is added, and a polymerizable functional group and a polymerizable monomer derived from the modifier are used as a substrate. A polymer for a portion of the surface of the particle of the present disclosure is synthesized using a polymerizable initiating group as a polymerizable initiator. As a result, a substrate surface having a scaffold (for example, a concave portion or a convex portion) is formed. The reversible linking group is cleaved to remove the particles of the disclosure. The reversible linking group that is cleaved and produced in the scaffold (for example, a concave portion or a convex portion) is modified with the group for binding a specific binding molecule and the group for binding a signal substance.

### (Use)

The sensor for analysis of the present invention is used for sensing an object to be detected. The more specific use is determined depending on the type of the specific binding group, and for example, the sensor for analysis can be used for the purpose of diagnosis or therapeutic monitoring based on renal function, liver function, and the presence or absence or a degree of inflammation; and the presence or absence or a degree of tumor. The technique of the present invention can be applied to all sensing chip production based on hole formation using molecular imprinting, and is not required for mass production of sensing sub chips.

The sensor for analysis of the present disclosure can be used for the following use.

The sensor for analysis can be used for in vivo/in vitro imaging of an object to be measured, a therapeutic application, a virus sensor in a human body or environment, and analysis of food, an agricultural product, and livestock.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present disclosure, but is limited only by the claims.

### Examples

In the present examples, examples related to production and use of the compound of the present disclosure will be described.

Note that CKX31 (OLYMPUS, Tokyo, Japan) was used for microscopic observation of cells, KUBOTA2800 (KUBOTA, Tokyo, Japan) was used for a centrifuge, CO2 WATER JACKETED INCUBATOR (Thermo Fisher Scientific Inc, Massachusetts, USA) was used for an incubator, KS-243 (TOMY SEIKO Co,Ltd., Tokyo, Japan) was used for an autoclave, and Bio Clean Bench (ORIENTAL GIKEN INC, Tokyo, Japan) was used for a clean bench. qNano Gold (Izon Science Ltd., Christchurch, New Zealand) was used for exosome concentration measurement. In addition, UV Ozone Cleaner (BioForce Nanosciences, Inc.) was used for a UV ozone treatment of a gold substrate, a fluorescence microscope (Olympus Corporation, Tokyo, Japan) with an SIC automatic dispensing device (SYSTEM INSTRUMENTS Co., Ltd., Tokyo, Japan) was used for fluorescence measurement, and Andor SOLIS (Andor Technology Ltd, Belfast, Northern Ireland) was used as spectroscopic software.

In addition, MALDI-TOF/MS (MALDI-7090, Shimadzu Corporation) was used for MALDI-TOF-MS, MALDI Solutions (Shimadzu Corporation) was used for analysis software, and protein calibration standard I (Bruker Corporation) was used for calibration. Sinapinic acid was used as a matrix. For a CD spectrum, a J-725 type circular dichroism spectropolarimeter (JASCO Corporation, Tokyo, Japan) was used. A pH was measured with a tabletop pH meter F-52 (HORIBA, Kyoto, Japan) for buffer preparation. Fluorescence spectrum measurement was performed using a fluorescence spectrophotometer F-2500 (Tokyo, Japan) manufactured by Hitachi High-Tech Corporation. As an ultrafiltration membrane used for ultrafiltration, Amicon Ultra-4 (10 kDa) was used. For absorption spectrum measurement, a Thermo ScientificTM Nano drop TM One ultra trace ultraviolet-visible spectrophotometer (Thermo Fisher) was used. An average particle size and a polydispersity index (PDI) of the produced particles were measured by a dynamic light scattering (DLS) method using ZETASIZER NANO-ZS MAL500735 (Malvern, UK), and Zetasizer software was used as analysis software. A transmission electron microscope (JEM-1230, manufactured by JEOL Ltd.) was used for TEM analysis.

### (Example 1: Synthesis of modifier)

### 1-1. Experimental operation

### 1-1-1. Synthesis of polymer

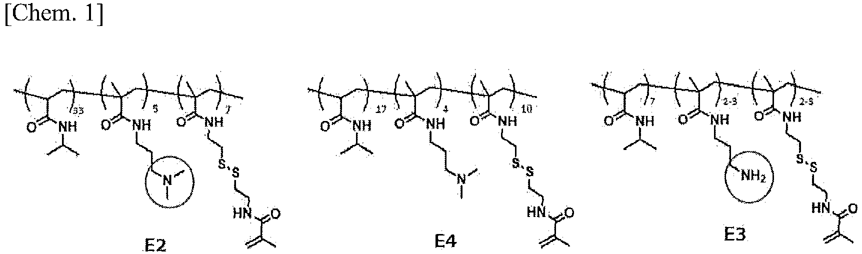

### Various synthesized cationic polymers

### 1-1-1. Synthesis of polymer E2

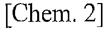

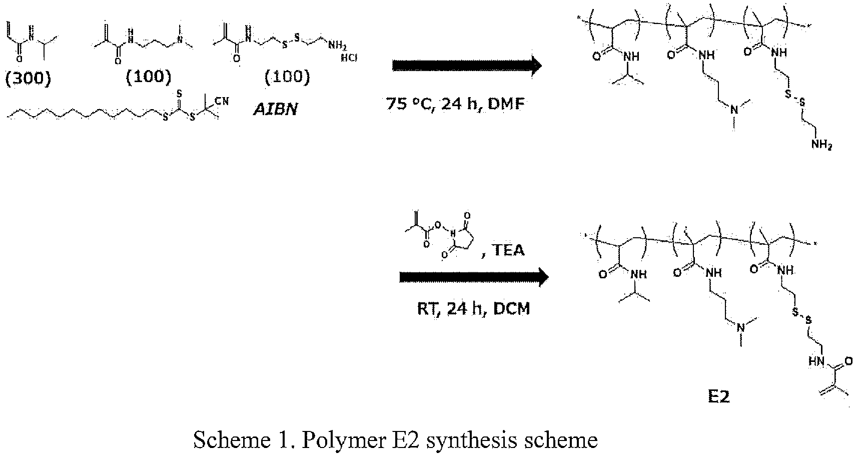

Reagents of the recipes shown in Table 1-1 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer (E2-0). (Yield: 100 mg)

100 mg of the obtained polymer was dispersed in a dichloromethane/dioxane = 1/1 (v/v) solution (10 mL), 92 mg (0.5 mmol) of N-succinimidyl methacrylate and 140 µL (1.0 mmol) of triethylamine (TEA) were added thereto, and stirring was performed at room temperature for 24 hours. The solvent after the reaction was distilled off under reduced pressure with an evaporator, a small amount of dichloromethane was added to the residue, and a precipitate generated by further adding an excessive amount of hexane was collected (this operation was performed twice). The obtained precipitate was vacuum-dried to obtain a polymer E2. (Yield: 101 mg)

A composition of the obtained polymer E2 was estimated from ¹H-NMR (in DMSO-d6) (AVANCE-500, Bruker). (FIG. 1-1)

**[Table 1-1]**

| Table 1-1 Polymer E2 synthesis recipe | | |
|---|---|---|
| | Sample | Concentration |
| Main raw material | N-isopropylacrylamide (NIPAm) | 300 mM |
| Cationic site | N-(3-Dimethylaminopropyl)methacrylamide hydrochloride | 100 mM |
| Modified site | Cys-methacrylamide hydrochloride | 100 mM |
| RAFT agent | 2-Cyano-2-[(dodecylsulfanylthiocarbonyl)sulfanyl]propane | 12.5mM |
| Initiator | 2,2'-Azobis(isobutyronitrile) (AIBN) | 6.25 mM |
| Solvent | DMF | 2 mL |

### 1-1-2. Synthesis of polymer E3

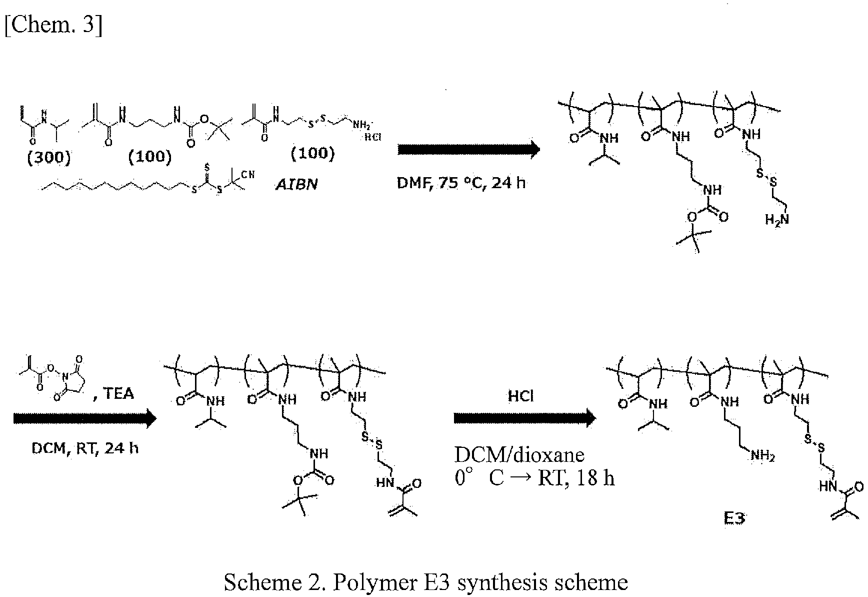

Reagents of the recipes shown in Table 1-2 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer.

### (Yield: 105 mg)

The polymer was dispersed in dichloromethane (10 mL), 110 mg (0.6 mmol) of N-succinimidyl methacrylate and 140 µL (1.0 mmol) of triethylamine (TEA) were added thereto, and stirring was performed at room temperature for 24 hours. A precipitate generated by adding an excessive amount of hexane to the solution after the reaction was collected (this operation was performed twice). The obtained precipitate was vacuum-dried to obtain a polymer E3. (Yield: 115 mg)

The obtained polymer was dispersed in dichloromethane (6 mL), 1.0 mL of 4 N HCl in dioxane was added thereto, and stirring was performed under ice cooling for 2 hours. Thereafter, stirring was further performed at room temperature overnight. A precipitate generated by adding hexane to the solution after the reaction was collected. A small amount of dichloromethane was added to the collected precipitate, an excessive amount of hexane was further added, and the precipitate was washed. The collected precipitate was vacuum-dried. (Yield: 105 mg)

A composition of the obtained polymer E4 was estimated from ¹H-NMR (in DMSO-d6) (AVANCE-500, Bruker). (FIG. 1-2)

**[Table 1-2]**

| Table 1-2 Polymer E3 synthesis recipe | | |
|---|---|---|
| | Sample | Concentration |
| Main raw material | N-isopropylacrylamide (NIPAm) | 300 mM |
| Cationic site | N-Boc-(3-aminopropyl)methacrylamide hydrochloride | 100 mM |
| Modified site | Cys-methacrylamide | 100 mM |
| RAFT agent | 2-Cyano-2-[(dodecylsulfanylthiocarbonyl)sulfanyl]propane | 12.5mM |
| Initiator | 2,2'-Azobis(isobutyronitrile) (AIBN) | 6.25 mM |
| Solvent | DMF | 2 mL |

### 1-1-3. Synthesis of polymer E4

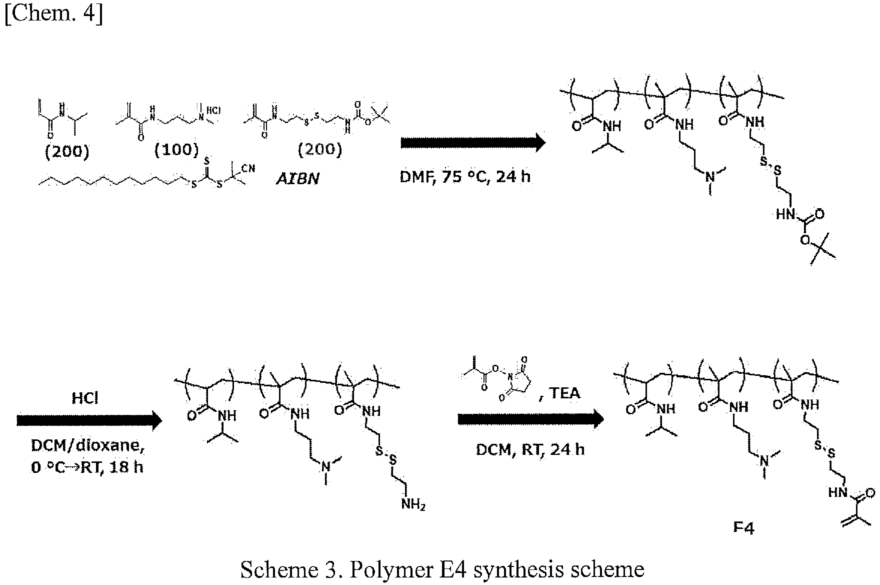

Reagents of the recipes shown in Table 1-3 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer. (Yield: 70 mg)

The obtained polymer was dispersed in dichloromethane (10 mL), 1.0 mL of 4 N HCl in dioxane was added thereto, and stirring was performed under ice cooling for 2 hours. Thereafter, stirring was further performed at room temperature overnight. A precipitate generated by adding hexane to the solution after the reaction was collected. A small amount of dichloromethane was added to the collected precipitate, an excessive amount of hexane was further added, and the precipitate was washed. The collected precipitate was vacuum-dried. Thereafter, the polymer was dispersed in dichloromethane (10 mL), 73 mg (0.4 mmol) of N-succinimidyl methacrylate and 84 µL (0.6 mmol) of triethylamine (TEA) were added thereto, and stirring was performed at room temperature for 24 hours. A precipitate generated by adding an excessive amount of hexane to the solution after the reaction was collected (this operation was performed twice). The obtained precipitate was vacuum-dried to obtain E4. (Yield: 62 mg)

A composition of the obtained E4 was estimated from ¹H-NMR (in DMSO-d6) (AVANCE-500, Bruker). (FIG. 1-3)

**[Table 1-3]**

| Table 1-3 Polymer E4 synthesis recipe | | |
|---|---|---|
| | Sample | Concentration |
| Main raw material | N-isopropylacrylamide (NIPAm) | 200 mM |
| Cationic site | N-(3-Dimethylaminopropyl)methacrylamide hydrochloride | 100 mM |
| Modified site | Cys-methacrylamide hydrochloride | 200 mM |
| RAFT agent | 2-Cyano-2-[(dodecylsulfanylthiocarbonyl)sulfanyl]propane | 12.5mM |
| Initiator | 2,2'-Azobis(isobutyronitrile) (AIBN) | 6.25 mM |
| Solvent | DMF | 2 mL |

### 1.1.3. Synthesis of polymer F1

Reagents of the recipes shown in Table 1-4 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer.

### (Yield: 105.7 mg)

The obtained polymer was dispersed in dichloromethane (5 mL), 34.1 mg (0.19 mmol) of N-succinimidyl methacrylate and 38.9 µL (0.28 mmol) of triethylamine (TEA) were added thereto, and stirring was performed at room temperature for 24 hours. A precipitate generated by adding an excessive amount of hexane to the solution after the reaction was collected (this operation was performed twice). The obtained precipitate was vacuum-dried to obtain F1. (Yield: 19.1 mg)

**[Table 1-4]**

| Table 1-4 Fluorescent polymer F1 synthesis recipe | | |
|---|---|---|
| | Sample | Concentration |
| Main raw material | N-isopropylacrylamide | 275 mM |
| Cationic site | N-(3-Dimethylaminopropyl)methacrylamide hydrochloride | 100 mM |
| Modified site | Cys-methacrylamide hydrochloride | 100 mM |
| Fluorescent site | Methacryloxyethyl thiocarbamoyl rhodamine B | 25 mM |
| RAFT agent | 2-Cyano-2-[(dodecylsulfanylthiocarbonyl)sulfanyl]propane | 12.5mM |
| Initiator | 2,2'-Azobis(isobutyronitrile): V-60 | 6.25 mM |
| Solvent | DMF | 2 mL |

### Synthesis of polymer (AN1)

64 mg of Boc-cystamine metacrylamide, 29 µL of t-butyl acrylate, and 68 mg of NIPAm were dissolved in 1.8 mL of DMF, and finally, 0.2 mL of DMF in which 8.6 mg of a RAFT agent (2-cyano-2-[(dodecylsulfanylthiocarbonyl)sulfanyl]propane) and 2.1 mg of an initiator (AIBN) were dissolved was added thereto, and degassing and replacement with argon were performed to remove dissolved oxygen. Thereafter, a polymerization reaction was performed in an oil bath set at 75°C (24 h). After the reaction, the reaction solution was dropped to an excessive amount of diethyl ether, and a precipitate was collected. (Yield: 51 mg)

The obtained polymer was dissolved in dichloromethane (5 mL) and stirring was performed under ice cooling. 1.5 mL of trifluoroacetic acid was added thereto, and stirring was performed overnight under light-shielding. After the reaction, a precipitate generated by adding diethyl ether was collected, and the obtained precipitate was washed with diethyl ether. (Yield: 22 mg)

The obtained polymer was dispersed in dichloromethane, 15 mg (0.08 mmol) of N-succinimidyl methacrylate and 17 µL (0.12 mmol) of triethylamine were added thereto, and stirring was performed at room temperature overnight. A precipitate generated by adding diethyl ether to the reaction solution was collected. The precipitate was dissolved in a small amount of dichloromethane and precipitated by adding an excessive amount of hexane thereto. This operation was repeated twice, and vacuum-drying was performed to obtain a target polymer (AN1). (Yield: 16 mg)

### (Example 1-2: Binding of polymer to particles (non-covalent bond))

### 1-2-1. Synthesis of E3 conjugated silica NPs

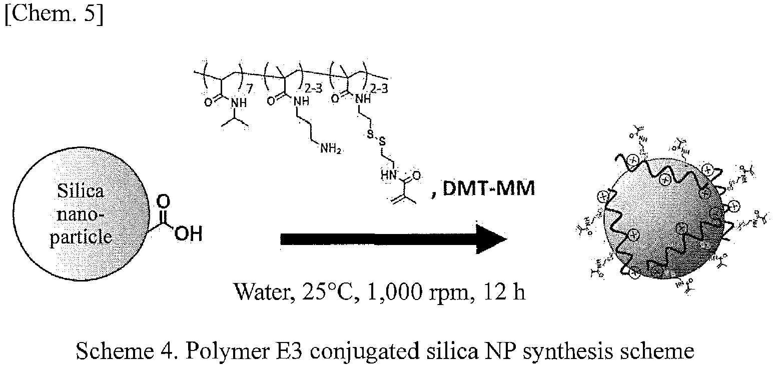

100 µL of a silica nanoparticle dispersion (2.0 × 10¹² particles/mL), 500 µL of a polymer E3 aqueous solution (2.0 mg/mL), and 400 µL of a 4-(4,6-dimethoxy-1,3,5-triazine-2-yl)-4 methylmorpholinium chloride (DMT-MM) aqueous solution (12.5 mM) were mixed, and stirring was performed with a thermoshaker at 25°C and 1,000 rpm for 12 hours. After the reaction, the reaction product in the solution was removed by centrifugation (25°C, 10,000 g, 15 min) × 5. Each supernatant was confirmed by UV-vis measurement (NanoDrop).

According to a similar procedure, the polymer E2 and the polymer E4 were integrated with silica particles.

### 1-2-2. Results

### 1-2-2-1. DLS and Z-potential measurement

20 µL of a silica nanoparticle dispersion (2.0 × 10¹² particles/mL), 100 µL of various cationic polymer aqueous solutions (2.0 mg/mL), and 80 µL of pure water were mixed and thoroughly stirred with a vortex. The solution was diluted 100-fold with pure water to obtain a DLS and Z-potential measurement sample. As a measuring instrument, Zetasizer pro (Marvern Panalytical, UK) was used.

From the results of particle size measurement by DLS (FIG. 1-4 and Table 1-5) and the results of Z-potential measurement (FIG. 1-5 and Table 1-5), it was shown that the produced cationic polymer was adsorbed in the form of silica nanoparticles, and surface characteristics thereof were changed from a negatively charged state to a positively charged state. In addition, since a large change in particle size was not observed before and after the polymer was adsorbed to the silica nanoparticles, it was shown that the silica nanoparticle monodispersity was maintained even after the polymer adsorption.

**[Table 1-5]**

| Table 1-5 DLS measurement and Z-potential measurement for each particle | | |
|---|---|---|
| | Average particle size (nm) | Surface charge (mV) |
| Silica nanoparticle | 208 | -53.2 |
| Silica nanoparticle + E2 | 216 | 56.2 |
| Silica nanoparticle + E4 | 237 | 60.9 |
| Silica nanoparticle + E3 | 220 | 40.5 |

### 1-1. Synthesis of polymer (E101)

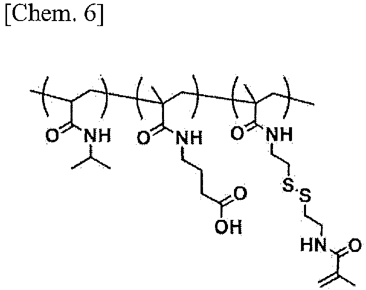

Polymer recipe:

**[Table A-1]**

| | **Mw.** | | |
|---|---|---|---|
| Sample | | Concert tration (mM) | Final liquid volume (mL) |
| *Cystamine monomer* (*N*-[2-[(2-Aminoethyl)dithio]ethyl]-2-methyl-2-propenamide, hydrochloride) | 256.82 | 100 | 1 |
| *4-[(2-Methyl-1-oxo-2-propen-1-yl)amino]butanoic acid* | 171.19 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E101).

### 1-2. Polymer (E101)-particle core complex formation and immobilization on substrate

A polymer (E101) aqueous solution (2 mg/mL) and a particle core dispersion having a basic group on a surface were mixed at 1/1, v/v. The solution was diluted 80-fold with DMF. 4 µL of the solution was dropped on a gold substrate modified with 2-(2-bromoisobutyryloxy)undecyl thiol and amino-EG6 undecanethiol hydrochloride, and the substrate was allowed to stand for 1 hour. Thereafter, the substrate was washed with EtOH to obtain a particle-immobilized substrate.

### 2-1. Synthesis of polymer (E102)

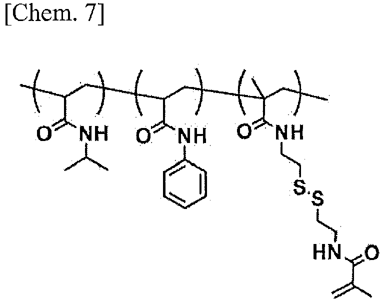

Polymer recipe:

**[Table A-2]**

| | **Mw.** | | |
|---|---|---|---|
| Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | 256.82 | 100 | 1 |
| *N-Phenyl acrylamide* | 147.17 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E102).

### 2-2. Polymer (E102)-particle core complex formation and immobilization on substrate

A polymer (E102) aqueous solution (2 mg/mL) and a particle core dispersion having a hydrophobic group such as a benzene ring on a surface were mixed at 1/1, v/v. The solution was diluted 80-fold with DMF. 4 µL of the solution was dropped on a gold substrate modified with 2-(2-bromoisobutyryloxy)undecyl thiol alone or SAM mixed with undecanethiol, and the substrate was allowed to stand for 1 hour. Thereafter, the substrate was washed with EtOH to obtain a particle-immobilized substrate.

### 3-1. Synthesis of polymer (E 103)

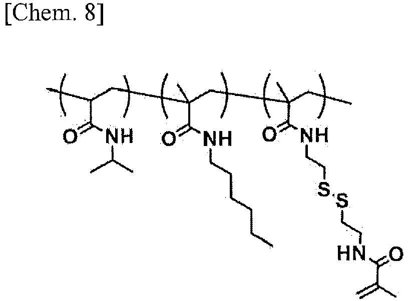

Polymer recipe:

**[Table A-3]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *N*-Hexylmethacrylamide | | 169.26 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E103).

### 3-2. Polymer (E103)-particle core complex formation and immobilization on substrate

A polymer (E103) aqueous solution (2 mg/mL) and a particle core dispersion having a hydrophobic group such as a benzene ring or an alkyl chain on a surface were mixed at 1/1, v/v. The solution was diluted 80-fold with DMF. 4 µL of the solution was dropped on a gold substrate modified with 2-(2-bromoisobutyryloxy)undecyl thiol alone or SAM mixed with undecanethiol, and the substrate was allowed to stand for 1 hour. Thereafter, the substrate was washed with EtOH to obtain a particle-immobilized substrate.

### 4-1. Synthesis of polymer (E104)

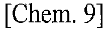

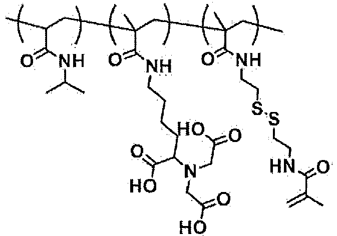

Polymer recipe:

**[Table A-4]**

| | | **Mw.** | | |
|---|---|---|---|---|
| | Sample | | Concentration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *NTA monomer (L-Lysine, N2,N2-bis(carboxymethyl)-N6-(2-methyl-1-oxo-2-propen-1-yl)-)* | | 330.33 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E104).

### 4-2. Polymer (E104)-particle core complex formation and immobilization on substrate

A polymer (E104) aqueous solution (2 mg/mL) and a particle core dispersion having a group capable of forming a coordinate bond such as a His-tag on a surface were mixed at 1/1, v/v. The solution was diluted 80-fold with DMF. A gold substrate modified with 2-(2-bromoisobutyryloxy)undecyl thiol and carboxy-EG6 undecanethiol was immersed in CH₂Cl₂ in which 0.1 M EDC and 0.05 M NHS were dissolved, and the substrate was allowed to stand for 1 hour. The substrate after the reaction was washed with CH₂Cl₂ and dried with nitrogen blow. 4 µL of a polymer (E104)-particle core mixed solution was dropped on the substrate, and the substrate was allowed to stand for 1 hour. Thereafter, the substrate was washed with EtOH to obtain a particle-immobilized substrate.

### 5-1. Synthesis of polymer (E105)

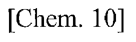

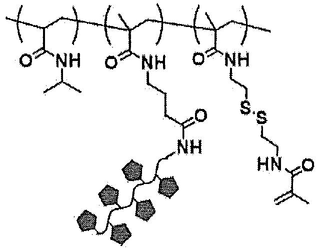

100 mg of the polymer (E101) and 140 µL (1.0 mmol) of TEA were dissolved in CH₂Cl₂ and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E101)-NHS(E112).

10 mg of the polymer prepared in the previous section and 28 µL of TEA were dissolved in DMF (1 mL), and stirring was performed. 3 mg of a synthetic peptide (six histidines were bonded from the N-terminal, three glycines were bonded, and a lysine was bonded to the C-terminal) was added thereto, and stirring was further performed overnight. After the reaction, a small amount of CH₂Cl₂ was added and then an excessive amount of n-hexane was added to generate a precipitate. The precipitate was collected, a small amount of CH₂Cl₂ was added thereto, an excessive amount of n-hexane was further added to wash the precipitate, and then the precipitate was vacuum-dried, thereby obtaining a polymer (E105).

### 5-2. Polymer (E105)-particle core complex formation and immobilization on substrate

A polymer (E105) aqueous solution (2 mg/mL) and a particle core dispersion having a group capable of forming a coordinate bond such as an NTA group on a surface were mixed at 1/1, v/v. The solution was diluted 80-fold with DMF. A gold substrate modified with 2-(2-bromoisobutyryloxy)undecyl thiol and amino-EG6 undecanethiol hydrochloride was immersed in DMSO in which 1 mM N-[5-(4-Isothiocyanatobenzyl)amido-1-carboxypentyl]iminodiacetic acid was dissolved, and the substrate was allowed to stand for 1 hour. The substrate after the reaction was washed with DMSO and EtOH and dried with nitrogen blow. 4 µL of a polymer (E105)-particle core mixed solution was dropped on the substrate, and the substrate was allowed to stand for 1 hour. Thereafter, the substrate was washed with EtOH to obtain a particle-immobilized substrate.

### 6-1. Synthesis of polymer (E106)

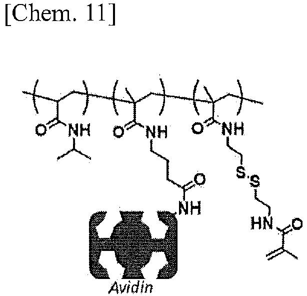

100 mg of the polymer (E101) and 140 µL (1.0 mmol) of TEA were dissolved in CH₂Cl₂ and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E101)-NHS(E112).

10 mg of the polymer prepared in the previous section was dissolved in a 50 mM carbonate buffer (pH 8.5) (1 mL), and stirring was performed. 3 mg of Avidin was added thereto, and stirring was further performed overnight. The reaction solution was placed in a dialysis membrane for 100 kDa, and dialysis was carried out in a phosphate buffer (pH 7.4). After the dialysis, concentration was performed by ultrafiltration to obtain a target solution of the polymer (E106).

### 6-2. Polymer (E106)-particle core complex formation and immobilization on substrate

A polymer (E106) aqueous solution (2 mg/mL) and a particle core dispersion having biotin or a derivative thereof on a surface were mixed at 1/1, v/v. The solution was appropriately diluted with a phosphate buffer. A gold substrate modified with 2-(2-bromoisobutyryloxy)undecyl thiol and amino-EG6 undecanethiol hydrochloride was immersed in DMSO in which 0.1 M EDC and 0.05 M biotin were dissolved, and the substrate was allowed to stand for 1 hour. The substrate after the reaction was washed with DMSO and EtOH and dried with nitrogen blow. 40 µL of a polymer (E105)-particle core mixed solution was dropped on the substrate, and the substrate was allowed to stand for 1 hour. Thereafter, the substrate was washed with a phosphate buffer to obtain a particle-immobilized substrate.

### 7-1. Synthesis of polymer (E107)

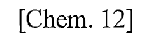

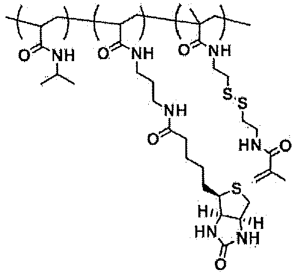

Polymer recipe:

**[Table A-5]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *biotin monomer ((3aS,4S,6aR)-Hexahydro-2-oxo-N-[3-[(1-oxo-2-propen-1-yl)amino]propyl]-1H-thieno[3,4-d]imidazole-4-pentanamide)* | | 354.47 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E107).

### 7-2. Polymer (E107)-particle core complex formation and immobilization on substrate

A polymer (E107) aqueous solution (2 mg/mL) and a particle core dispersion whose surface was modified with Avidin at a ratio of 1/1, v/v. The solution was appropriately diluted with PBS. A gold substrate modified with 2-(2-bromoisobutyryloxy)undecyl thiol and carboxy-EG6 undecanethiol was immersed in CH₂Cl₂ in which 0.1 M EDC and 0.05 M NHS were dissolved, and the substrate was allowed to stand for 1 hour. The substrate after the reaction was washed with CH₂Cl₂ and dried with nitrogen blow. 1 mg/mL of an Avidin solution was dropped on the substrate, and the substrate was allowed to stand for 1 hour. After the reaction, the substrate was washed with a phosphate buffer. A polymer (E107)-particle core mixed solution was dropped on the substrate, and the substrate was allowed to stand for 1 hour. Thereafter, the substrate was washed with a phosphate buffer to obtain a particle-immobilized substrate.

### 8-1. Synthesis of polymer (E108)

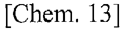

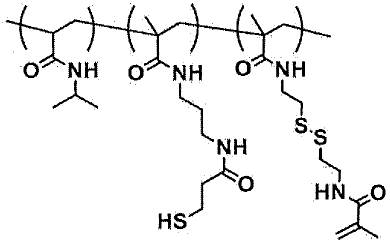

100 mg of the polymer (E3) and 140 µL (1.0 mmol) of TEA were dissolved in CH₂Cl₂ and stirring was performed under ice cooling. 40 mg (0.2 mmol) of EDC and 22 mg (0.2 mmol) of 3-mercaptopropionic acid were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E108).

### 8-2. Polymer (E106)-particle core complex formation and immobilization on substrate

A polymer (E108) aqueous solution (2 mg/mL) and a particle core dispersion having a thiol-reactive group such as a maleimide group or a pyridyl disulfide group on a surface were mixed at 1/1, v/v. The solution was appropriately diluted with DMF. A gold substrate modified with 2-(2-bromoisobutyryloxy)undecyl thiol and amino-EG6 undecanethiol hydrochloride was immersed in DMF in which 0.1 M EDC and 0.05 M 3-maleimidopropionic acid or 3-(2-pyridyldithio)propionic acid was dissolved, and the substrate was allowed to stand for 1 hour. The substrate after the reaction was washed with EtOH and dried with nitrogen blow. 4 µL of a polymer (E108)-particle core mixed solution was dropped on the substrate, and the substrate was allowed to stand for 1 hour. Thereafter, the substrate was washed with EtOH to obtain a particle-immobilized substrate.

### 9-1. Synthesis of polymer (E109)

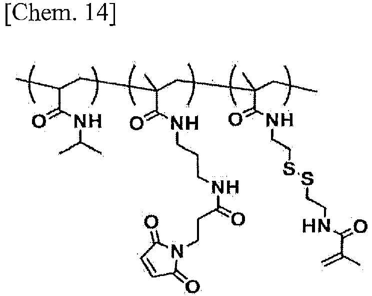

100 mg of the polymer (E3) and 140 µL (1.0 mmol) of TEA were dissolved in CH₂Cl₂ and stirring was performed under ice cooling. 40 mg (0.2 mmol) of EDC and 34 mg (0.2 mmol) of 3-maleimidopropionic acid were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E108).

### 9-2. Polymer (E109)-particle core complex formation and immobilization on substrate

A polymer (E109) aqueous solution (2 mg/mL) and a particle core dispersion having a thiol group on a surface were mixed at 1/1, v/v. The solution was appropriately diluted with DMF. A gold substrate modified with 2-(2-bromoisobutyryloxy)undecyl thiol and amino-EG6 undecanethiol hydrochloride was immersed in DMF in which 0.1 M EDC and 0.05 M 3-mercaptopropionic acid were dissolved, and the substrate was allowed to stand for 1 hour. The substrate after the reaction was washed with EtOH and dried with nitrogen blow. 4 µL of a polymer (E109)-particle core mixed solution was dropped on the substrate, and the substrate was allowed to stand for 1 hour. Thereafter, the substrate was washed with EtOH to obtain a particle-immobilized substrate.

### 10-1. Synthesis of polymer (E110)

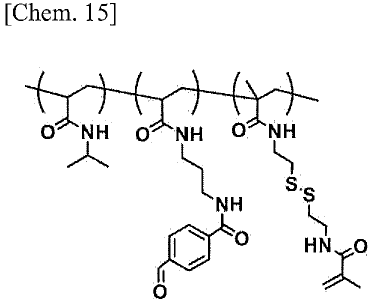

Polymer recipe:

**[Table A-6]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *aldehyde monomer (4-Formyl-N-[2-[(1-oxo-2-propen-1-yl)amino]propyl]benzamide)* | | 258.26 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E110).

### 10-2. Polymer (E110)-particle core complex formation and immobilization on substrate

A polymer (E110) aqueous solution (2 mg/mL) and a particle core dispersion whose surface was modified with an amino group at a ratio of 1/1, v/v. The solution was appropriately diluted with DMF. A polymer (E110)-particle core mixed solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

11-1. Synthesis of polymer (E111)

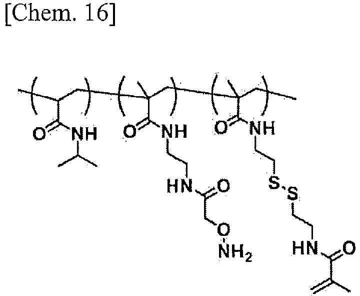

Polymer recipe:

**[Table A-7]**

| | | **MW.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *Boc-oxime monomer (1,1-Dimethylethyl 11-methyl-5,10-dioxo-3-oxa-2,6,9-triazadodec-11-enoate)* | | 301.34 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

The obtained polymer was dissolved in CH₂Cl₂ and stirring was performed under ice cooling. 1 mL of 4 N HCl in dioxane was added thereto, and stirring was further performed overnight. After the reaction, a precipitate generated by adding n-hexane was collected. A small amount of CH₂Cl₂ was added to the precipitate, an excessive amount of n-hexane was further added to wash the precipitate, and then the precipitate was vacuum-dried, thereby obtaining a polymer (E111).

### 11-2. Polymer (E111)-particle core complex formation and immobilization on substrate

A polymer (Bill) aqueous solution (2 mg/mL) and a particle core dispersion whose surface was modified with an aldehyde group at a ratio of 1/1, v/v. The solution was appropriately diluted with DMF. A gold substrate modified with 2-(2-bromoisobutyryloxy)undecyl thiol and carboxy-EG6 undecanethiol was immersed in DMF in which 0.1 M EDC and 0.05 M 4-formylbenzoic acid were dissolved, and the substrate was allowed to stand for 1 hour. The substrate after the reaction was washed with EtOH and dried with nitrogen blow. 4 µL of a polymer (E111)-particle core mixed solution was dropped on the substrate, and the substrate was allowed to stand for 1 hour. Thereafter, the substrate was washed with EtOH to obtain a particle-immobilized substrate.

### 12-1. Synthesis of polymer (E112)

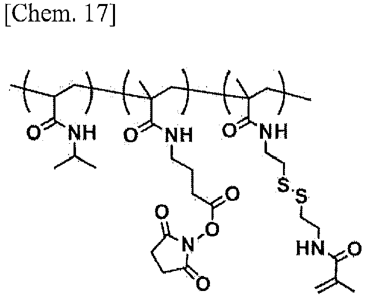

100 mg of the polymer (E101) and 140 µL (1.0 mmol) of TEA were dissolved in CH₂Cl₂ and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E112).

### 12-2. Polymer (E112)-particle core complex formation and immobilization on substrate

A polymer (E112) aqueous solution (2 mg/mL) and a particle core dispersion having an amino group at a ratio of 1/1, v/v. The solution was allowed to stand overnight and diluted 80-fold with DMF. 4 µL of a polymer (E112)-particle core mixed solution was dropped on a gold substrate modified with 2-(2-bromoisobutyryloxy)undecyl thiol and 1-decanaminium, 10-mercapto-N,N,N-trimethyl-, and chloride, and the substrate was allowed to stand for 1 hour. Thereafter, the substrate was washed with EtOH to obtain a particle-immobilized substrate.

### 13-1. Synthesis of polymer (E113)

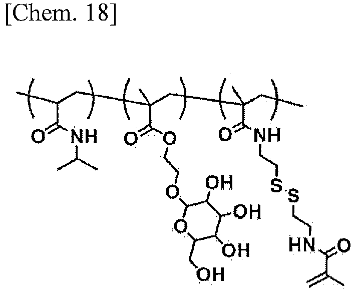

Polymer recipe:

**[Table A-8]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *GEMA* | | 292.28 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E113).

### 13-2. Polymer (E111)-particle core complex formation and immobilization on substrate

A polymer (E113) aqueous solution (2 mg/mL) and a particle core dispersion whose surface was modified with a phenylboronic acid group at a ratio of 1/1, v/v. The solution was appropriately diluted with DMF. A gold substrate modified with 2-(2-bromoisobutyryloxy)undecyl thiol and amino-EG6 undecanethiol hydrochloride was immersed in DMF in which 0.1 M EDC and 0.05 M 4-carboxyphenylboronic acid (or 3-fluoro-4-carboxyphneyl boronic acid) was dissolved, and the substrate was allowed to stand for 1 hour. The substrate after the reaction was washed with EtOH and dried with nitrogen blow. 4 µL of a polymer (E113)-particle core mixed solution was dropped on the substrate, and the substrate was allowed to stand for 1 hour. Thereafter, the substrate was washed with EtOH to obtain a particle-immobilized substrate.

### 14-1. Synthesis of polymer (E114)

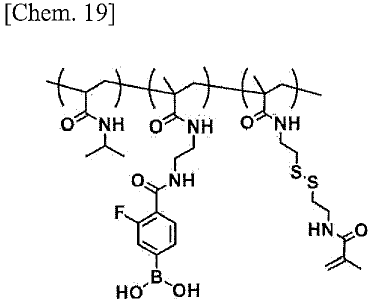

Polymer recipe:

**[Table A-9]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *4-(2-Methacrylamidoethylcarbamoyl)-3-fluorophenylboronic acid* | | 292.06 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E114).

### 14-2. Polymer (E114)-particle core complex formation and immobilization on substrate

A polymer (E114) aqueous solution (2 mg/mL) and a particle core dispersion whose surface was modified with group having a cis-diol structure at a ratio of 1/1, v/v. The solution was appropriately diluted with DMF. A gold substrate modified with 2-(2-bromoisobutyryloxy)undecyl thiol and amino-EG6 undecanethiol hydrochloride was immersed in DMF in which TEA and α-D-mannopyranosylphenyl isothiocyanate were dissolved, and the substrate was allowed to stand for 1 hour. The substrate after the reaction was washed with EtOH and dried with nitrogen blow. 4 µL of a polymer (E114)-particle core mixed solution was dropped on the substrate, and the substrate was allowed to stand for 1 hour. Thereafter, the substrate was washed with EtOH to obtain a particle-immobilized substrate.

### 15-1. Synthesis of polymer (E115)

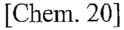

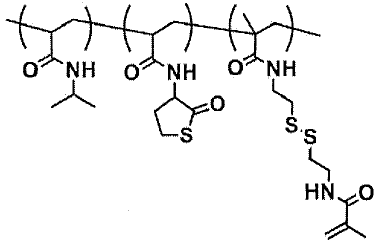

Polymer recipe:

**[Table A-10]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *N-Acryloylhomocysteine thiolactone* | | 171.22 | 100 | 1 |
| NIPAm | | 113.16 | 300 | 1 |
| 2-Cyano-2-((dradecylsulfanylthiocarbonyl)sulfanyi]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E115).

### 15-2. Polymer (E115)-particle core complex formation and immobilization on substrate

A polymer (E114) aqueous solution (2 mg/mL) and a particle core dispersion whose surface was modified with an amino group at a ratio of 1/1, v/v. The solution was appropriately diluted with DMF. A gold substrate modified with 2-(2-bromoisobutyryloxy)undecyl thiol and amino-EG6 undecanethiol hydrochloride was immersed in DMF in which EDC and 3-(2-pyridyldithio)propionic acid were dissolved, and the substrate was allowed to stand for 1 hour. The substrate after the reaction was washed with EtOH and dried with nitrogen blow. 4 µL of a polymer (E115)-particle core mixed solution was dropped on the substrate, and the substrate was allowed to stand for 1 hour. Thereafter, the substrate was washed with EtOH to obtain a particle-immobilized substrate.

### 16-1. Synthesis of polymer (E116)

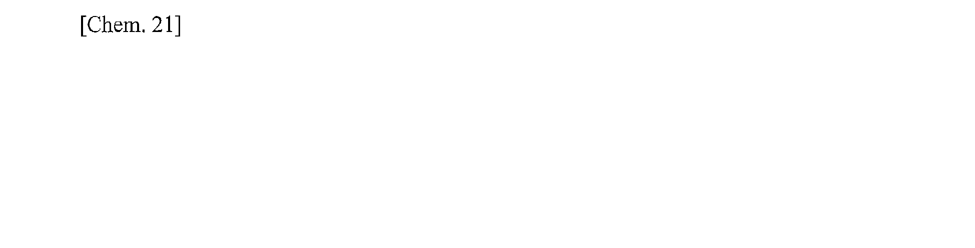

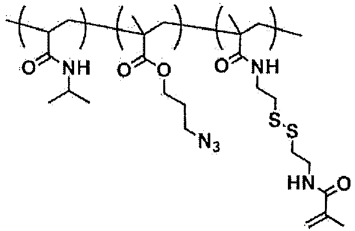

Polymer recipe:

**[Table A-11]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *3-Azidopropyl methacrylate* | | 169.18 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E116).

### 16-2. Polymer (E116)-particle core complex formation and immobilization on substrate

A polymer (E116) aqueous solution (2 mg/mL) and a particle core dispersion whose surface was modified with an alkyne group at a ratio of 1/1, v/v. Copper sulfate and sodium ascorbate were added thereto, and stirring was performed for 1 hour. After the reaction, the solution was replaced with pure water by centrifugation. The solution was appropriately diluted with DMF. A gold substrate modified with 2-(2-bromoisobutyryloxy)undecyl thiol and amino-EG6 undecanethiol hydrochloride was immersed in DMF in which EDC and 4-(3-butyn-1-yldithio)butanoic acid were dissolved, and the substrate was allowed to stand for 1 hour. The substrate after the reaction was washed with EtOH and dried with nitrogen blow. 4 µL of a polymer (E116)-particle core mixed solution (containing copper sulfate and ascorbic acid) was dropped on the substrate, and the substrate was allowed to stand for 1 hour. Thereafter, the substrate was washed with EtOH to obtain a particle-immobilized substrate.

### 17-1. Synthesis of polymer (E116)

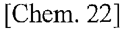

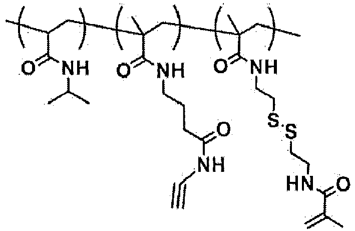

The polymer (E112) and 140 µL (1.0 mmol) of TEA were dissolved in CH₂Cl₂ and stirring was performed under ice cooling. 11 mg (0.2 mmol) of propargylamine were added thereto and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E117).

### 17-2. Polymer (E117)-particle core complex formation and immobilization on substrate

A polymer (E117) aqueous solution (2 mg/mL) and a particle core dispersion whose surface was modified with an azide group at a ratio of 1/1, v/v. Copper sulfate and sodium ascorbate were added thereto, and stirring was performed for 1 hour. After the reaction, the solution was replaced with pure water by centrifugation. The solution was appropriately diluted with DMF. A gold substrate modified with 2-(2-bromoisobutyryloxy)undecyl thiol and amino-EG6 undecanethiol hydrochloride was immersed in DMF in which EDC and 3-[(2-azidoethyl)dithio]propanoic acid were dissolved, and the substrate was allowed to stand for 1 hour. The substrate after the reaction was washed with EtOH and dried with nitrogen blow. 4 µL of a polymer (E117)-particle core mixed solution (containing copper sulfate and ascorbic acid) was dropped on the substrate, and the substrate was allowed to stand for 1 hour. Thereafter, the substrate was washed with EtOH to obtain a particle-immobilized substrate.

### 18-1. Synthesis of polymer (E201)

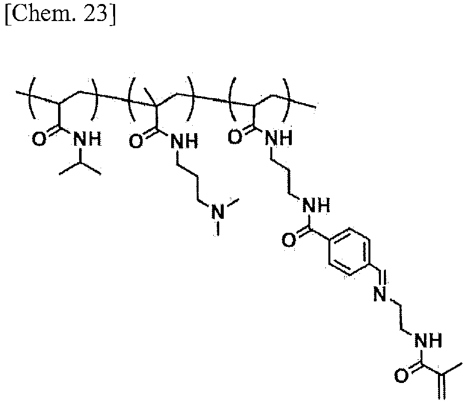

Polymer recipe:

**[Table A-12]**

| | **Mw.** | | |
|---|---|---|---|
| Sample | | Concen tration (mM) | Final liquid volume (mL) |
| *aldehyde monomer (4-Formyl-N-[2-[(1-oxo-2-propen-1-yl)amino]propyl]benzamide)* | 258.26 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propone | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 25 mg (0.2 mmol) of N-(2-aminoethyl)methacrylamide was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E201).

### 18-2. Polymer (E201)-particle core complex formation and immobilization on substrate

A polymer (E201) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 19-1. Synthesis of polymer (E202)

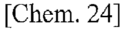

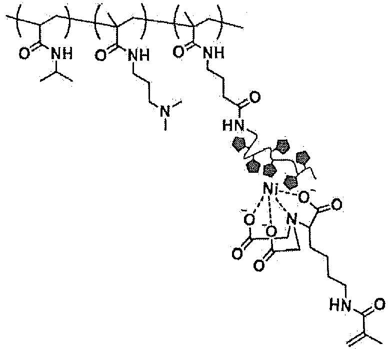

Polymer recipe:

**[Table A-13]**

| | **Mw.** | | |
|---|---|---|---|
| Sample | | Concentration (mM) | Final liquid volume (mL) |
| *4-[(2-Methyl-1-oxo-2-propen-1-yl)amino]butanoic acid* | 171.19 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C) (20 h). After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

10 mg of the polymer prepared in the previous section and 28 µL of TEA were dissolved in DMF (1 mL), and stirring was performed. 3 mg of a synthetic peptide (six histidines were bonded from the N-terminal, three glycines were bonded, and a lysine was bonded to the C-terminal) was added thereto, and stirring was further performed overnight. After the reaction, a small amount of CH₂Cl₂ was added and then an excessive amount of n-hexane was added to generate a precipitate. The precipitate was collected, a small amount of CH₂Cl₂ was added thereto, an excessive amount of n-hexane was further added to wash the precipitate, and then the precipitate was vacuum-dried.

The polymer prepared in the previous section was dissolved in MeOH, Ni-complexed L-Lysine and N2,N2-bis(carboxymethyl)-N6-(2-methyl-1-oxo-2-propen-1-yl)- was added thereto, and stirring was performed. CH₂Cl₂ was added, and the precipitated precipitate was collected and vacuum-dried to obtain a polymer (E202).

### 19-2. Polymer (E202)-particle core complex formation and immobilization on substrate

A polymer (E202) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 20-1. Synthesis of polymer (E203)

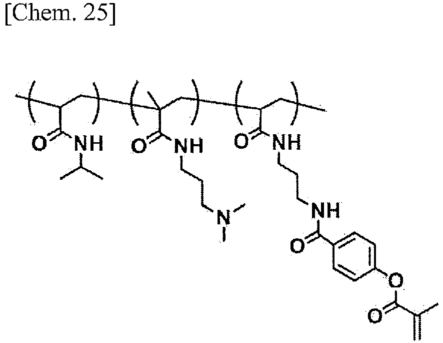

Polymer recipe:

**[Table A-14]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N*-(3-(Dimethylamino)propyl)methacrylamide | 170.25 | 100 | 1 |
| NiPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 40 mg (0.2 mmol) of 4-methacryloyloxybenzoic acid was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E203).

### 20-2. Polymer (E203)-particle core complex formation and immobilization on substrate

A polymer (E203) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 21-1. Synthesis of polymer (E204)

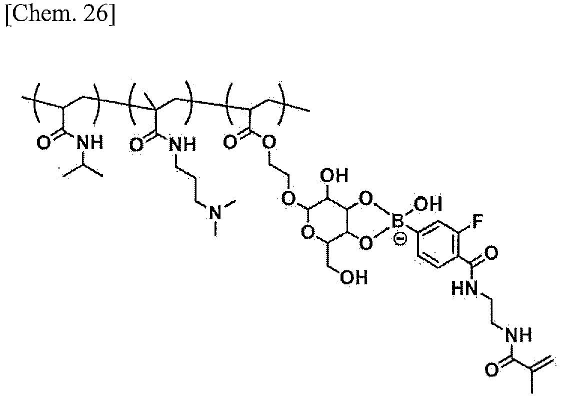

Polymer recipe:

**[Table A-15]**

| | **Mw.** | | |
|---|---|---|---|
| Sample | | Concen tration (mM) | Final liquid volume (mL) |
| *GEMA* | 292.28 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C).

### (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 59 mg (0.2 mmol) of 4-(2-methacrylamidoethylcarbamoyl)-3-fluorophenylboronic acid was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E204).

### 21-2. Polymer (E204)-particle core complex formation and immobilization on substrate

A polymer (E204) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 22-1. Synthesis of polymer (E205)

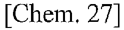

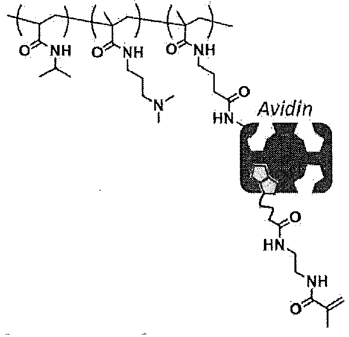

Polymer recipe:

**[Table A-16]**

| | **Mw.** | | |
|---|---|---|---|
| Sample | | Concen tration (mM) | Final liquid volume (mL) |
| *4-[(2-Methyl-1-oxo-2-propen-1-yl)amino]butanoic acid* | 171.19 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C).

### (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

10 mg of the polymer prepared in the previous section was dissolved in a 50 mM carbonate buffer (pH 8.5) (1 mL), and stirring was performed. 3 mg of Avidin was added thereto, and stirring was further performed overnight. The reaction solution was placed in a dialysis membrane for 100 kDa, and dialysis was carried out in a phosphate buffer (pH 7.4). After dialysis, concentration was performed by ultrafiltration. Thereafter, a biotin monomer ((3aS,4S,6aR)-hexahydro-2-oxo-N-[3-[(1-oxo-2-propen-1-yl)amino]propyl]-1H-thieno[3,4-d]imidazole-4-pentanamide) was bonded to obtain a target polymer (E205).

### 22-2. Polymer (E205)-particle core complex formation and immobilization on substrate

A polymer (E205) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 23-1. Synthesis of polymer (E206)

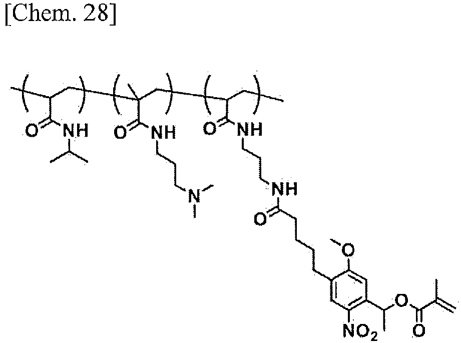

Polymer recipe:

**[Table A-17]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 38 mg (0.2 mmol) of EDC and 73 mg (0.2 mmol) of 4-[4-[1-(methacryloyloxy)ethyl]-2-methoxy-5-nitrophenoxy]butanoic acid were added thereto, and stirring was performed overnight under light-shielding. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E206).

### 23-2. Polymer (E206)-particle core complex formation and immobilization on substrate

A polymer (E206) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour under light-shielding. Thereafter, the substrate was washed with EtOH to obtain a particle-immobilized substrate.

### 24-1. Synthesis of polymer (E207)

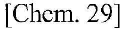

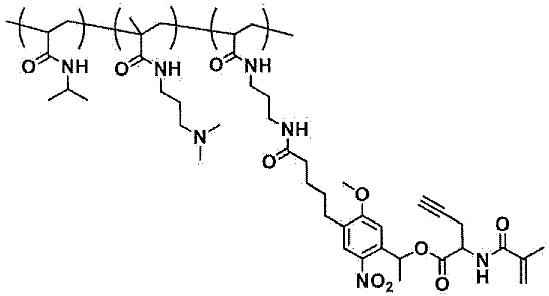

Polymer recipe:

**[Table A-18]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 38 mg (0.2 mmol) of EDC and 92 mg (0.2 mmol) of a compound A were added thereto, and stirring was performed overnight under light-shielding. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E207).

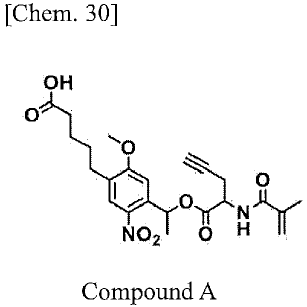

### 24-2. Polymer (E207)-particle core complex formation and immobilization on substrate

A polymer (E207) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour under light-shielding. Thereafter, the substrate was washed with EtOH to obtain a particle-immobilized substrate.

### 25-1. Synthesis of polymers (E301 to E312)

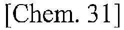

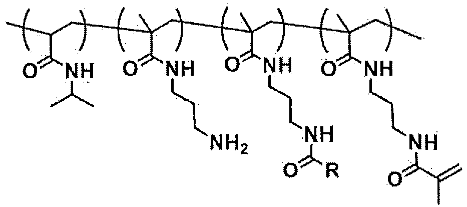

Polymer recipe:

**[Table A-19]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N-(t-BOC-aminopropyl)methacrylamide* | 242.31 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The prepared polymer and 140 µL (1.0 mmol) of TEA were dissolved in CH₂Cl₂, and stirring was performed under ice cooling. 36 mg (0.2 mmol) of N-succinimidyl methacrylate was added thereto, and stirring was performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

The polymer was dissolved in CH₂Cl₂ and stirring was performed under ice cooling. 1 mL of 4 N HCl in dioxane was added thereto, and stirring was further performed overnight. After the reaction, a precipitate generated by adding n-hexane was collected. A small amount of CH2Cl2 was added to the precipitate, an excessive amount of n-hexane was further added to wash the precipitate, and then the precipitate was vacuum-dried.

The polymer and 140 µL (1.0 mmol) of TEA were dissolved in CH₂Cl₂ and stirring was performed under ice cooling. 0.25 mmol of any of the compounds in the table on the right and 20 mg (0.1 mmol) of *EDC were added thereto, and stirring was performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain target polymers (E301 to E312).

Use at time of synthesizing *E304 to E309 and E311 and E312

**[Table A-20]**

| | Substituent to be introduced | Compound example |
|---|---|---|
| **E301** | Carboxy | 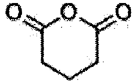 |
| **E302** | Carboxylic acid active ester group | 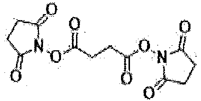 |
| **E303** | Metal complex (NTA group) | 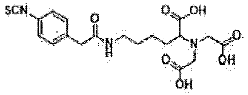 |
| **E304** | Thiol group | 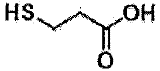 |
| **E305** | Pyridyl disulfide group | 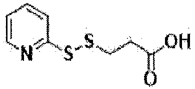 |
| **E306** | Maleimide group | 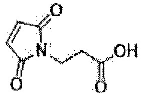 |
| **E307** | Halogenated acetamide group | 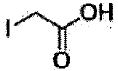 |
| **E308** | Azide group | 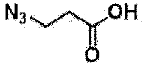 |
| **E309** | Alkyne group | 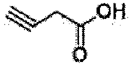 |
| **E310** | cis-diol group (sugar group) | 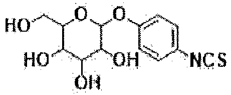 |
| **E311** | Boronyl group | 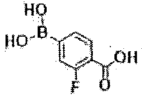 |
| **E312** | Thiolactone group | 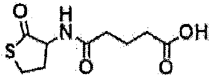 |

### (Example 1-3: Binding of polymer to particles)

### Synthesis of polymer (E2-0)-silica nanoparticle complex (RM)

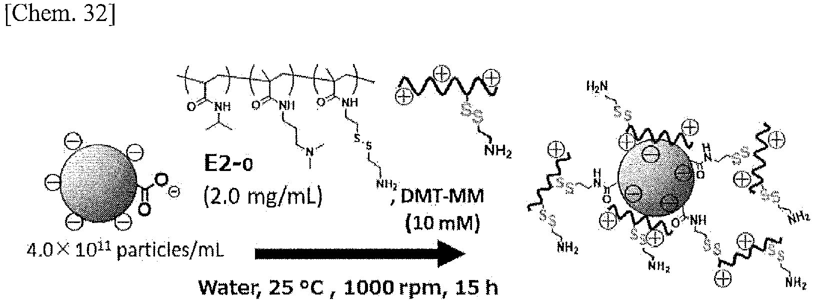

Silica nanoparticles (200 nm, terminal COOH), 2.0 mg/mL of E2-0, and 10 mM DMT-MM were mixed in pure water (500 µL) so as to be 4.0 × 10¹¹ particles/mL, and the mixture was shaken by a thermoshaker at 25°C and 1,000 rpm for 15 hours. After the reaction, 500 µL of pure water was added, the particles were precipitated by centrifugation (25°C, 1,000 rpm, 15 min), and 900 µL of the supernatant was removed. This operation was performed five times in total to purify the particles. The results of performing DLS measurement on the particle size and surface charge of the obtained particles are illustrated in FIG. 1-6.

Since the average particle size (Z-Average) was 232 nm (pdi: 0.022) and the surface charge (Z-potential) was +55.3 mV, it was confirmed that the polymer was modified because the surface charge was greatly positively changed while maintaining the monodispersibility of the particles.

### 25-2. Polymer (E301)-particle core complex formation and immobilization on substrate

A polymer (E301) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### (Example 2: Preparation of measurement substrate)

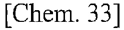

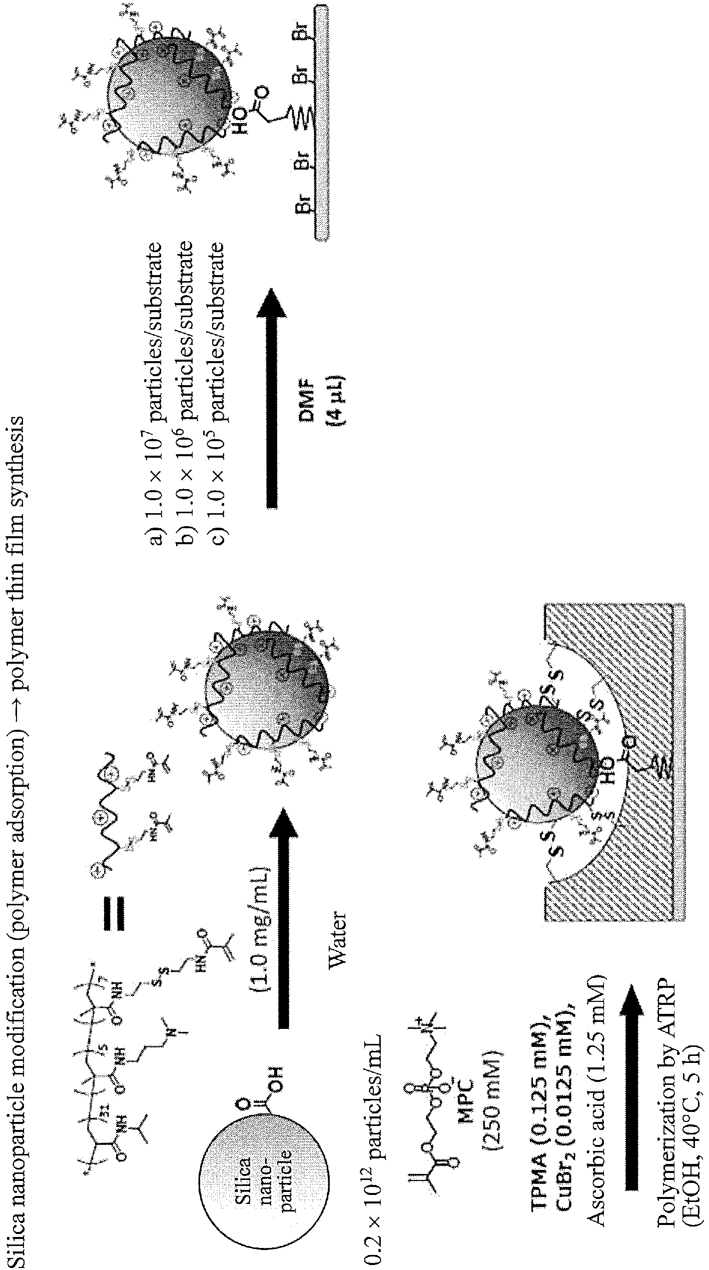

### 2-1. Experiment

### 2-1-1. Surface modification of gold substrate

A gold substrate (9.8 mm × 4.3 mm) was rinsed with EtOH and then subjected to UV-O3 treatment for 15 min to clean up a substrate surface. A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 2-1-2. Preparation of particle-immobilized substrate

A silica nanoparticle dispersion (particle size: 200 nm, particle concentration: 2.0 × 10¹¹ particles/mL) and the polymer E2 (1.0 mg/mL) were mixed in water at the above-described final concentration to form a complex.

The complex solution was diluted with DMF, dropped (4 µL) on the area of the substrate on which the complex was immobilized so as to have the number shown in Table 2-1, and allowed to stand at 25°C for 1 hour. The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Table 2-1.

**[Table 2-1]**

| Table 2-1 Experiment condition table | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁷ | 1.5 sec | 0.25 sec | 0.02 sec |
| 2 | 3×10⁶ | ↑ | ↑ | ↑ |
| 3 | 1×10⁶ | ↑ | ↑ | ↑ |
| 4 | 3×10⁵ | 2 sec | ↑ | ↑ |
| 5 | 1×10⁵ | ↑ | 0.5 sec | ↑ |

### 2-1-3. Synthesis of silica nanoparticle imprinted polymer on particle-immobilized substrate

Prepolymer solutions having compositions shown in Table 2-2 were prepared, dissolved oxygen was removed by freeze-degassing, and then polymerization was performed at 25°C for 18 hours in a glove box. The substrate after polymerization was washed with pure water.

The substrate was immersed in a 100 mM tris(2-carboxyethyl)phosphine hydrochloride solution (methanol : pure water = 1 : 1), and an ultrasonic treatment was performed for 5 min to remove silica nanoparticles, thereby forming pores in a surface layer of a polymer thin film.

**[Table 2-2]**

| Table 2-2 Prepolymer solution composition | | |
|---|---|---|
| | Sample | Concentration |
| Main raw material | MPC (2-methacryloyloxyethyl phosphorylcholine) | 500 mM |
| Initiator | EBIB (ethyl α-bromoisobutyrate) | 1.25 mM |
| Ligand | TPMA (tris(2-pyridylmethyl)amine) | 0.125 mM |
| Catalyst | CuBr₂ | 0.0125 mM |
| Reducing agent | Ascorbic acid | 1.25 mM |
| Solvent | EtOH | 270 *µ* L |

### 2-2. Results

### 2-2-1. Preparation of particle-immobilized substrate

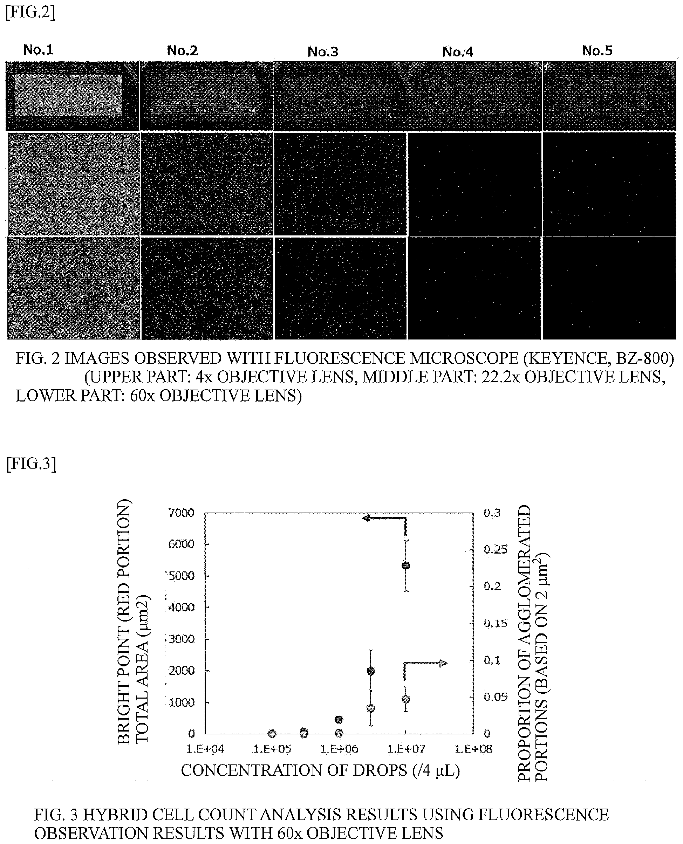

The observation results with a fluorescence microscope are illustrated in FIG. 2. The fluorescence derived from the silica nanoparticles was confirmed on the substrate under any conditions, and thus it was confirmed that the silica nanoparticles (polymer complex) were immobilized on the substrate.

In addition, FIG. 3 illustrates a result of performing analysis using fluorescence observation with a 60-fold objective lens and hybrid cell count. From FIG. 3, it was found that the number of silica nanoparticles on the substrate tended to increase as the number of drops increased. In addition, in No. 4 and No. 5 in FIG. 2 in which it was clearly determined from the image that the particles were monodispersed, since there were almost no bright spots with an area of more than 2 µm², the particles having a bright spot area exceeding this were determined to be an aggregate. By setting the numerical value as a boundary value, it was possible to objectively express by a numerical value that agglomeration occurred at No. 4 (3 × 10⁶ particles/4 µL) or more where bright spots exceeding the boundary value 2 µm² occurred and no agglomeration occurred at No. 5 (1 × 10⁶ particles/4 µL) or less, and it was possible to control the particle form on the substrate depending on the number of drops on the substrate.

### (Example 3) Preparation of particle-immobilized substrate by anionic polymer and cationic silica nanoparticles

An anionic polymer (1.0 mg/mL) obtained by copolymerizing any one of 2-acrylamide-2-methylpropane sulfonic acid, 3-acrylamide phenyl boronic acid, monoacryloyl ethyl phosphate, 2-(trifluoromethyl)acrylic acid, acrylic acid, methacrylic acid, and the like as an anionic monomer instead of the cationic monomer N-(3-dimethylaminopropyl)methacrylamide at the time of E2 synthesis with the cationic silica nanoparticle dispersion (particle size: 200 nm, particle concentration: 2.0 × 10¹¹ particles/mL) into which an amine was introduced was mixed in water at the above-described final concentration to form a complex.

The complex solution was diluted with DMF, dropped (4 µL) on the area of the substrate on which the complex was immobilized so as to have the number shown in Table 2-2, and allowed to stand at 25°C for 1 hour. The substrate was washed with DMF, EtOH, and pure water in this order to obtain a particle-immobilized substrate.

By evaluating a sensor produced using the particle-immobilized substrate by a conventional method, it is demonstrated that a sensor can also be produced by a particle-immobilized substrate using an anionic polymer and cationic silica nanoparticles.

### (Example 4: Sensing of hepatitis B mimetic particles)

### 4.1. Preparation of silica particles in which cystamine and polymerizable functional group are adsorbed by electrostatic interaction

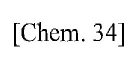

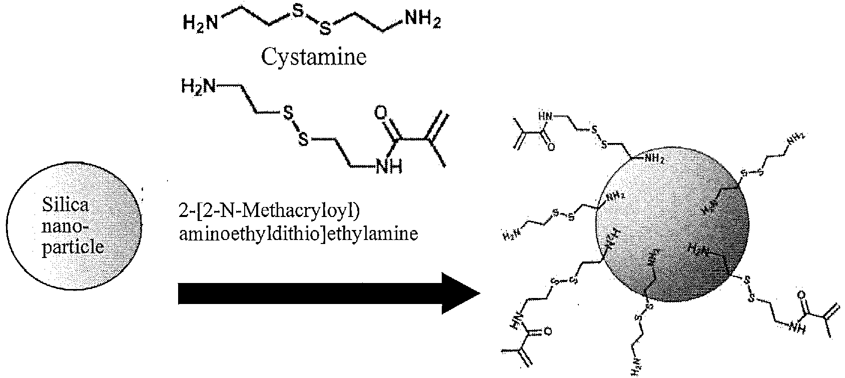

5 mg/mL of rhodamine-labeled silica nanoparticles (without a functional group: 200 nm), 500 µM of cystamine hydrochloride, and 500 µM of 2-[2-N-(methacryloyl)aminoethyldithio]ethylamine (polymerizable functional group) were added to 500 µL of pure water. Thereafter, shaking (25 °C, 1,400 rpm, 1 h) was performed to prepare silica particles in which cystamine and a polymerization functional group were non-covalently adsorbed. Thereafter, centrifugation (4,000 G, 10 min) was performed once to collect 400 µL of a supernatant, and 400 µL of ethanol was added thereto, thereby replacing the dispersion medium with ethanol.

### 4-1-2. Preparation of sensor chip

### • Preparation of SAM

The gold sputter glass substrate (9.8 mm × 4.3 mm) was washed with EtOH and then subjected to UV ozone treatment (20 min) to decompose organic substances on the surface of the gold substrate. Thereafter, the substrate was immersed in a 1 mM 11-mercaptoundecanoic acid EtOH solution and allowed to stand (30°C, 18 h) to prepare an SAM. After the substrate was washed with EtOH and pure water and dried with a N₂ gun, the substrate was immersed in a 0.2 M 1-ethyl-3-(3-dimethylaminopropyl)carbodiimide hydrochloride (EDC) and 0.05 MN-hydroxysuccinimide (NHS) aqueous solution, allowed to stand (4°C, 30 min), and washed with cold water (4°C). After the substrate was dried with a N₂ gun, the substrate was immersed in a 100 mM NaHCOs solution (pH 8.0) in which 5 mM N-(2-aminoethyl)-2-bromo-2-methyl-propropionamide hydrochloride and 5 mM β-alanine were dissolved, and allowed to stand (r.t., 2h) to introduce a bromo group and a carboxy group into the SAM.

### 4-1-3. Immobilization of silica particles

After the substrate was washed with pure water and dried with a N₂ gun, the substrate was placed in a PCR tube, 50 µg/mL cystamine-adsorbed silica and 270 µL of an EtOH solution containing 20 mM 4-(4,6-dimethoxy-1,3,5-triazin-2-yl)-4-4-methylmorpholinium chloride (DMT-MM) were added, and shaking was performed (25°C, 300 rpm, 18 h) to immobilize the silica particles.

### 4-1-4. Preparation of MPC thin film

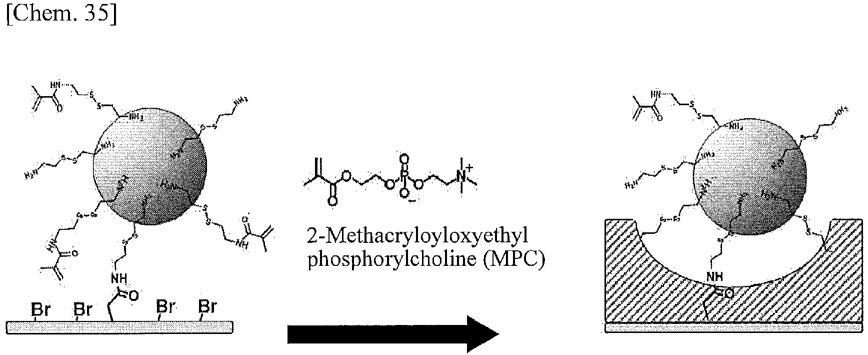

According to the recipe in Table 3-1, polymer thin films were synthesized on substrates by surface-initiated atom transfer radical polymerization (SI-ATRP). In a glove box, each reagent, degassed EtOH, and pure water were prepared, components other than L-ascorbic acid were dissolved in EtOH, and L-ascorbic acid was dissolved in pure water to prepare a polymerization solution. The substrate was placed in a 2 mL tube so that the two glass surfaces faced each other. Polymerization was performed under an Ar atmosphere at 25°C for 18 hours with shaking at 500 rpm.

**[Table 3-1]**

| Table 3-1 SI-ATRP synthesis recipe | |
|---|---|
| Sample | Concentration |
| 2-Methacryloyloxyethyl phosphorylcholine (MPC) | 500 mM |
| Tris(2-pyridylmethyl)amine (TPMA) | 0.25 mM |
| CuBr₂ | 0.025 mM |
| L-ascorbic acid | 2.5 mM |
| EtOH | 675 *µ* L |
| Pure water | 75 *µ* L |

### 4-1-5. Cutting out silica particle

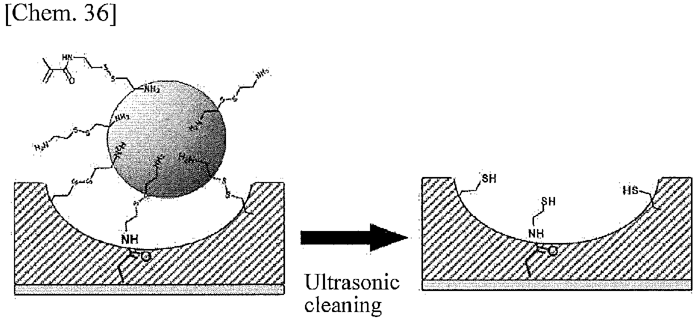

After the polymerization, the substrate was washed with pure water, dried with a N₂ gun, and placed in a 2 mL tube together with 1 mL of pure water. The silica particle was cut out from the polymer thin film by performing ultrasonic cleaning (High, Level 4.5) three times each for 5 min. The cut-out substrate was immersed in a 10 mM tris(2-carboxyethyl)phosphine hydrochloride (TCEP) aqueous solution to perform a reduction (25°C, 1 h, 1,000 rpm). 33 µL of a 100 µM Alexa Fluor (registered trademark) 647 C₂ maleimide, 100 µM N-[5-(3'-maleimidopropylamide)-1-carboxypentyl]iminodiacetic acid, and a disodium salt (maleimide-C3-NTA) 20.5% DMSO-containing phosphate buffer solution (PBS, 10 mM phosphate, 140 mM NaCl, pH 7.4) solution were dropped on the substrate, and the substrate was allowed to stand (r.t., 1 h).

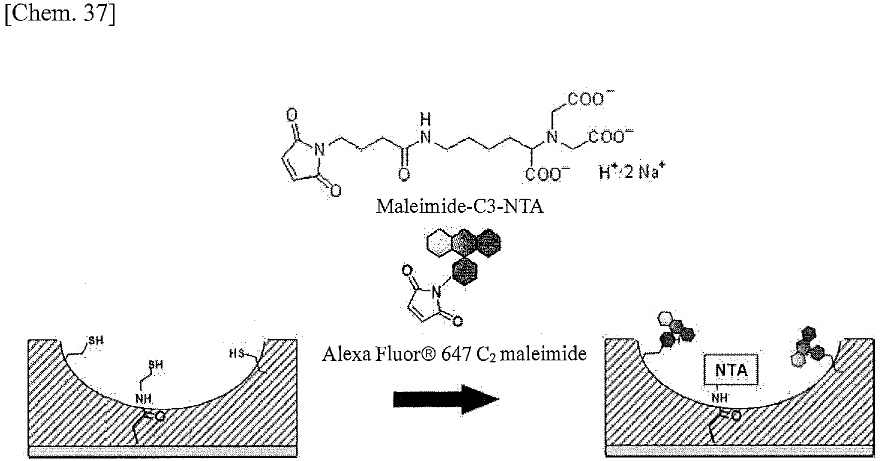

After washing with pure water and drying with a N₂ gun, 35 µL of a 4 mM NiCl₂ aqueous solution was dropped on the substrate, and the substrate was allowed to stand (room temperature, 15 min). After washing with pure water and drying with a N₂ gun, a 100 µM His-tagged proteinG PBS solution was dropped on the substrate, and the substrate was allowed to stand (r.t., 30 min) to immobilize His-tagged proteinG. After washing with pure water and drying with a N₂ gun, a PBS solution of 10 µg/mL Anti-HBs Pre-S1 antibody was dropped on the substrate, and the substrate was allowed to stand (r.t., 30 min) to introduce the antibody into the substrate. Anti-HBs Pre-S1 is a monoclonal antibody that recognizes a Pre-S1 domain of the hepatitis B virus surface antigen. The Pre-S1 domain plays an important role in the recognition of hepatocytes by the hepatitis B virus.

### 4-1-6. Sensing of bio-nanocapsules as model of hepatitis B virus (HBV)

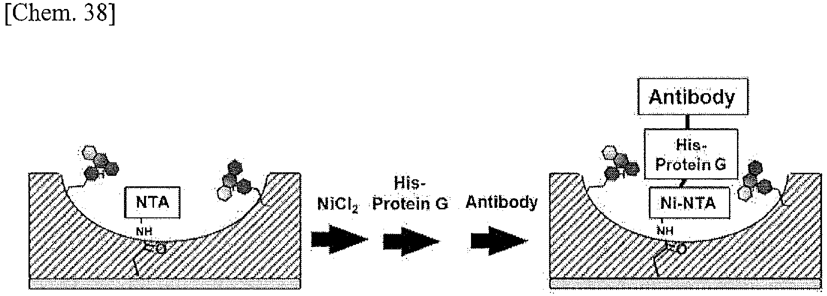

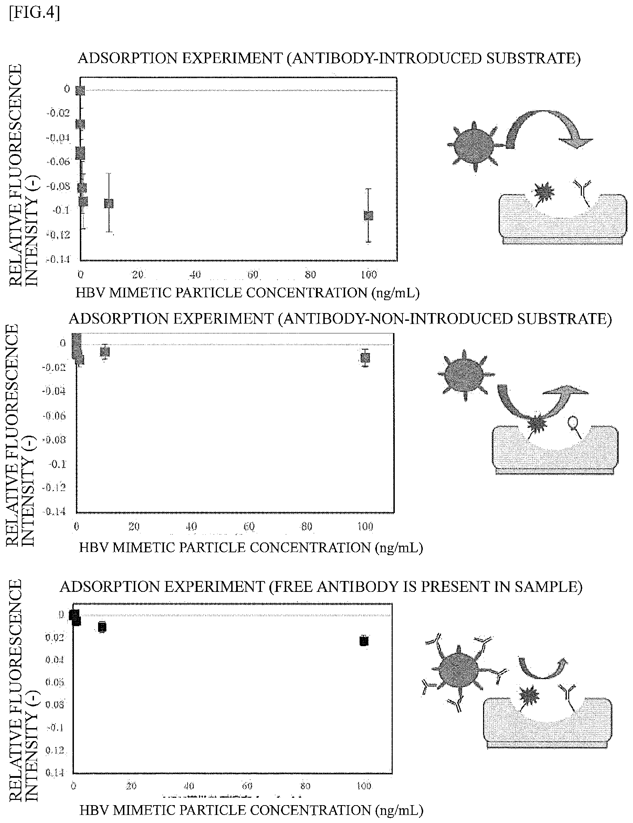

The concentrations of the HBV mimetic particle PBS solution were set to 0.01, 0.05, 0.1, 0.5, 1, 10, and 100 ng/mL. Measurement conditions of the fluorescence microscope were as follows: a filter was set to an excitation wavelength of 604 to 644 nm and a fluorescence wavelength of 672 to 712 nm, an objective lens was 5 times, an exposure time was 0.1 sec, a light amount was 25%, and a light source was a mercury lamp. For the measurement, an antibody-introduced or non-introduced substrate was used. In addition, for the antibody introduction substrate, an antibody was reacted in advance, and measurement was also performed using HBV mimetic particles in which an antigenic protein on a membrane was neutralized in advance with a free antibody. HBV mimetic particles neutralized with free antibodies were prepared by incubating 100 µL of a 2 µg/mL HBV mimetic particle PBS (10 mM phosphate, 140 mM NaCl, pH 7.4) solution and 100 µg/mL of Anti-HBs Pre-S1 PBS (10 mM phosphate, 140 mM NaCl, pH 7.4) solution at 4°C for 1 h. The results are summarized in FIG. 4.

### 4-2. Results

4-2-1. It was suggested that hepatitis B virus was measured by changing the antibody of the exosome sensing pore.

### (Example 5: Sensing of SARS-CoV-2 (COVID-19 cause virus) mimic particles)

Since an S protein-expressing exosome is similar in size to SARS-CoV-2 and has no viral activity, the exosome was used as a mimic particle of SARS-CoV-2 virus, and was sensed by a fluorescence microscope with a SIC automatic dispensing device. 5-1-1. Preparation of sensor chip

### • Preparation of SAM

The gold sputter glass substrate (9.8 mm × 4.3 mm) was washed with EtOH and then subjected to UV ozone treatment (20 min) to decompose organic substances on the surface of the gold substrate. Thereafter, the substrate was immersed in a 0.5 mM 11-mercaptoundecanoic acid solution and a 0.5 mM 2-(2-bromoisobutyryloxy)undecyl thiol EtOH solution and allowed to stand (30°C, 18 h) to prepare a self-assembled monolayer (SAM). The substrate was washed with EtOH and pure water, dried with a N₂ gun, and then stored.

### 5-1-2. Immobilization of silica particles

As the silica nanoparticles to be immobilized, the same silica nanoparticles as those used for the sensing substrate of the hepatitis B mimetic particles were used. After the substrate was washed with pure water and dried with a N₂ gun, the substrate was placed in a PCR tube, 50 µg/mL cystamine-adsorbed silica and 270 µL of an EtOH solution containing 20 mM DMT-MM were added, and shaking was performed (25°C, 300 rpm, 18 h) to immobilize the silica particles.

### 5-1-3. Preparation of MPC thin film

According to the following recipe, polymer thin films were synthesized on substrates by surface-initiated atom transfer radical polymerization (SI-ATRP). In a glove box, each reagent, degassed EtOH, and pure water were prepared, components other than L-ascorbic acid were dissolved in EtOH, and L-ascorbic acid was dissolved in pure water to prepare a polymerization solution. The substrate was placed in a 2 mL tube so that the two glass surfaces faced each other. Polymerization was performed under an Ar atmosphere at 25°C for 18 hours with shaking at 500 rpm.

**[Table 4]**

| SI-ATRP synthesis recipe | |
|---|---|
| Sample | Concentration |
| MPC | 500 mM |
| TPMA | 0.25 mM |
| CuBr₂ | 0.025 mM |
| L-ascorbic acid | 2.5 mM |
| EtOH | 675 *µ* L |
| Pure water | 75 *µ* L |

### 5-1-4. Cutting out silica particle

After the polymerization, the substrate was washed with pure water, dried with a N₂ gun, and placed in a 2 mL tube together with 1 mL of pure water. The silica particle was cut out from the polymer thin film by performing ultrasonic cleaning (High, Level 4.5) three times each for 5 min.

### 5-1-5. Introduction of fluorescent molecules and antibodies

The cut-out substrate was immersed in a 10 mM TCEP aqueous solution to perform a reduction (25°C, 1 h, 1,000 rpm). 33 µL of a 100 µM Alexa Fluor (registered trademark) 647 C₂ maleimide and 100 µM maleimide-C3-NTA 20.5% DMSO-containing PBS (10 mM phosphate, 140 mM NaCl, pH 7.4) solution were dropped on the substrate, and the substrate was allowed to stand (r.t., 1 h). After washing with pure water and drying with a N₂ gun, 35 µL of a 4 mM NiCl₂ aqueous solution was dropped on the substrate, and the substrate was allowed to stand (r.t., 15 min). After washing the full-body antibody-introduced substrate with pure water and drying with a N₂ gun, 10 µM His-tagged proteinG in a PBS (10 mM phosphoric acid, 140 mM NaCl, pH 7.4) solution was dropped on the substrate, and the substrate was allowed to stand (r.t., 30 min) to immobilize His-tagged proteinG. After the substrate was washed with pure water and dried with a N₂ gun, a 0.3 µM anti-SARS-CoV-2 spike protein monoclonal antibody (Hakareru lot A-2-5A12) PBS (10 mM phosphoric acid, 140 mM NaCl, pH 7.4) solution was dropped on the substrate, and the substrate was allowed to stand (r.t., 1 h) to introduce a full-body antibody into the substrate. In addition, in the self-assembled nanobody introduction substrate, after Ni complex formation, a 0.3 µM Covid VHH (H1-H4-Cmyc-His) PBS solution was added dropped on the substrate, and the substrate was allowed to stand (r.t, 30 min) to introduce the nanobody into the substrate.

### 5-1-6. Adsorption experiment

An S protein-expressing exosome capture experiment was performed using a fluorescence microscope equipped with a SIC automatic dispensing device. The concentrations of the S protein-expressing exosome PBS (10 mM phosphoric acid, 140 mM NaCl, pH 7.4) solution used in the experiment were set to 0, 0.01, 0.05, 0.1, 0.5, 1, 10, and 100 ng/mL. Measurement conditions of the fluorescence microscope were as follows: a filter was Cy5 (excitation wavelength of 604 to 644 nm, fluorescence wavelength of 672 to 712 nm), an objective lens was 5 times, an exposure time was 0.2 sec, a light amount was 12%, and a light source was a mercury lamp. Measurement was performed in the range of the central portion of the substrate. The sequence of the automatic dispensing device was: 1. chip attachment, 2. sample suction (100 µL), 3. reaction (1 min, 25°C), 4. full discharge, 5. suction of 150 µL of 10 mM PBS (140 mM NaCl, pH 7.4), and 6. measurement position keep (then, repeat 2 → 6).

### 5-2. Results

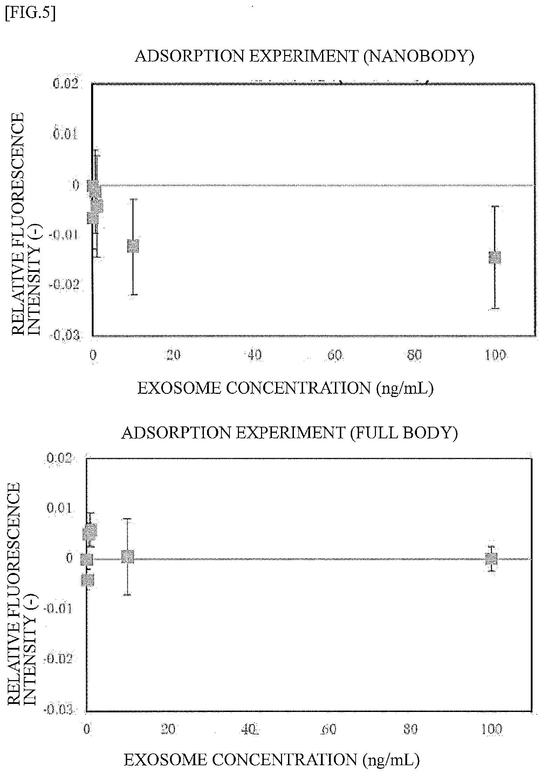

5-2-1. As a result of the measurement, it has been found that sensing was performed more sensitively with the nanobody than with the full-body antibody. (FIG. 5)

### (Example 6: Binding of polymer to particles (covalent bonding))

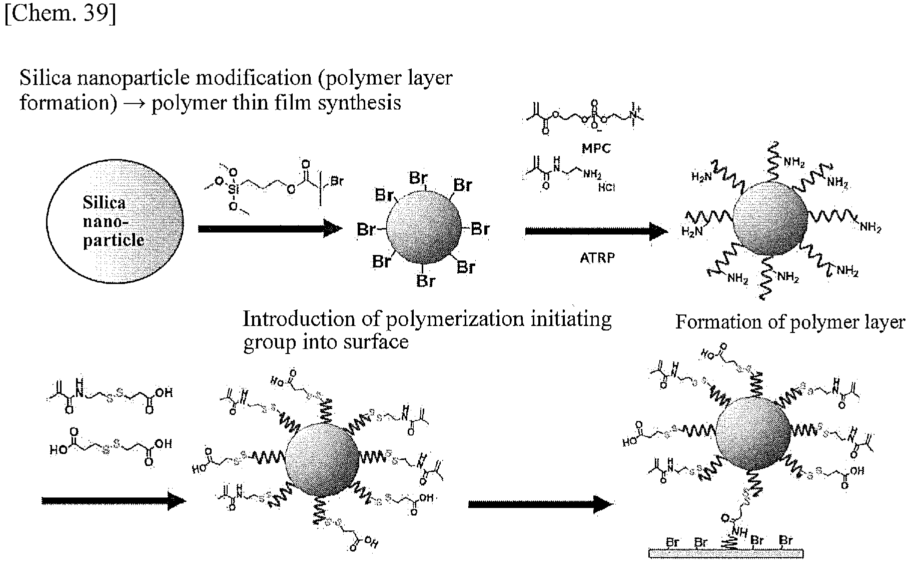

### 6.1. Modification and polymerization of silica particles

39.6 µl (1.98 mg) of silica particles (plane, red, 200 nm, 50 mg/mL), 60.4 µl of pure water, 100 µl of 2 M HCl, and 20.6 µl of 3-(triethoxysilyl)propyl 2-bromo-2-methylpropanoate) were added to 800 µL of ethanol. After mixing by vortexing, the mixture was mixed by inversion and reacted at 25°C for 19 h. The silica particles after the reaction were centrifugally washed with pure water and then re-dispersed in ethanol.

1 mL (1.98 mg/mL) of the Br-modified silica particles were mixed according to the formulation in Table 5-1, and ATRP was performed at 25°C for 16.5 h to synthesize an MPC polymer brush containing a primary amine on the silica surface. The polymer brush-modified silica particles after polymerization were centrifugally washed with a 50% ethanol aqueous solution and then dispersed by ultrasonic waves.

**[Table 5-1]**

| | |
|---|---|
| MPC | 500 mM |
| 2-Aminoethyl methacrylate hydrochloride | 250 mM |
| EBIB | 5 mM |
| TPMA | 0.5 mM |
| CuBr₂ | 0.05 mM |
| Ascorbic acid | 5 mM |
| Solvent: 50% ethanol aqueous solution | |

The polymer brush-modified silica particles after washing were mixed so as to have the formulation in Table 5-2, and reacted at 25°C and 1,000 rpm for 1 h to introduce a polymerization functional group (methacryloyl group) and a reactive functional group (carboxyl group) into the polymer brush on the surface of the silica particle via a disulfide bond. The polymerizable polymer brush-modified silica particles after reaction were centrifugally washed with a 50% ethanol aqueous solution.

**[Table 5-2]**

| | |
|---|---|
| 3,3'-Dithiodipropionic acid | 15 mM |
| COOH-SS-methacrylamide | 15 mM |
| DMT-MM | 30 mM |
| Solvent: 50% ethanol aqueous solution | |

### 6.2. Immobilization on substrate

### • Surface modification of gold substrate

A gold substrate (9.8 mm × 4.3 mm) was rinsed with EtOH and then subjected to UV-O3 treatment for 15 min to clean up a substrate surface.

A 1 mM EtOH solution of 2-(2-bromoisobutyryloxy)undecyl thiol was prepared in an EtOH solvent, and the gold substrate described above was immersed in the solution. After the substrate was allowed to stand at 30°C for 20 hours, the substrate was washed with EtOH and pure water to form a self-assembled monolayer (SAM) on the surface of the gold substrate. A substrate modified with a polymerization initiating group was bought into contact with a prepolymer solution mixed with the composition shown in Table 5-3, and ATRP was performed at 25°C for 20 h to synthesize an MPC polymer brush containing a primary amine on the surface of the gold substrate. The substrate after polymerization was washed with ethanol and pure water.

**[Table 5-3]**

| | |
|---|---|
| MPC | 500 mM |
| 2-Aminoethyl methacrylate hydrochloride | 250 mM |
| EBIB | 5 mM |
| TPMA | 0.5 mM |
| CuBr₂ | 0.05 mM |
| Ascorbic acid | 5 mM |
| Solvent: 50% ethanol aqueous solution | |

### Immobilization of particles

The polymerizable silica particle dispersion (3.3 µg/mL) and a condensing agent (5 mM, DMT-MM) were mixed in a 50% ethanol aqueous solution, and the mixture was added dropwise to the substrate (25°C, 1 h). Thereafter, the substrate was washed with EtOH and pure water in this order to obtain a particle-immobilized substrate in which a carboxyl group on the polymer brush and an amino group on the surface of the substrate were covalently bonded.

### 6.3. Particle variation

The Br group at the polymer brush terminal on the silica particle surface was converted into an azide (N₃) group by mixing the reagent of Table 5-4 in the polymer brush-modified silica particle dispersion (1 mg/mL, 50% ethanol aqueous solution) and polymerizing the mixture to prepare azidated polymer brush-modified silica particles. After the polymerization, centrifugal washing was performed using pure water and methanol.

Subsequently, 80 µL of methanol and a 3 mM DBCO-amine solution (DMSO, 20 µL) were added to the azidated polymer brush-modified silica particles (1 mg/mL, 80 µL), and the mixed solution was reacted at 25°C for 1 hour to convert an azide group into an amino group, thereby preparing aminated polymer brush-modified silica particles. After the reaction, centrifugal washing was performed using a 50% ethanol aqueous solution. The aminated polymer brush-modified silica particles were brought into contact with a substrate having a carboxyl group on a surface in the presence of a condensing agent, such that a particle-immobilized substrate by a covalent bonding can be obtained.

**[Table 5-4]**

| EBIB | TPMA | CuBr₂ | NaN₃ | Ascorbic acid |
|---|---|---|---|---|
| 5mM | 0.5mM | 0.05mM | 60mM | 5mM |

### (Example 7: Polymer residual sensor)

### 7. Examination using fluorescent cationic polymer

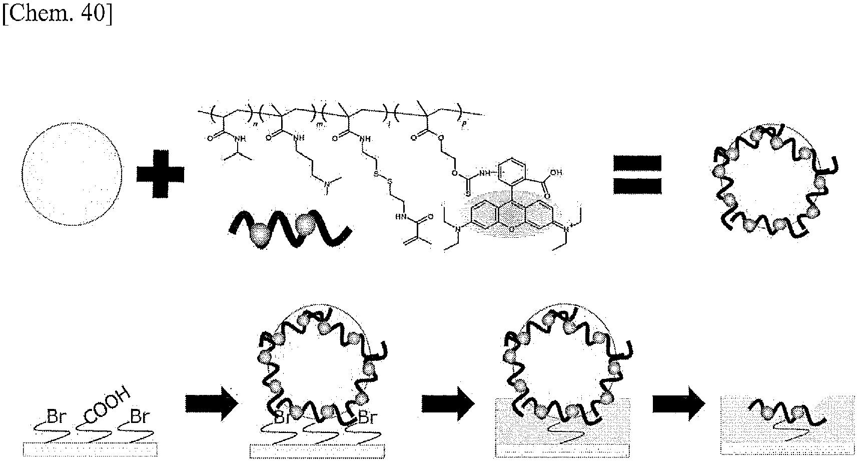

### 7-1. Experiment

### 7-1-1. Surface modification of gold substrate

A gold substrate (9.8 mm × 4.3 mm) was rinsed with EtOH and then subjected to UV-O₃ treatment for 15 min to clean up a substrate surface.

A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 7-1-2. Preparation of particle-immobilized substrate

A silica nanoparticle dispersion (particle size: 200 nm, particle concentration: 2.0 × 10¹¹ particles/mL) and the polymer F1 (1.0 mg/mL) were mixed in water at the above-described final concentration to form a complex.

The complex solution was diluted with DMF, dropped (4 µL) on the area of the substrate on which the complex was immobilized as shown in Table 6-1, and allowed to stand at 25°C for 1 hour.

The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Table 6-1.

**[Table 6-1]**

| Table 6-1 Experiment condition table (polymer F1) | | | | | | | |
|---|---|---|---|---|---|---|---|
| No. | Number of particles [particles/4 µL] | Fluorescence microscope measurement conditions (exposure time) | | | | | |
| | | Green | | | Red | | |
| | | After particle immobilization | After polymerization | After template removal | After particle immobilization | After polymerization | After template removal |
| 1 | 1×10⁷ | 0.1 sec | 0.1 sec | 0.2 sec | 0.01 sec | 0.01 sec | 0.02 sec |
| 2 | 3×10⁶ | ↑ | ↑ | ↑ | ↑ | ↑ | ↑ |
| 3 | 1×10⁶ | ↑ | ↑ | 0.5 sec | ↑ | ↑ | 0.05 sec |

### 7-1-3. Synthesis of silica nanoparticle imprinted polymer on particle-immobilized substrate

Prepolymer solutions having compositions shown in Table 6-2 were prepared, dissolved oxygen was removed by freeze-degassing, and then polymerization was performed at 25°C for 18 hours in a glove box. The substrate after polymerization was washed with pure water.

The substrate was immersed in a solution of methanol : pure water = 1 : 1, and an ultrasonic treatment was performed for 0.5 min to remove silica nanoparticles, thereby forming pores in a surface layer of a polymer thin film.

**[Table 6-2]**

| Table 6-2 Prepolymer solution composition | | |
|---|---|---|
| | Sample | Concentration |
| Main raw material | MPC (2-methacryloyloxyethyl phosphorylcholine) | 500 mM |
| Initiator | EBIB (ethyl α-bromoisobutyrate) | 1.25 mM |
| Ligand | TPMA (tris(2-pyridylmethyl)amine) | 0.125 mM |
| Catalyst | CuBr₂ | 0.0125 mM |
| Reducing agent | Ascorbic acid | 1.25 mM |
| Solvent | EtOH | 270 *µ*L |

7-1-4. Preparation of sensor substrate for direct analysis (direct sensing chip)

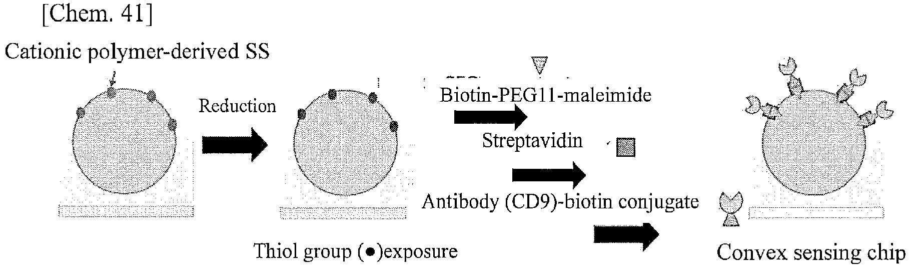

After a polymer layer was formed on the substrate in 7-1-3, the substrate was immersed in a 50 mM tris(2-carboxyethyl)phosphine hydrochloride aqueous solution and reacted at 25°C for 1 h to reduce a particle surface cationic polymer, thereby producing a thiol group.

A 100 µM biotin-PEG 11 maleimide solution (10 mM phosphate buffer containing 10% DMSO (pH 7.4)) was added dropwise to the thiol group, and the mixed solution was reacted at 25°C for 1 hour to modify biotin. The substrate after reaction was washed with pure water.

A 1 µM streptavidin solution (140 mM NaCl-containing 10 mM phosphate buffer (pH 7.4): PBS) was dropped on the substrate modified with biotin, and a reaction was allowed to proceed 25°C for 1 hour to modify streptavidin. The substrate after reaction was washed with PBS.

A 0.1 µM antibody-biotin conjugate solution (PBS) was dropped on the substrate modified with streptavidin and reacted at 25°C for 1 hour to obtain a sensor substrate for analysis (direct sensing chip) having a fluorescent molecule derived from an antibody and a cationic polymer. The substrate after reaction was washed with PBS.

### 7-1-5. Evaluation of sensor substrate for analysis (direct sensing chip)

The sensing chip obtained in 7-1-4 was mounted in a flat pipette tip manufactured by FUKAE KASEI Co., Ltd., and a change in fluorescence intensity on the surface of the substrate was measured with an automatic dispensing device with a fluorescence microscope.
1. Chip attachment
2. Suction and discharge of 150 µL PBS were repeated 10 times to clean inside of substrate and chip
3. Suction of 100 µL of sample (exosome derived from PC3)
4. Reaction 1 min (25°C)
5. Discharge sample
6. Suction of 150 µL of 10 mM PBS (140 mM NaCl, pH 7.4)
7. Measurement (objective lens x5, cy3 filter, lamp intensity 12, exposure time 0.5 sec)

### 7-2. Results

### 7-2-1. Preparation of particle-immobilized substrate

Table 6-3 shows the results of particle size and surface Z-potential measurement with Zeta Sizer for the complex of silica nanoparticles and a fluorescent cationic polymer F1. Since the surface Z potential changed from negative to positive before and after formation of the complex, it was confirmed that the silica nanoparticles and the fluorescent cationic polymer F1 formed a complex, and it was also confirmed from the particle size and the PDI that the particles were not agglomerated and the dispersibility was not impaired.

**[Table 6-3]**

| Table 6-3 Particle size and surface Z-potential measurement results by Zeta Sizer | | | |
|---|---|---|---|
| Object to be measured | Particle size | PDI | Surface Z-potential |
| Silica nanoparticle | 201.2 nm | 0.01824 | -29.97 mV |
| Silica nanoparticle + F1 complex | 225.8 nm | 0.1005 | 57.94 mV |

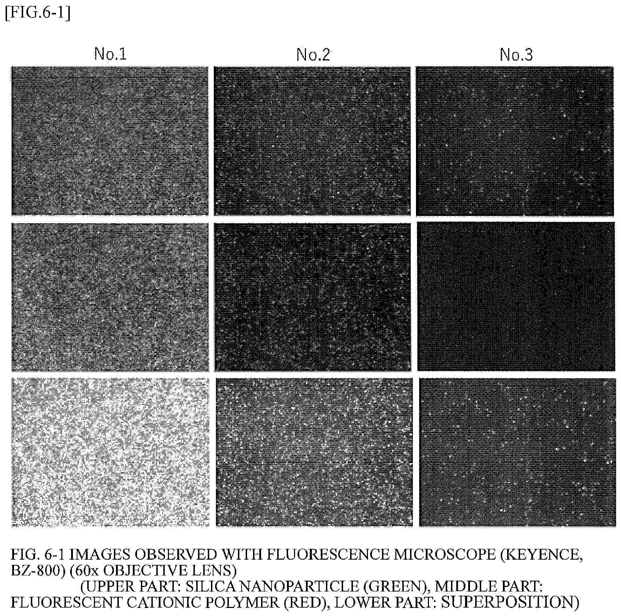

In addition, the observation results with a fluorescence microscope are illustrated in FIG. 6-1. From the fact that green fluorescence derived from the silica nanoparticles and red fluorescence derived from the fluorescent cationic polymer were confirmed in all the samples, it was confirmed that the silica nanoparticle-fluorescent cationic polymer complex was immobilized on the substrate.

### 7-2-2. Confirmation of fluorescent cationic polymer F1 after removal of silica nanoparticles

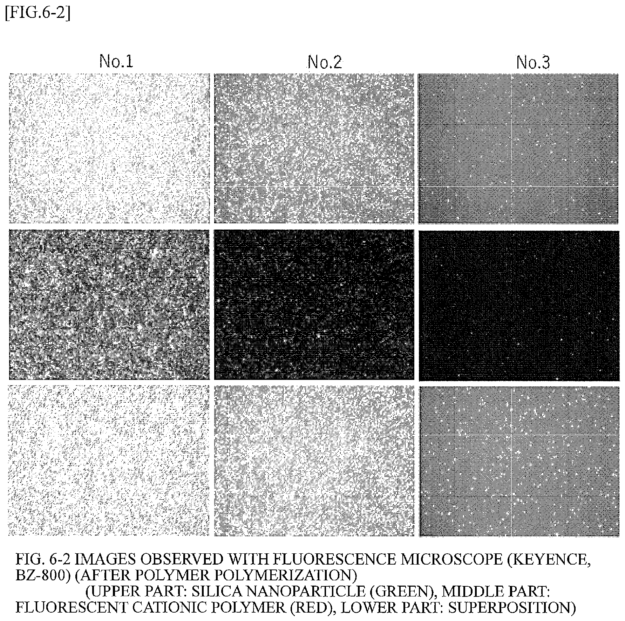

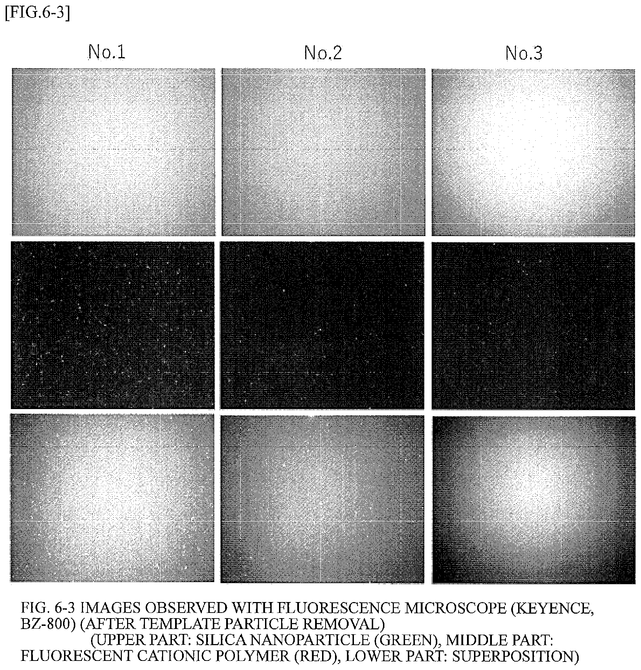

The results of observation with a fluorescence microscope after polymerization of the polymer layer on the substrate after immobilization of the silica nanoparticle-fluorescent cationic polymer complex and the results of observation with a fluorescence microscope after removal of the silica nanoparticles after polymerization are illustrated in FIGS. 6-2 and 6-3, respectively.

From FIG. 6-2, the fluorescence derived from the silica nanoparticles and the fluorescent cationic polymer was confirmed on the substrate even after polymerization. However, from FIG. 6-3 after removal of the silica nanoparticles, the green fluorescence derived from the silica nanoparticles was confirmed, but the red fluorescence derived from the fluorescent cationic polymer was confirmed. From this, it was confirmed that the fluorescent cationic polymer remained in the polymer layer (in the pores after removal of silica nanoparticles) prepared on the substrate even after the silica nanoparticles were removed by an experimental operation.

### 7-2-3. Evaluation of sensor substrate for direct analysis

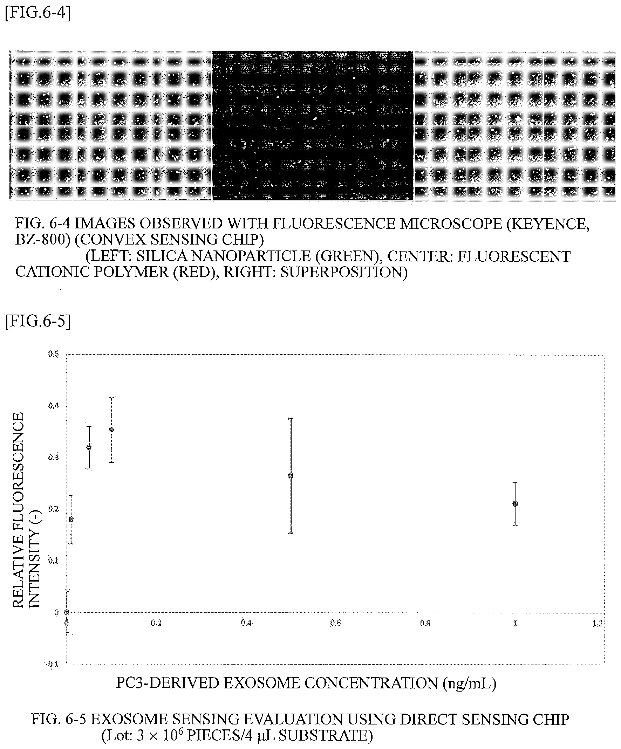

From FIG. 6-4, green fluorescence derived from silica nanoparticles and red fluorescence derived from a cationic polymer were confirmed, and thus, it was confirmed that the direct sensing chip had these elements on the substrate. The results of the exome sensing experiment using the substrate are illustrated in FIG. 6-5. From this result, enhanced fluorescence of the fluorescent molecule (rhodamine B) due to addition of the exosome was confirmed, and it was confirmed that the direct sensing chip had exosome detection ability.

### (Example 8)

### 8. Comparative examination of non-covalent bonding type and covalent bonding type in template particle immobilization

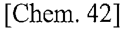

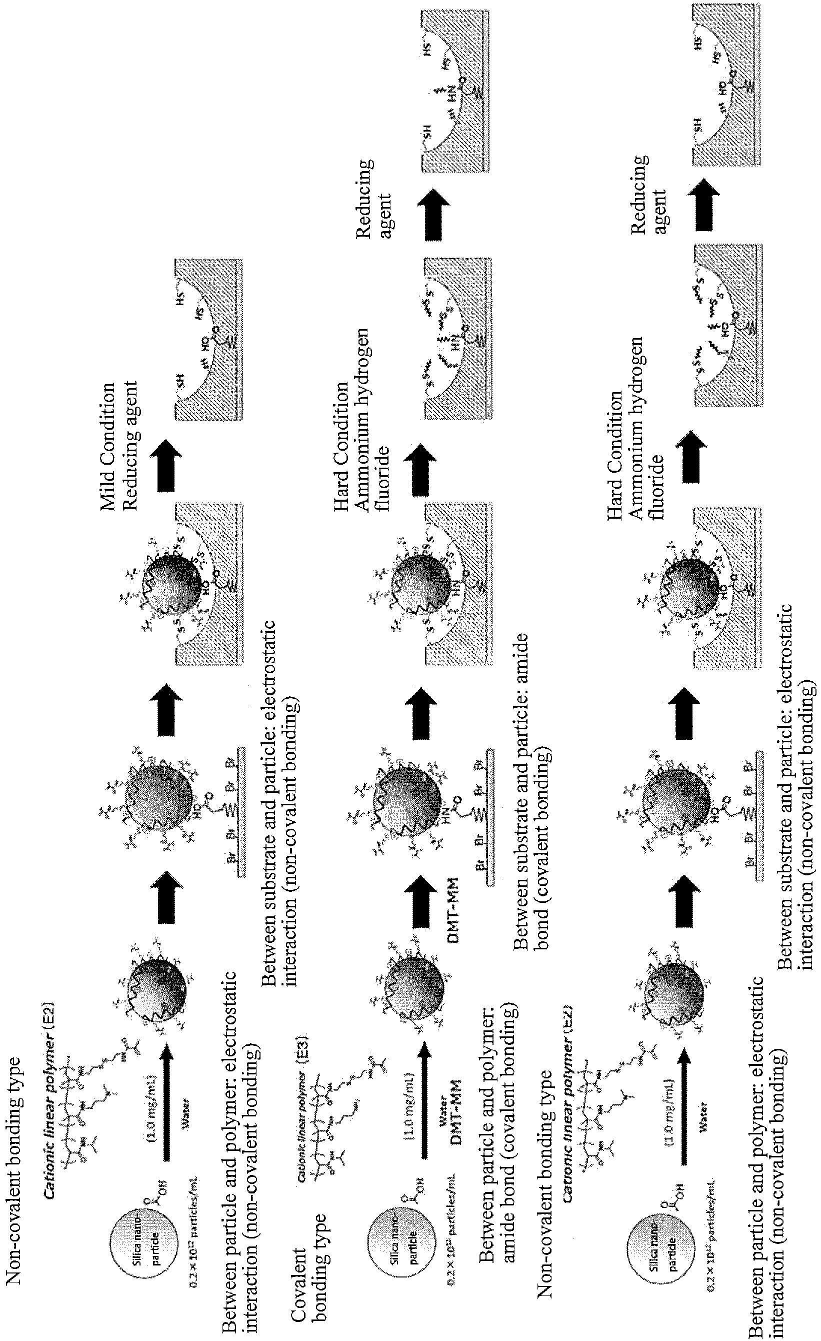

### 8-1-1. Surface modification of gold substrate

A gold substrate (9.8 mm × 4.3 mm) was rinsed with EtOH and then subjected to UV-O3 treatment for 20 min to clean up a substrate surface.

An EtOH solution obtained by mixing a 1 mM 2-(2-bromoisobutyryloxy)undecyl thiol solution and a 1 mM carboxy-EG6 undecanethiol (each final concentration was 0.5 mM) at a ratio of 1 : 1 was prepared, and the gold substrate was immersed in the solution. After the substrate was allowed to stand at 30°C for 20 hours, the substrate was washed with EtOH to form a self-assembled monolayer (SAM) on the surface of the gold substrate.

### 8-1-2. Preparation of particle-immobilized substrate

### 8-1-2-1. Preparation of covalent particle-immobilized substrate

E3 conjugated silica NP silica nanoparticles (particle size: 200 nm, particle concentration: 2.0 × 10¹¹ particles/mL) were diluted with DMF, and a solution obtained by adding DMT-MM thereto so as to be 5 mM was dropped (4 µL) on the area of the substrate as in Table 7-1, and the substrate was allowed to stand at 25°C for 1 hour.

The substrate was washed with EtOH and pure water in this order to obtain a particle-immobilized substrate.

The substrate was evaluated with a fluorescence microscope (KEYENCE, BZ-800). The measurement conditions are shown in Table 7-1.

### 8-2-2. Preparation of non-covalent particle-immobilized substrate

A silica nanoparticle dispersion (particle size: 200 nm, particle concentration: 2.0 × 10¹¹ particles/mL) and the polymer E2 (1.0 mg/mL) were mixed in water at the above-described fmal concentration to form a complex.

The complex solution was diluted with DMF, dropped (4 µL) on the area of the substrate on which the complex was immobilized so as to have the number shown in Table 7-2, and allowed to stand at 25°C for 1 hour.

The substrate was washed with EtOH and pure water in this order to obtain a particle-immobilized substrate.

The substrate was evaluated with a fluorescence microscope (KEYENCE, BZ-800). The measurement conditions are shown in Table 7-2.

**[Table 7-1]**

| Table 7-1 Experiment condition table (covalent bonding type) | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁷ | 1.0 sec | 0.2 sec | 0.02 sec |
| 2 | 1×10⁶ | ↑ | ↑ | ↑ |
| 3 | 1×10⁵ | ↑ | ↑ | ↑ |

**[Table 7-2]**

| Table 7-2 Experiment condition table (non-covalent bonding type, polymer: E2) | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 4 | 1×10⁷ | 1.0 sec | 0.2 sec | 0.02 sec |
| 5 | 1×10⁶ | ↑ | ↑ | ↑ |
| 6 | 1×10⁵ | ↑ | ↑ | ↑ |

8-1-3. Synthesis of silica nanoparticle imprinted polymer on particle-immobilized substrate

After a prepolymer solution and an ascorbic acid solution were prepared with the composition shown in Table 7-3 and dissolved oxygen was removed by freeze freeze-degassing, the solution was mixed in a glove box and polymerization was performed at 25°C for 20 hours. The substrate after polymerization was washed with pure water.

**[Table 7-3]**

| Table. 7-3 Prepolymer solution composition | | |
|---|---|---|
| | Sample | Concentration |
| Main raw material | MPC (2-methacryloyloxyethyl phosphorylcholine) | 500 mM |
| Initiator | EBIB (ethyl *α*-bromoisobutyrate) | 1.25 mM |
| Ligand | TPMA (tris(2-pyridylmethyl)amine) | 0.125 mM |
| Catalyst | CuBr₂ | 0.0125 mM |
| Reducing agent | Ascorbic acid | 1.25 mM |
| Solvent | EtOH | 270 *µ*L |

### 8-1-4. Cutting out silica particle

A 30 mM ammonium hydrogen fluoride aqueous solution was dropped on the substrate (covalent, non-covalent) to remove silica nanoparticles. The substrate after removal of silica particles was immersed in a 50 mM tris(2-carboxyethyl)phosphine hydrochloride solution (pure water : methanol = 1 : 1) and reacted at 40°C for 1 h to produce thiol groups in the pores after removal of the silica particles by reduction.

The substrate after removal of the silica particles was evaluated with a fluorescence microscope (KEYENCE, BZ-800). The measurement conditions are shown in Tables 7-4 and 7-5.

**[Table 7-4]**

| Table 7-4 Experiment condition table (covalent bonding type) | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁷ | 1.0 sec | 1 sec | 0.2 sec |
| 2 | 1×10⁶ | ↑ | ↑ | ↑ |
| 3 | 1×10⁵ | ↑ | ↑ | ↑ |

**[Table 7-5]**

| Table 7-5 Experiment condition table (non-covalent bonding type, polymer E2) | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 4 | 1×10⁷ | 1.0 sec | 1 sec | 0.2 sec |
| 5 | 1×10⁶ | ↑ | ↑ | ↑ |
| 6 | 1×10⁵ | ↑ | ↑ | ↑ |

### 8-1-5. Post-modification in pores

A 100 µM biotin-PEG 11 maleimide solution (10 mM phosphate buffer containing 10% DMSO (pH 7.4)) was added dropwise to the thiol group produced in the pore by reduction, and the mixed solution was reacted at 25°C for 1 hour to modify biotin. The substrate after reaction was washed with pure water.

A 1 µM streptavidin solution (140 mMNaCl-containing 10 mM phosphate buffer (pH 7.4): PBS) was dropped on the substrate modified with biotin, and a reaction was allowed to proceed 25°C for 1 hour to modify streptavidin. The substrate after reaction was washed with PBS.

A 0.1 µM mixed biotin solution (PBS, Antibody-biotin conjugate : Cy5-PEG-biotin conjugate = 1 : 1) was dropped on the substrate modified with streptavidin and reacted at 25°C for 1 hour to obtain a sensing chip into which an antibody and a fluorescent molecule were introduced. The substrate after reaction was washed with PBS.

### 8-1-6. Evaluation of sensing chip

The sensing chip obtained in 8-1-5 was mounted in a flat pipette tip manufactured by FUKAE KASEI Co., Ltd., and a change in fluorescence intensity on the surface of the substrate was measured with an automatic dispensing device with a fluorescence microscope.
1. Chip attachment
2. Suction and discharge of 150 µL PBS were repeated 10 times to clean inside of substrate and chip
3. Suction of 100 µL of sample (exosome derived from PC3)
4. Reaction 1 min (25°C)
5. Discharge sample
6. Suction of 150 µL of 10 mM PBS (140 mM NaCl, pH 7.4)
7. Measurement (objective lens x5, cy5 filter, lamp intensity 12, exposure time 0.5 sec)

### 7-2. Results

### 8-2-1. Preparation of particle-immobilized substrate

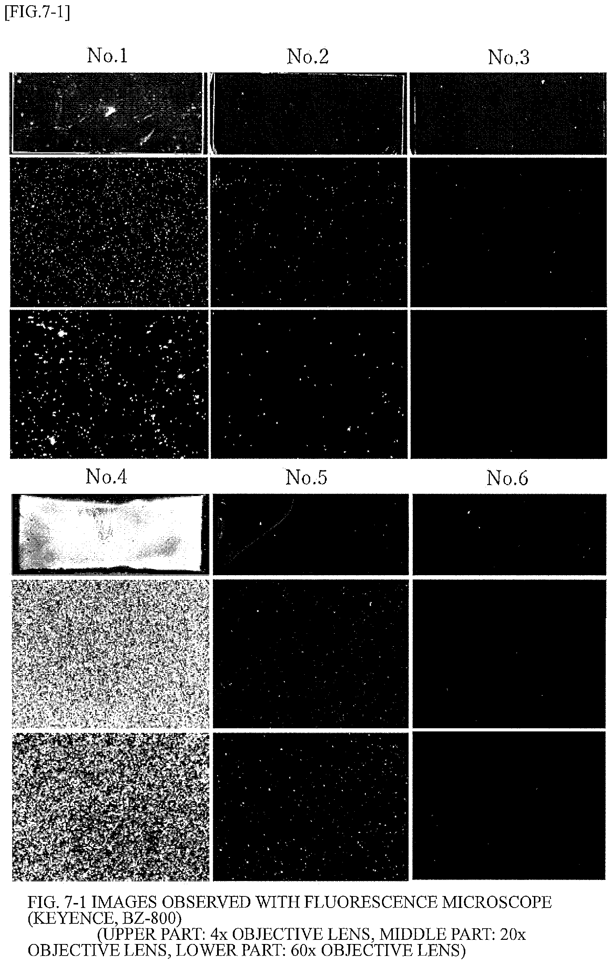

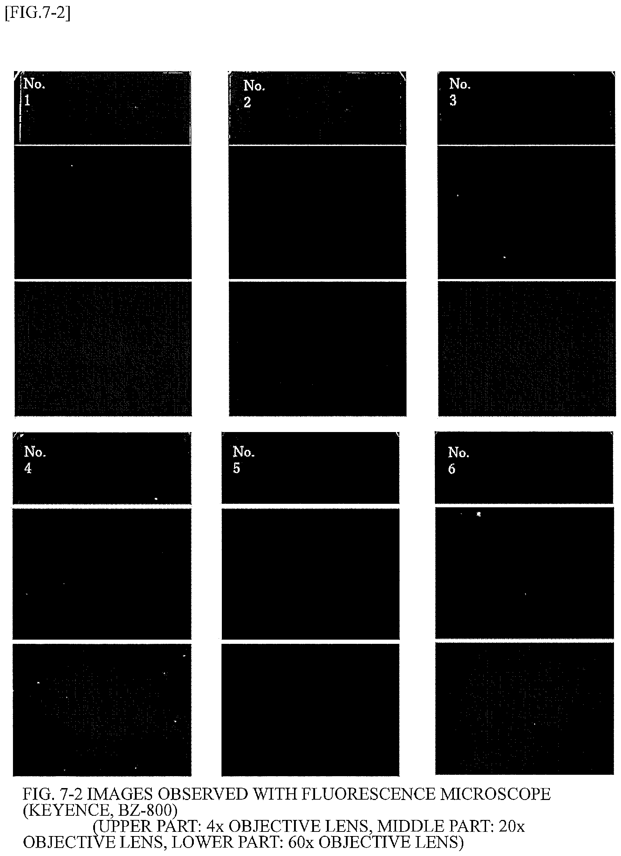

The observation results with a fluorescence microscope are illustrated in FIG. 7-1. The fluorescence derived from the silica nanoparticles was confirmed on the substrate under any conditions, and thus it was confirmed that the silica nanoparticles were immobilized on the substrate. FIG. 7-2 illustrates results after the ammonium hydrogen fluoride treatment observed with a fluorescence microscope. Under any conditions, almost no fluorescence derived from silica nanoparticles was observed on the substrate, and it was confirmed that the silica nanoparticles were removed by an ammonium hydrogen fluoride treatment.

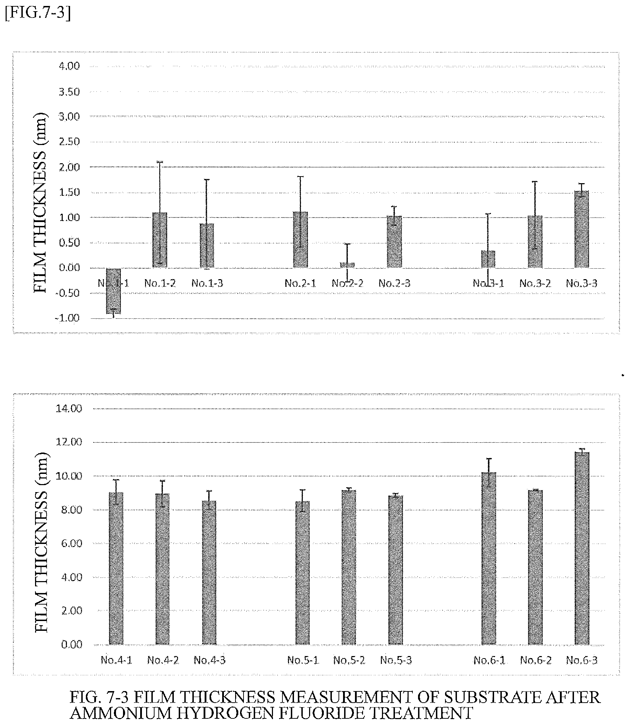

FIG. 7-3 illustrates film thickness measurement results with the ellipsometer after the ammonium hydrogen fluoride treatment. For the covalent type, the film thickness formed was around 1 nm. The non-covalent type was measured to be around 9 to 10 nm.

### 8-2-3. Post-modification in pores

A 100 µM biotin-PEG 11 maleimide solution (10 mM phosphate buffer containing 10% DMSO (pH 7.4)) was added dropwise to the thiol group produced in the pore by reduction, and the mixed solution was reacted at 25°C for 1 hour to modify biotin. The substrate after reaction was washed with pure water.

A 1 µM streptavidin solution (140 mM NaCl-containing 10 mM phosphate buffer (pH 7.4): PBS) was dropped on the substrate modified with biotin, and a reaction was allowed to proceed 25°C for 1 hour to modify streptavidin. The substrate after reaction was washed with PBS.

A 0.1 µM mixed biotin solution (PBS, Antibody-biotin conjugate : Cy5-PEG-biotin conjugate = 1 : 1) was dropped on the substrate modified with streptavidin and reacted at 25°C for 1 hour to obtain a sensing chip into which an antibody and a fluorescent molecule were introduced. The substrate after reaction was washed with PBS.

### 8-2-4. Evaluation of sensing chip

The results of evaluating the recognition ability of the exosome using the concave sensing chip prepared by a non-covalent bonding are shown below. From this result, in the sensing chip in which the particles were immobilized on the substrate at a concentration of 1 × 10⁶ particles/4 µL, the fluorescence intensity change of the fluorescent molecule by the exosome recognition was the largest, and the fluorescence intensity change increased in the order of 1 × 10⁶ particles/4 µL, 1 × 10⁷ particles/4 µL, 1 × 10⁸ particles/4 µL, and 1 × 10⁵ particles/4 µL.

It was considered that the sensitivity was lowered because the density of the recognized holes on the substrate was low at 1 × 10⁵ pieces/4 µL with the smallest change, and the fluorescence background was increased at 1 × 10⁷ pieces/4 µL and 1 × 10⁸ pieces/4 µL, such that the change in relative fluorescence intensity was small.

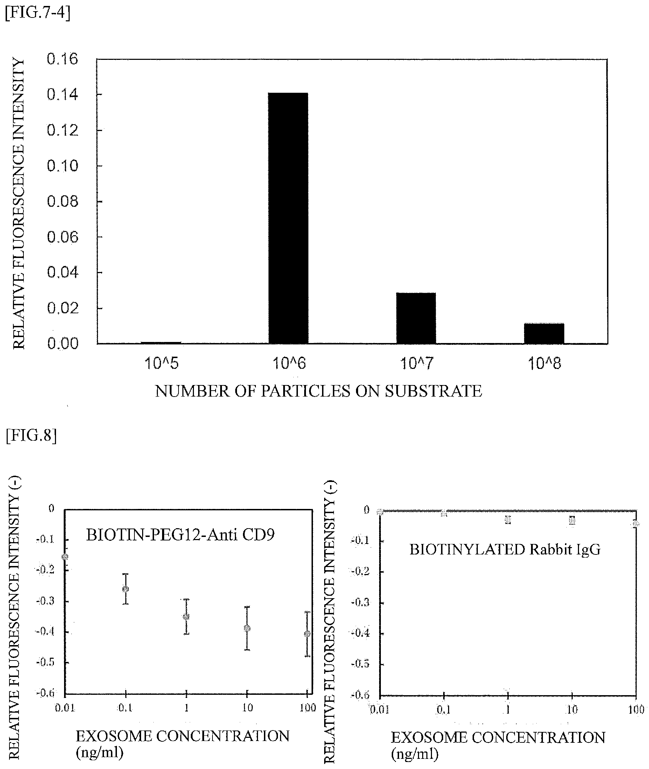

From the above, the recognized pore density of the substrate surface was controlled, such that the exosome recognition ability (detection sensitivity) was improved. (FIG. 7-4)

### (Example 9)

Fluorescence introduction using amine polymer (PD-NIPAM-Amine) with pyridyl disulfide introduced at terminal

### (Experimental operation)

### (SAM formation on substrate)

The gold substrate was immersed (30°C, 20 h) in a 0.5 mM 2-(2-bromoisobutyryloxy)undecyl thiol and 0.5 mM carboxy-EG6 undecanethiol EtOH solution to form an SAM.

### (Preparation of particles)

Polymer that clings to COOH introduced silica particle (particle core, 200 nm) aqueous suspension

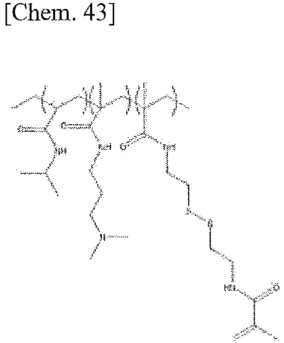

### Polymer that clings to particle core

The solution was mixed with an aqueous solution so as to have final concentrations of 1.67 mg/mL and 0.1 mg/mL, respectively, immediately diluted 250 times with DMF, and 4 µL of the solution was dropped on a substrate (3.2 × 10⁶ drops/4 µL) (25°C, 1 h).

### (Polymer matrix synthesis)

The substrate on which the core-shell particles were immobilized was washed with EtOH, and then a thin film was formed (40°C, 18 h) by atom transfer radical polymerization using the raw materials in the following table.

**[Table 8]**

| MPC : EBIB = 800 : 1 | | |
|---|---|---|
| | MPC | 500 mM |
| | Ethyl α-bromoisobutyrate | 0.625 nM |
| | TPMA | 0.0625 mM |
| | CuBr₂ | 6.25 µM |
| | L-ascorbic acid | 0.625 mM |
| | EtOH | 270 µL |

### (Removal of particle core)

After washing with ethanol and pure water, the substrate was immersed in a solution obtained by mixing acetic acid and ethanol at a ratio of 1 : 1 and applied to an ultrasonic cleaner (Bransonic) for 5 minutes.

### (Removal of copolymerized polymer)

The substrate was immersed in a solution in which a 100 mM TCEP aqueous solution and methanol were mixed at a ratio of 1 : 1, and stirring was performed at 40°C and 1,000 rpm for 1 h by a thermoshaker.

### (Introduction of biotin and PD-NIPAM-Amine)

30 µL of a solution obtained by mixing 100 µM biotin-PEG11-maleimide in PB (10% DMSO) and a 0.15 mg/mL PD-NIPAM-amine aqueous solution at a ratio of 1 : 1 was dropped on the substrate. (25°C, 1 h)

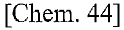

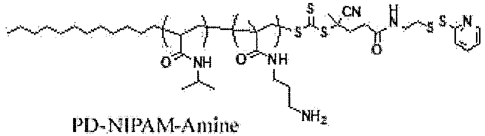

### (Introduction of fluorescent molecule (ATTO647) into PD-NIPAM-Amine)

30 µL of 100 µM ATTO 647 NHS in a 0.1 M sodium bicarbonate buffer with pH 8.0 (10% DMSO) was dropped (25°C, 1 h) on the substrate.

### (Introduction of streptavidin)

30 µL of 0.1 µM streptavidin in PBS was added dropped (25°C, 30 min).

### (Introduction of biotinylated antibody (Anti-CD9 antibody))

0.1 µM Biotin-PEG 12-Anti-CD9 in PBS (25°C, 30 min) was prepared and the substrate was stored in the PBS. Fluorescence measurement was performed under the following conditions using a fluorescence microscope equipped with a SIC automatic dispensing device
Fluorescence microscope
Camera: Zyla 4.2
Filter: Cy5
Objective lens: ×5
Exposure time: 0.5 sec
Light amount: 12%
Light source: mercury lamp
Sequence of automatic dispensing device
   1. Chip attachment
   2. Suction and discharge of 150 µL of PBS 10 times
   3. Suction of 100 µL of sample (exosome derived from PC3)
   4. Reaction 1 min (25°C)
   5. Total discharge
   6. Suction of 150 µL of 10 mM PBS (140 mM NaCl, pH 7.4)
   7. Measurement position keep Repeat 3 to 7

### (Comparison of biotin-PEG12-Anti-CD9 with biotinylated Rabbit IgG)

The results of the comparison of biotin-PEG12-Anti-CD9 with biotinylated Rabbit IgG were summarized in FIG. 8.

### (Discussion)

When an anti-CD9 antibody recognizing the surface antigen CD9 of the exosome derived from PC3 was introduced, a fluorescence response was observed in an exosome concentration-dependent manner, but when Rabbit IgG not recognizing the surface antigen of the exosome derived from PC3 was introduced, an exosome concentration-dependent response was not observed. This indicates that the binding molecule and the signal substance are disposed in the formed molecular imprint scaffold (for example, a concave portion or a convex portion), the binding molecule selectively captures the target, and the signal substance reads the capturing behavior.

### (Example 10: Complexation of His-Tag polymer and silica nanoparticles)

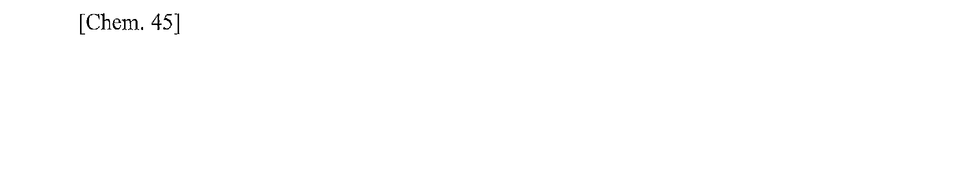

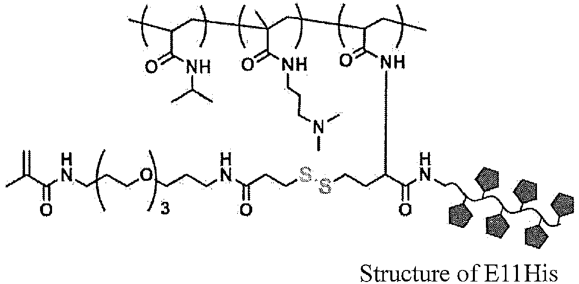

**[Table B]**

| | | Mw. | | | |
|---|---|---|---|---|---|
| | Sample | | Concentration (mM) | Final liquid volume (mL) | Weight (mg) |
| | N-Acryloylhomocysteine thiolactone | 171. 22 | 100 | 1 | 17 |
| | N-(3-(Dimethylamino)propyl)methacrylamide | 170.25 | 200 | 1 | 35 |
| NIPAm | | 113.16 | 700 | 1 | 79 |
| | 2-Cyano-2-[(dodecylsulfanylthiocarbonyl)sulfanyl]propane | 345. 63 | 10 | 1 | 3. 5 |
| ATBN | | 164.21 | 5 | 1 | 0.8 |

The compound of the recipe was placed in a Schlenk flask (25 mL) and dissolved in DMF (final liquid volume: 1 mL). A RAFT agent and an initiator were added last. A stirrer was placed, degassing and replacement with argon were performed, and polymerization was performed in an oil bath (set temperature: 70°C) (17 h).

### (Stirring speed: 300 rpm)

After the polymerization, the reaction was stopped by ice-cooling the Schlenk flask and introducing air. The reaction solution was added to a large amount of diethyl ether, and the generated precipitate was collected. The precipitate was washed with diethyl ether and vacuum-dried.

5 mg of the obtained polymer, 4 mg of His-tag (a structure in which six histidines are bonded from the N-terminal, three glycines are bonded, and lysine is bonded to the C-terminal has an amidated carboxyl group at the C-terminal), 3.5 mg of pyridyldithioethyl-PEG3-methylamide and 2.8 µL of TEA were dissolved in DMF (0.3 mL), and stirring was performed for 9 hours. CH₂Cl₂ and n-hexane were added to the reaction solution, and the generated precipitate was collected and vacuum-dried to obtain a polymer (E11His).

### iii) E11His

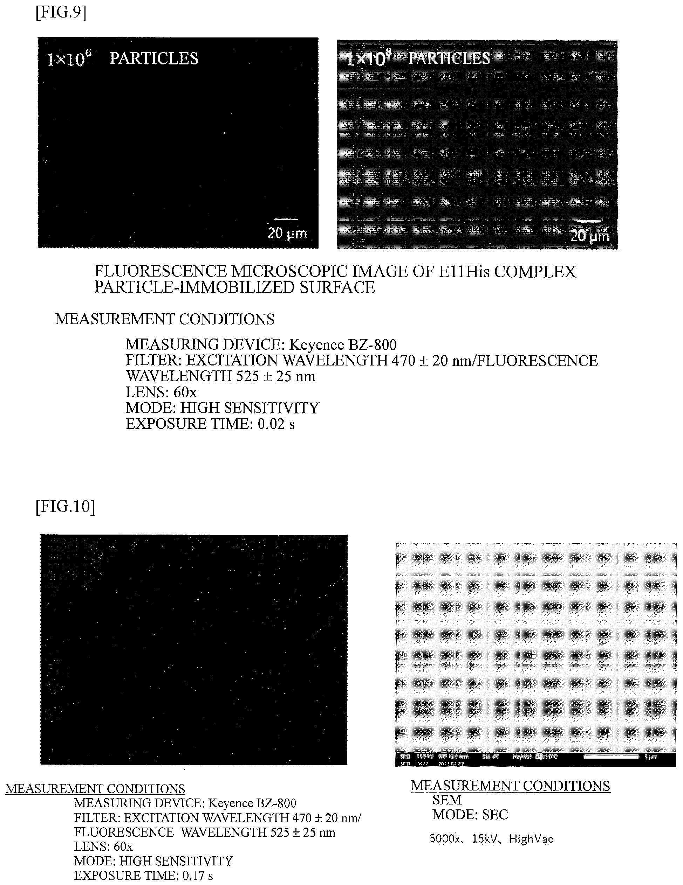

On a gold substrate modified with 2-(2-bromoisobutyryloxy)undecyl thiol and an NTA-SAM formation regent, a 4 mM NiCl₂ aqueous solution was dropped and allowed to stand (room temperature, 15 min), and then the substrate was washed with pure water. A mixed solution (silica concentration 2.0 × 10¹¹ particles/mL, polymer concentration 1 mg/mL) of silica nanoparticles (200 nm, COOH-terminated) and an E11 His aqueous solution was diluted 4-fold or 400-fold with DMF. 4 µL of the solution was dropped on the gold substrate after the reaction with NiCl₂ and allowed to stand for 1 hour. Thereafter, the surface was washed with EtOH and observed with a fluorescence microscope. The results are shown in FIG. 9.

### (Example 11: Cationic silica nanoparticle + anionic polymer Ex8)

### 1. Method for synthesizing anionic polymer Ex8

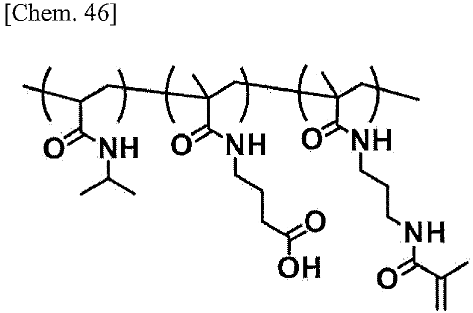

The compound of the recipe in Table C was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 70°C). (16 h)

After the polymerization, the reaction was stopped by ice-cooling the Schlenk flask and adding air, and a precipitate generated by adding a large amount of diethyl ether to the reaction solution was collected. The collected precipitate was washed with diethyl ether and then vacuum-dried to obtain a polymer.

The obtained polymer and 70 µL (0.5 mmol) of TEA were dissolved in CH₂Cl₂, stirring was performed under ice cooling, 54 mg (0.3 mmol) of N-succinimidyl methacrylate was added thereto, and stirring was performed overnight. Thereafter, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a target polymer (Ex8).

### Yield: 120 mg

**[Table C]**

| | | Mw. | | |
|---|---|---|---|---|
| | Sample | | Concen tration (mM) | Final liquid volume (mL) |
| N-(3-Aminopropyl)methacrylamide hydrochloride | | 178.66 | 100 | 1 |
| 4-[(2-Methy\|-1-oxo-2-propen-1-yl)amino]butanoic acid | | 171.19 | 200 | 1 |
| Isopropylacrylamide (NIPAm) | | 113.16 | 700 | 1 |
| 2-Cyano-2-[(dodecylsulfanylthiocarbbnyl) sulfanyl]propane | | 345.63 | 10 | 1 |
| AIBN | | 164.21 | 5 | 1 |

### 2. Complex of silica nanoparticles and polymer

After 1.98 mg (39.6 µL) of silica (Red-plane, 200 nm, 50 mg/mL) and 60.4 µL (total 100 uL) of pure water were mixed and added to 800 uL of EtOH contained in 1.5 mL Eppendorf, 100 uL of 2 M HCl and 15 uL of APTES were further added thereto, and the mixture was reacted with a thermoshaker at 25°C and 1,000 rpm for 20 h. After the reaction, the mixture was centrifugally washed with ethanol (5,000 rpm, 10 min × 3).

20 µL of the prepared silica nanoparticle dispersion (NH₂-terminated, 200 nm, 4.0 × 10¹¹ particles/mL, 50 mM carbonate buffer (pH 8.0)) and 20 µL of an Ex8 carbonate buffer solution (2 mg/mL) were mixed, and the mixed solution was allowed to stand at 25°C for 15 minutes.

### 3. DLS measurement

The complex of 2 was diluted 100-fold with pure water, and the particle size and Z-potential were measured using DLS.

### 4. Immobilization on substrate

The complex of 2 was diluted 80-fold with DMF. 4 µL of the solution was dropped on a gold substrate modified with 2-(2-bromoisobutyryloxy)undecyl thiol:amino-EG6 undecanethiol (1:1 vol), and the substrate was allowed to stand at 25°C for 1 h. Thereafter, the surface was washed with EtOH, and the surface was observed with a fluorescence microscope and an SEM (FIG. 10).

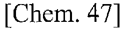

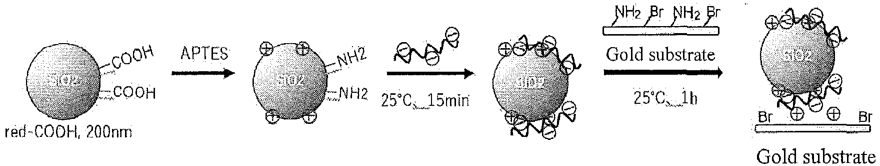

From the results of particle size measurement by DLS and the results of Z-potential measurement, it was suggested that the anionic polymer was adsorbed to the silica nanoparticles, and the surface was changed from a positively charged state to a negatively charged state. In addition, since there was no large change in particle size, it was shown that dispersibility was maintained even after polymer adsorption.

**[Table D]**

| | | Silica nanoparticle | | Ex8 complex |
|---|---|---|---|---|
| size (nm) | 221: 5 | | 251. 7 | |
| zeta (mV) | 34.4 | | -14. 4 | |

### (Results)

From the observation results of FIG. 10, it was observed that the fluorescent bright spots derived from the silica nanoparticles were monodispersed, not agglomerated, and distributed in a single layer. From this, it was shown that even when the anionic polymer Ex8 and the cationic silica nanoparticles were complexed, the particles were immobilized on the substrate in a monodisperse form, without agglomeration, and in a single layer as in the case of the cationic polymer Ex8 and the anionic silica nanoparticles.

### (Example 12: Cationic nanoparticle + anionic polymer)

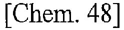

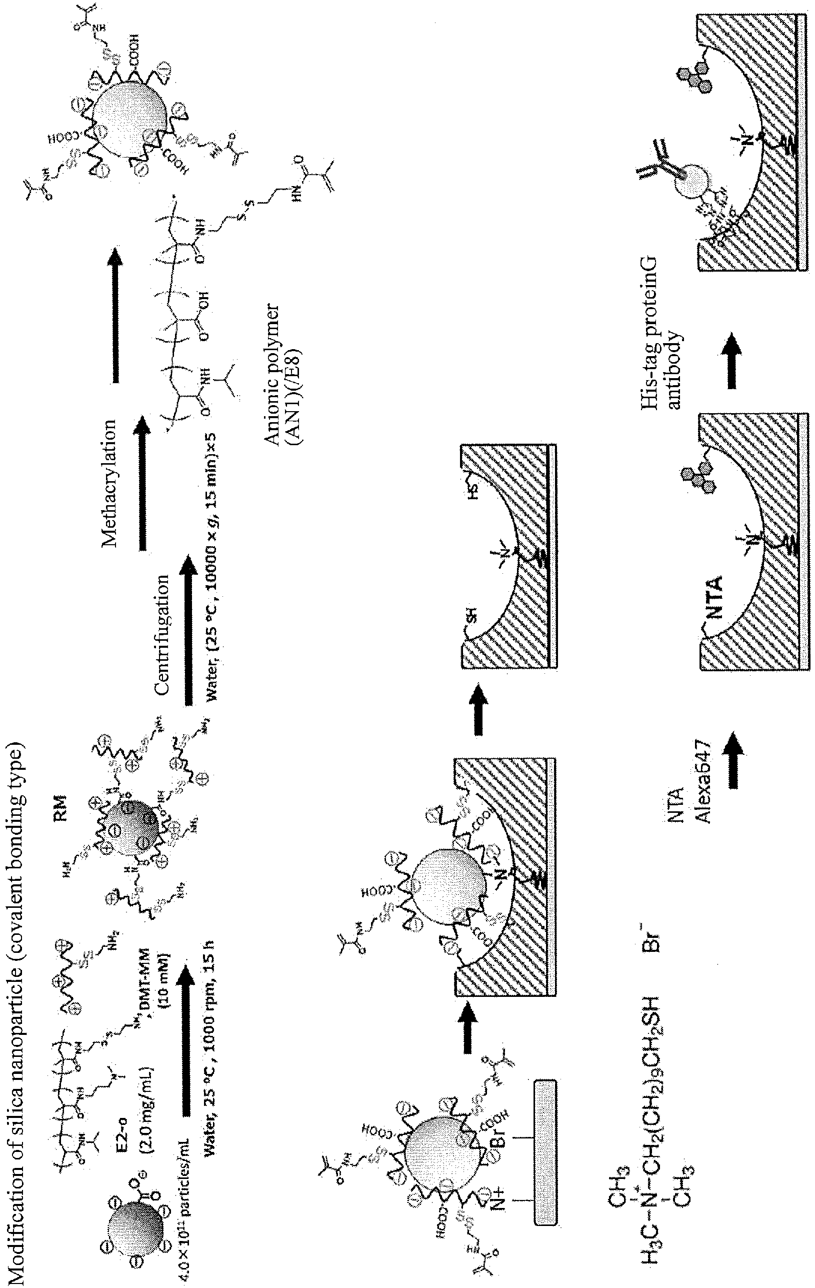

### (Silica nanoparticles used)

• E2-0 conju red-COOH silica (RM)200 nm
4.0 × 10¹¹ particles/ml

### (Polymer solution)

• Polymer final concentration 0.87 mg/mL (10 mM PB, pH 7.47)

### (Anionic silica)

• The silica nanoparticle dispersion and the polymer AN-1 were mixed to form a complex. (Silica final concentration 1.0 × 10¹¹ pieces/ml, polymer final concentration 0.435 mg/mL (5 mM PB))

The complex solution was diluted 4-fold, 40-fold, 400-fold, and 4,000-fold with DMF, dropped (4 µL) on the substrate on which the complex was immobilized, and allowed to stand at 25°C for 1 hour. The substrate was washed with ethanol to obtain a particle-immobilized substrate.

### (Polymer matrix synthesis)

Form thin film by ATRP (25°C, 20 h)

### (Removal source of particle core)

300 µL of 50% acetic acid/MeOH was placed in a 1.5 mL tube, and the substrates were immersed one by one, subjected to an ultrasonic treatment for 8 minutes, and then washed with pure water.

### (S-S reductive cleavage)

The substrate was placed in a 50 mM tris(2-carboxyethyl)phosphine hydrochloride 50% MeOH aqueous solution and reacted at 40°C for 1 h. The substrate after reaction was washed with pure water.

### (Introduction of fluorescent dye)

30 µL of a 50 µM Alexa 647-C2-maleimide and 50 µM maleimido-C3-NTA (5% DMSO 10 mM PB (7.4)) solution was dropped on a substrate, the substrate was allowed to stand at 25°C for 1 hour to allow a reaction to proceed, and then washing with pure water was performed.

A flat pipette tip manufactured by FUKAE KASEI Co., Ltd. was mounted, and a change in fluorescence intensity on the surface of the substrate was measured with an automatic dispensing device with a fluorescence microscope.

### Sequence

1. Chip attachment/PBS suction (150 µL) and image acquisition before antibody immobilization

### 2. Ni complex formation

Suction of 100 µL of 4 mM NiCl₂ (aqueous solution), reaction 5 min (25°C) Wash pure water × 4 times and suction and discharge 150 µL

### 3. His-tagged proteinG immobilization

Suction of 100 µL of 1 µM His-tagged proteinG (PBS, abeam), reaction 10 min (25°C)

### Wash

0.01% tween (registered trademark) 20 PBS × 2 times and PBS × 2 times Suction and discharge 150 µL

### 4. Antibody immobilization

Suction of 100 µL of 100 nM Anti-CD9 (commercially available product) (PBS), reaction 10 min (25°C)

### Wash

0.01% tween (registered trademark) 20 PBS × 2 times and PBS × 2 times Suction and discharge 150 µL

### 5. Background (F0: 0.1% BSA, 100 mM PB (pH 7.0), 0.15 M NaCl) 5 times of measurements

### 6. Exosome adsorption

SKBR3 culture supernatant exosome adjusted to 0, 3, 30, 300, and 1,000 fM (0.1% BSA, 100 mM PB (pH 7.0), 0.15 M NaCl)

Suction of 100 µL of sample (SKBR3-derived exosome), reaction 5 min (25°C)
Image acquisition
Fluorescence microscope
Camera: Zyla 4.2
Filter: Cy5

(Excitation wavelength 604 to 644 nm, fluorescence wavelength 672 to 712 nm)
Objective lens: ×5
Exposure time: 0.5 sec
Light amount: 12%
Light source: mercury lamp
Suction and discharge 150 µL of 0.01% tween (registered trademark) 20 PBS Repeat 1 to 6

### (Results)

For a sensing chip produced using the anionic polymer-modified silica particles for the substrate on the cationic surface, a relative fluorescence intensity change of about 5% was confirmed by addition of 10 fM of an exosome derived from SK-BR3 cell line.

### (Example 13: Biodegradable nanoparticles:polylactic acid/glycolic acid copolymer

### (PGLA))

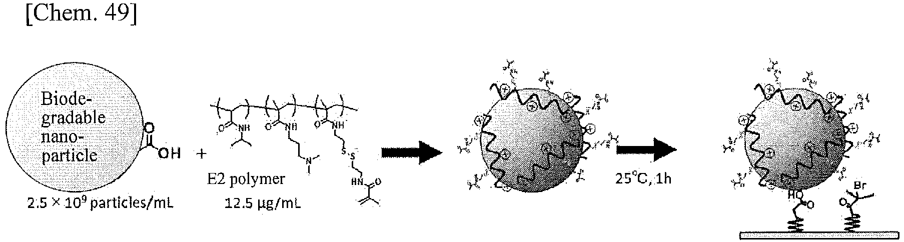

### (SAM formation on substrate)

A gold substrate was immersed in an EtOH solution with 0.5 mM 2-(2-bromoisobutyryloxy) undecyl thiol and 0.5 mM carboxy-EG6 undecane thiol to form SAM (30°C, 20 h)

### (Preparation of particles)

After the fluorescent carboxylated PGLA nanoparticle (200 nm) aqueous suspension was ultrasonicated (Lv2) for 1 min, the nanoparticles and the E2 polymer aqueous solution were mixed so that the final concentration was 2 × 10¹¹ particles/mL and 1 mg/mL, and 4 µL of a solution diluted 80-fold with EtOH was dropped on the substrate (1.0 × 10⁷ particles/4 µL) (25°C, 1 h), allowed to stand, and then washed three times with EtOH.

### (SEM observation and fluorescence observation)

After the E2 polymer aqueous solution was mixed so as to be 1 mg/mL, the solution was diluted with EtOH, 4 µL of the solution was dropped on the substrate (1.0 × 10⁷ particles/4 µL) (25°C, 1 h), and the substrate was allowed to stand and then washed with EtOH three times.

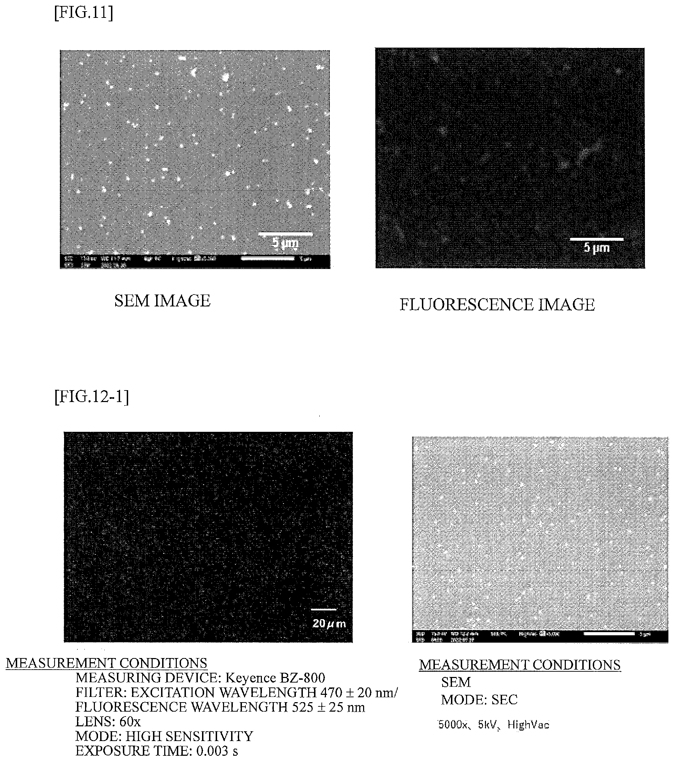

Fluorescence observation on the substrate in EtOH was performed (KEYENCE BZ-X800, excitation wavelength 470 ± 20 nm/fluorescence wavelength 525 ± 25 nm). Thereafter, vacuum drying was performed, gold sputtering was performed, and then, a scanning electron microscope (SEM) image was observed. (FIG. 11)

### (Results)

The results showed that the state was comparable to that of the silica nanoparticles or polystyrene nanoparticles, and it was found that this method was applicable to all types of core particles.

### (Example 14: Silica nanoparticle + cationic polymer E2P20 containing aromatic component)

### 1. E2P20 synthesis method

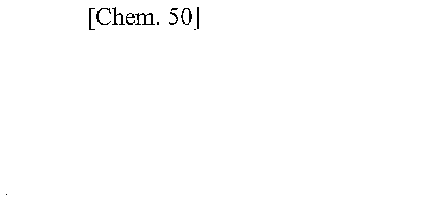

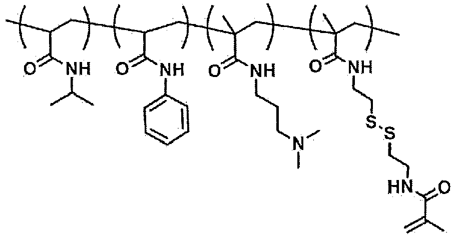

A cationic polymer E2P20 containing an aromatic component (phenyl group) was synthesized as follows.

The compound of the recipe was placed in a Schlenk flask (25 mL) and dissolved in DMF (final liquid volume: 2 mL). A RAFT agent and an initiator were added last. A stirrer was placed, degassing and replacement with argon were performed, and polymerization was performed in an oil bath (set temperature: 75°C) (24 h).

### (Stirring speed: 300 rpm)

After the polymerization, the reaction was stopped by ice-cooling the Schlenk flask and introducing air. The reaction solution was added to a large amount of diethyl ether, and the generated precipitate was collected. The precipitate was washed with diethyl ether and vacuum-dried.

The polymer prepared in the previous section and 42 µL of TEA were dissolved (or dispersed) in CH₂Cl₂, and stirring was performed. 37 mg of N-succinimidyl methacrylate was added thereto, and stirring was performed overnight. After the reaction, a precipitate generated by adding n-hexane to the reaction solution was collected. A precipitate generated by adding MeOH, CH₂Cl₂, and n-hexane to the precipitate was collected and vacuum-dried to obtain a target polymer (E2P20).

### Yield: 110 mg

**[Table E]**

| | Sample | Mw. | Conce ntratio n (mM) | Final liquid volume (mL) | Weight (mg) | Liquid volume (µL) |
|---|---|---|---|---|---|---|
| Cys metacrylamide | | 256.82 | 100 | 2 | 51.364 | - |
| N-(3-(Dimethylamino)propyl)methacrylamide | | 170.25 | 100 | 2 | 34.050 | 36.223 |
| N-Phenylacrylamide | | 147.18 | 100 | 2 | 29.436 | |
| N-isopropylacrylamide (NIPAm) | | 113.16 | 200 | 2 | 45.264 | - |
| 2-Cyano-2-[(dodecylsulfanylthiocarbonyl) sulfanyl]propane | | 345.63 | 12.5 | 2 | 8.641 | - |
| AIBN | | 164.21 | 6.25 | 2 | 2.053 | |

### 2. Complex of silica nanoparticles and polymer

40 µL of a silica nanoparticle aqueous dispersion (COOH-terminated, 200 nm, Lot.0921940-03, 4.0 × 10¹¹ particles/mL) and 40 µL of an E2P20 aqueous solution (2 mg/mL) were mixed, and the mixed solution was allowed to stand at 25°C for 15 minutes, thereby forming a complex of silica nanoparticles and E2P20.

### 3. DLS measurement

The complex of silica nanoparticles and E2P20 was diluted 100-fold with pure water, and the particle size and Z-potential were measured using DLS.

### 4. Immobilization on substrate

The complex of silica nanoparticles and E2P20 was diluted 80-fold with DMF, 4 µL of the diluted complex was dropped on a gold substrate modified with 2-(2-bromoisobutyryloxy)undecyl thiol : carboxy-EG6 undecanethiol (1 : 1), and the substrate was allowed to stand at 25°C for 1 h. Thereafter, the surface was washed with EtOH, and the surface was observed with a fluorescence microscope and an SEM.

From the results of particle size measurement by DLS and the results of Z-potential measurement, it was suggested that the cationic polymer was adsorbed to the silica nanoparticles, and the surface was changed from a negatively charged state to a positively charged state. In addition, since there was no large change in particle size, it was shown that dispersibility was maintained even after polymer adsorption.

**[Table F]**

| | | Silica nanoparticle | | After E2P20 complexation |
|---|---|---|---|---|
| size (nm) | 210.1 | | 223.2 | |
| zeta (mV) | -41.2 | | 45.8 | |

### (Results)

From the observation results illustrated FIG. 12-1, it was observed that the fluorescent bright spots derived from the silica nanoparticles were monodispersed, not agglomerated, and distributed in a single layer. The results showed that when E2P20 in which an aromatic component (phenyl group) was introduced into E2 was complexed with silica nanoparticles and immobilized on a substrate as in the case of E2, E2P20 was monodispersed, not agglomerated, and was immobilized on the substrate in a single layer.

### (Example 15: Polystyrene nanoparticle + cationic polymer E2P20 containing aromatic component)

A cationic polymer E2P20 containing an aromatic component was complexed using polystyrene nanoparticles instead of silica nanoparticles in Example 14.

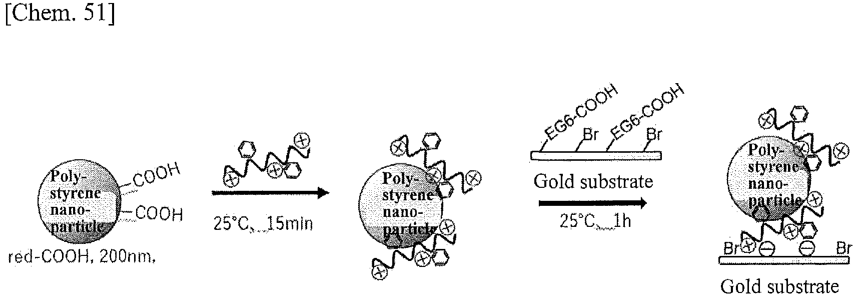

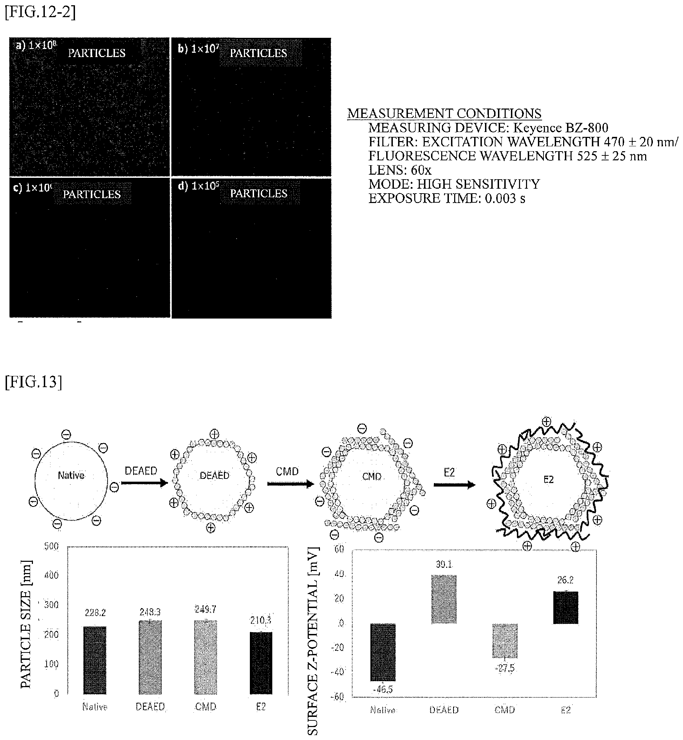

20 µL of polystyrene (PS) nanoparticles (250 nm, COOH-terminated, 2.0 × 10¹² particles/mL) and 20 µL of an E2P20 aqueous solution (2 mg/mL) were mixed, and the solution was diluted 40-fold, 400-fold, 4,000-fold, and 40,000-fold with DMF. 4 µL of the solution was dropped on a gold substrate modified with 2-(2-bromoisobutyryloxy)undecyl thiol and carboxy-EG₆ undecanethiol, and the substrate was allowed to stand for 1 hour. Thereafter, the surface was washed with EtOH and observed with a fluorescence microscope. (FIG. 12-2)

### (Results)

As in the case of the silica nanoparticles, it was observed that even when polystyrene nanoparticles were used, the fluorescent bright spots were distributed in a monodisperse form, without agglomeration, and in a single layer regardless of the number of dropped particles. From this, it was shown that even when an aromatic component was introduced into a cationic polymer, the aromatic component was immobilized in a monodisperse form, without agglomeration, and in a single layer.

### (Example 16: Preparation of particles including negatively charged core)

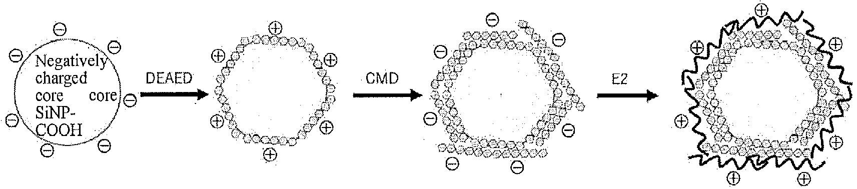

### 1. Preparation of DEAED-modified silica nanoparticles

Silica nanoparticles (2.0 × 10¹¹ particles/mL) were subjected to an ultrasonic treatment (High, Level 4.5, 5 min), and mixed with diethylaminoethyl-dextran: DEAED (1.0 mg/mL) at the concentration described above as a final concentration in water. The mixture was subjected to an ultrasonic treatment (High, Level 4.5, 5 min), and the DEAED was modified on the outermost surface of the particle by an electrostatic interaction. Excess free DEAED was removed by centrifugation (5,000 rpm, 5 min).

### 2. Modification of CMD

DEAED-modified silica nanoparticles (2.0 × 10¹¹ particles/mL) were subjected to an ultrasonic treatment (High, Level 4.5, 5 min), and mixed with carboxymethyl-Dextran: CMD (1.0 mg/mL) at the concentration described above as a final concentration in water. The mixture was subjected to an ultrasonic treatment (High, Level 4.5, 5 min), and the CMD was modified on the outermost surface of the particle by an electrostatic interaction. Excess free CMD was removed by centrifugation (5,000 rpm, 5 min).

### 3. Modification of E2B

CMD-modified silica nanoparticles (2.0 × 10¹¹ particles/mL) were subjected to an ultrasonic treatment (High, Level 4.5, 5 min), and mixed with a cationic polymer: E2 (1.0 mg/mL) at the concentration described above as a final concentration in water. The mixture was subjected to an ultrasonic treatment (High, Level 4.5, 5 min), and the E2 was modified on the outermost surface of the particle by an electrostatic interaction → The particle size and the surface Z-potential were measured by DLS measurement for each step.

DLS measurement was performed by diluting with pure water so that each particle concentration was in the order of 10⁸ particles/mL

### (Apparatus: Zetasizer Advance Ultra (manufactured by Malvern Panalytical)) (Evaluation of particles including negatively charged core)

The results of the surface Z-potential measurement by DLS are illustrated in FIG. 13, and it was found that the positive and negative of the surface Z-potential were reversed for each polymer modification step, and a particle in which a plurality of polymers having different charges were deposited on the core particle was obtained.

### (Immobilization on substrate)

### SAM formation on substrate

An EtOH solution obtained by mixing a 1 mM 2-(2-bromoisobutyryloxy)undecyl thiol solution and a 1 mM carboxy-EG6 undecanethiol (each final concentration was 0.5 mM) at a ratio of 1 : 1 was prepared, and the gold substrate was immersed in the solution. After the substrate was allowed to stand at 30°C for 20 hours, the substrate was washed with EtOH to form a self-assembled monolayer (SAM) on the surface of the gold substrate. Immobilization of particles

A stock solution of particles coated with a plurality of polymers (25 mg/mL, 3 × 10¹² particles/mL) was diluted with DMF to 1 × 10⁷ particles/4 µL, and 4 µL of the diluted solution was dropped on the substrate.

The substrate was allowed to stand at 25°C for 1 hour to immobilize the particles on the substrate, and then the substrate was washed with EtOH.

### (Polymerization and core particle removal)

### Polymerization

Prepolymer solutions were prepared according to the recipes shown in Table G, and after degassing, the solutions were dispensed into PCR tubes including substrates under an argon atmosphere, and polymerization was performed at 25°C and 500 rpm for 20 h.

Table G polymer matrix recipe:

**[Table G]**

| Compound | Amount |
|---|---|
| MPC : 2-(Methacryloyloxy) ethyl phoshorylcholine | 500 mM |
| TPMA : Tris (2-pyridylmethyl) amine | 1 mM |
| CuBr2 | 1 mM |
| L-Ascorbic acid | 0.5 mM |
| EtOH | 270 *µ* L |

### Water washing

After the polymerization, the substrate was taken out to a petri dish, excess pure water was added thereto, and the mixture was slowly stirred overnight to remove an excess MPC polymer.

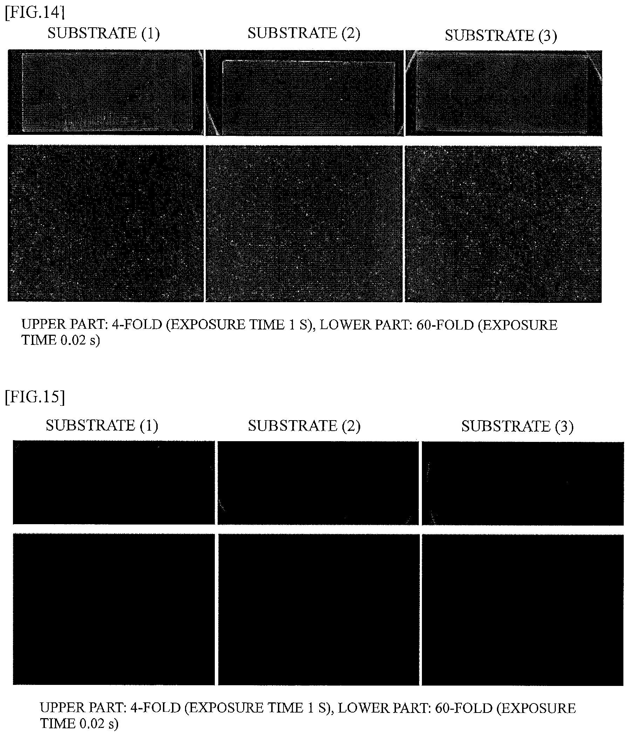

FIG. 14 illustrates fluorescence microscopic images after immobilization and polymerization.

As in the case of using a single polymer, it was found that there was no aggregation, and the polymer was uniformly immobilized and polymerized.

### Core particle removal

The substrate was placed in a 1.5 mL Eppendorf tube, 500 µL of a washing liquid (1 M NaCl in 50 mM carbonate buffer (pH 9.0)) was dispensed, and stirring was gently performed at 80°C and 250 rpm for 1 h to remove particles. Thereafter, the substrate was washed with pure water.

The surface of the substrate after polymerization and after removal of the core particles was observed with a fluorescence microscope (BZ-800). The observation conditions were an objective lens magnification of 4 times, a filter: TRITC, an exposure time of 1 second, an objective lens magnification of 60 times, a filter: TRITC, and an exposure time of 0.02 seconds.

As a result, it was confirmed that even particles coated with a plurality of polymers were immobilized on the substrate without aggregation, and that the core particles were removed from the substrate by a washing liquid. (FIG. 15)

(Example 17: Introduction of fluorescent molecules and complex formation sites into pores after removal of particles)

### (Reduction and washing of cationic polymer E2)

30 µL of a 0.5 mM TCEP-HCl aqueous solution was dropped on the substrate after core particle removal, and the substrate was allowed to stand at 25°C for 10 minutes to reduce a disulfide bond. Thereafter, washing by immersing the substrate in pure water was performed twice.

Subsequently, the substrate was placed in a 1.5 mL Eppendorf tube, 500 µL of a washing liquid (1 M NaCl in 50 mM carbonate buffer (pH 9.0)) was dispensed, and stirring was gently performed at 80°C and 250 rpm for 0.5 h to remove the cationic polymer remaining on the substrate. Thereafter, the substrate was washed with pure water.

### (Introduction of fluorescent molecule and complex formation sites to produced thiol group)

20 µL of a 50 µM Alexa 647-C2-maleimide and 50 µM maleimido-C3-NTA (5% DMSO 10 mM PB (7.4)) solution was dropped on a substrate, and the substrate was allowed to stand at 25°C for 1 hour under light-shielding to introduce the fluorescent molecules and the complex formation sites into the substrate by a reaction of a thiol group and a maleimide group. Thereafter, washing by immersing the substrate in DMSO was performed twice, and washing by immersing the substrate in pure water was performed twice.

### (Introduction of antibody into pore and detection of exosome)

### (NTA-Ni complex)

20 µL of a 4 mM NiCl₂ aqueous solution was dropped on the substrate, and the substrate was allowed to stand at 25°C for 15 min under light-shielding to form an NTA-Ni complex. Thereafter, washing by immersing the substrate in pure water was performed twice.

### (Binding of His-tagged proteinG)

20 µL of 1 µM His-tagged ProteinG in 10 mM PBS (pH 7.4) was dropped on the substrate, and the substrate was allowed to stand at 25°C for 1 h under light-shielding to introduce His-tagged ProteinG by complex formation with NTA-Ni. Thereafter, washing by immersing the substrate in PBS was performed twice

### (Binding of anti-CD9)

20 µL of 0.3 µM anti-CD9 in 10 mM PBS (pH 7.4) was dropped on the substrate, and the substrate was allowed to stand at 25°C for 1 h under light-shielding to introduce an antibody into His-tagged ProteinG. Thereafter, washing by immersing the substrate in PBS was performed twice

### Adsorption experiment

The substrate was inserted into a flat pipette tip manufactured by FUKAE KASEI Co., Ltd., and an exosome detection experiment was performed by fluorescence measurement using a fluorescence microscope with an automatic dispensing device. Measurement conditions of the fluorescence microscope were as follows: a filter was Cy5, an objective lens was 5 times, an exposure time was 0.5 seconds, a light amount was 12%, and a light source was a mercury lamp. In addition, the sample was dissolved in a 10 mM phosphate buffer (140 mM NaCl, pH 7.4) to adjust the concentration to a predetermined concentration.

### (Results)

A relative fluorescence intensity change of about 5% was observed with respect to the PC3-derived exosome concentration of 1 fM. It was found that even when a plurality of polymers were used for the modifier core, the same response as in the case of using a single polymer was exhibited

(Comparative Example 1: Silica nanoparticle agglomeration immobilization under conditions of WO 2018/221271 A and Toshifumi Takeuchi et al., J. Am. Chem. Soc., March 10, 2020, Vol. 142, Page. 6617-6624)

### 1. Introduction of His-tag and thiol group to silica nanoparticles

Fluorescent silica nanoparticles (red-COOH, 200 nm, Lot. 0442240-01) were adjusted to 25 mg/mL (3.0 × 10¹² part./mL) with water. To 1 mL of a silica nanoparticle aqueous suspension, 10 µL of a 50 mM His-tag (having a structure in which six histidines were bonded from the N-terminal, three glycines were bonded, and lysine was bonded to the C-terminal, and a carboxyl group at the C-terminal was amidated) aqueous solution, 10 µL of a 50 mM 2-aminoethanthiol-HCl aqueous solution, and 10 µL of a 50 mM DMT-MM aqueous solution were added, and the mixed solution was reacted at 25°C and 1,000 rpm for 19 hours using a thermoshaker to introduce a His-tag and a thiol group into silica nanoparticles.

### 2. Production of substrate

A gold substrate was immersed in an EtOH solution of 0.5 mM 2-(2-bromoisobutyryloxy) undecyl thiol and 0.5 mM amino-EG6 undecane thiol to introduce an amino group and a bromo group to the substrate surface (30°C, 20 h).

### 3. Introduction of NTA onto substrate to form nickel complex

80 µL of a DMSO solution of 5 mM isothiocyanobenzyl-NTA was dropped on a substrate into which an amino group and a bromo group were introduced, and the substrate was allowed to stand at 25°C for 2 hours. The substrate was washed with DMSO twice and pure water twice, and drying with N₂ was performed. 30 µL of a 4 mM NiCl₂ aqueous solution was dropped on the substrate, and the substrate was allowed to stand at 25°C for 15 minutes and washed with pure water 3 times to form a Ni complex of NTA.

### 4. Immobilization of silica nanoparticles in which His-tag and thiol group are introduced

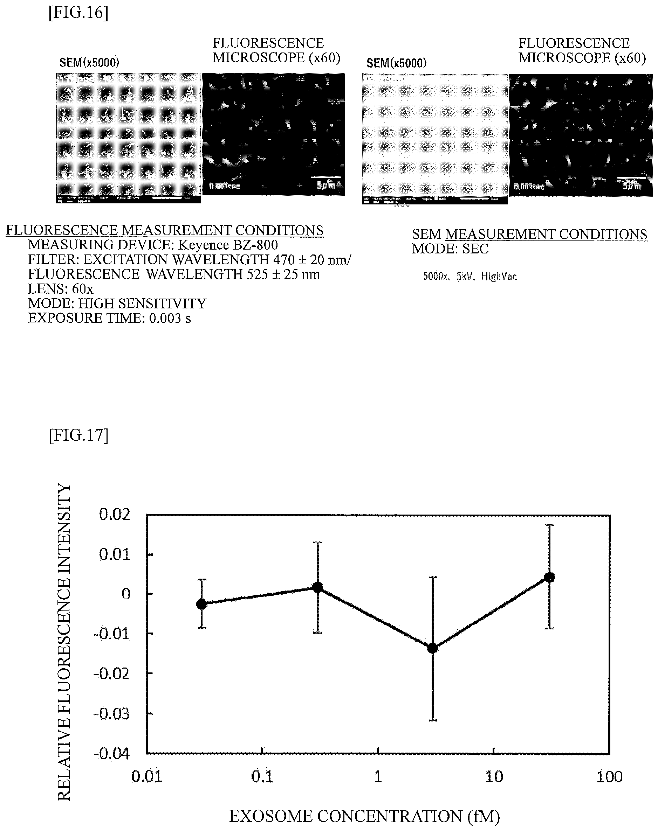

50 µL of silica nanoparticles into which a His-tag and a thiol group were introduced was dropped on the substrate using phosphate buffered saline (PBS) as a solvent, and the substrate was allowed to stand at 25°C for 1 hour. The substrate was washed with PBS 3 times, and the substrate surface was observed with a fluorescence microscope (Keyence BZ-800) and an SEM. (FIG. 16)

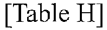

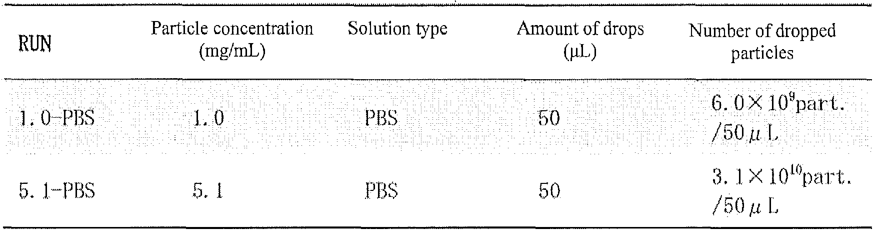

From the observation results illustrated in FIG. 16, it was observed that silica particles were partially single, but most of the silica particles were agglomerated. From this, it was shown that when the silica nanoparticles were modified with a His-tag and immobilized on the substrate, the silica nanoparticles were monodispersed, not agglomerated, and immobilized in a single layer.

### (Comparative Example 2: Sensitivity of sensor produced without using particles)

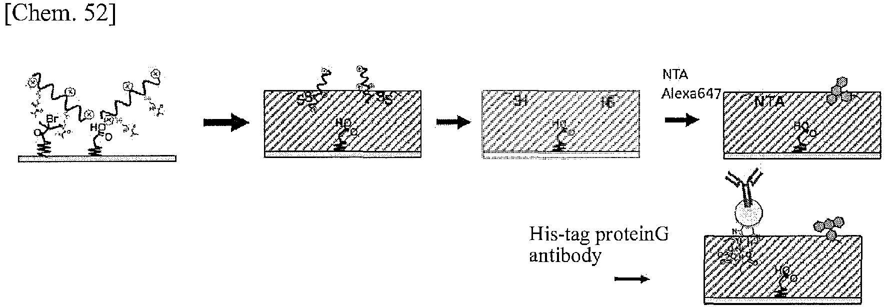

### (SAM formation on substrate)

A gold substrate (9.8 mm × 4.3 mm) was subjected to UV-O3 treatment for 20 min to clean up a substrate surface. A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH to form a self-assembled monolayer (SAM) on the surface of the gold substrate.

A polymer E2 aqueous solution (1 mg/mL) was diluted 80-fold with DMF, 4 µL of the diluted solution was dropped on the substrate on which the SAM was formed, and the substrate was allowed to stand at 25°C for 1 hour. The substrate was washed with ethanol.

### (Polymer matrix synthesis)

After a prepolymer solution and an ascorbic acid solution were prepared and dissolved oxygen was removed by freeze freeze-degassing, the solution was mixed in a glove box and polymerization was performed at 25°C for 20 hours. The substrate after polymerization was washed with pure water.

**[Table I]**

| ATRP recipe | |
|---|---|
| MFC | 500 mM |
| Ethyl α⁻bromoisobutyrate | 1.25 mM |
| TPMA | 0.125 mM |
| CuBr₂ | 0.0125 mM |
| L-Ascorbic Acid | 1.25 nM |
| EtOH | 270 *µ* L |

### (Acetic acid treatment)

The substrate was placed in a 50% MeOH and 50% acetic acid solution, subjected to an ultrasonic treatment for 5 minutes, and then washed with pure water.

### (S-S reductive cleavage)

A 0.5 mM tris(2-carboxyethyl)phosphine hydrochloride aqueous solution was placed in a thermoshaker and preheated at 40°C for 5 minutes. Then, the acetic acid-treated substrate was placed and reacted at 40°C for 1 min. The substrate after reaction was washed with pure water.

### (Fluorescence, NTA introduction)

30 µL of a 20% DMSO-containing 10 m MPB (pH 7.4) solution of 50 µM maleimido-C3-NTA and 50 µM Alexa 647-C2-maleimide was dropped on the substrate, and the substrate was allowed to stand at 25°C for 1 h and reacted and then washed with DMSO and pure water.

### (His-tagged proteinG, introduction of antibody)

30 µL of a 4 mM NiCl₂ aqueous solution was dropped on the substrate, and the substrate was allowed to stand (25°C, 15 min). The substrate after reaction was washed with pure water. 30 µL of a PBS solution of 1 µM His-tagged proteinG was dropped on the substrate, and the substrate was allowed to stand at 25°C for 1 hour to immobilize His-tagged proteinG. The substrate after His-tagged proteinG immobilization was washed with PBS.

30 µL of a PBS solution of 0.1 µM Anti-CD9 was dropped, and the substrate was allowed to stand at 25°C for 1 hour to introduce an antibody into the substrate. The substrate after introduction of the antibody was washed with PBS.

A flat pipette tip manufactured by FUKAE KASEI Co., Ltd. was mounted, and a change in fluorescence intensity on the surface of the substrate was measured with an automatic dispensing device with a fluorescence microscope.

The concentrations of the PC3 exosome particle PBS solutions were 0.03, 0.3, 3, and 30 fM.
1. Chip attachment
2. Suction of 150 µL of PBS and F0 measurement × 5
3. Suction of 100 µL of sample (exosome derived from PC3)
4. Reaction 5 min (25°C)
5. Total discharge
6. Suction of 150 µL of 10 mM PBS (140 mM NaCl, pH 7.4)
7. Automatic measurement

Fluorescence microscope
Camera: Zyla 4.2
Filter: Cy5

### (Excitation wavelength 604 to 644 nm, fluorescence wavelength 672 to 712 nm)

Objective lens: ×5
Exposure time: 0.5 sec
Light amount: 12%
Light source: mercury lamp
8. Repeat 3 to 7

### (Results)

The results are illustrated in FIG. 17. There was no response to the exosome in the non-pore-formed substrate using no silica particle core.

### (Example 18: Example of diagnosis in animal)

The exosome was measured by collecting blood of dogs and cats with cancer or visceral diseases determined by a veterinarian. After the completion, the exosome was measured again and the difference was observed to examine whether cancer and visceral disease of dogs and cats were detected.

### Use of sensor for analysis of present disclosure

### Non-human use

1. Examination of diseases including cancer of pet (cats, dogs, birds, and the like) animals
2. Detection of infectious disease causing viruses and infectious proteins (such as prions) and other pathogenic/infectious factors of livestock (cows, horses, pigs, chicken, and the like)
3. Detection of infectious disease causing viruses and infectious proteins (such as prions) and other pathogenic/infectious factors of harmful animals (birds, bats, foxes, mongooses, raccoons, and the like)
4. Quality and freshness check of marine products
5. Quality and freshness check of agricultural products
6. Check for pathogens and viruses of plants and trees

### (Example 19: Clinical application example)

Whether or not cancer can be detected by the sensor is examined by observing a difference in response between the sensors before and after a total resection surgery for the presence or absence of cancer detection under the patient's consent in a clinical study approved by a clinical research review board of a clinical experiment implementation hospital. Whether it is possible to check cancer metastasis or recurrence is examined by continuous observation. Whether the therapeutic effect of the drug therapy can be evaluated by the difference in sensor response before and after the medication is examined. Whether the lifestyle disease can be diagnosed from the relationship between the ingested food and the sensor response is examined. In addition, the presence of a cancer cell surface antigen is estimated from the sensor response, and whether companion diagnosis is possible is examined. Furthermore, it is examined whether the quality control of the cell in the treatment using the cell is possible from the sensor response.
Use of sensor for analysis of present disclosure
Cancer test
Check of remainder after cancer surgery
Check of metastasis and recurrence of cancer
Therapeutic effect of pharmacotherapy
Examination of lifestyle diseases
Companion diagnosis for dosing
Quality control of cells in cell engineering

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present disclosure are to be incorporated by reference in the present specification in the same manner as the contents is specifically described in the present disclosure.

### Industrial Applicability

The technique provided in the present disclosure is applicable to all sensing chip production based on hole formation using molecular imprinting, is required for a large amount of sensing sub-chips, and can be utilized in any field (including diagnosis) utilizing inspection analysis technique.

## Claims

1. A particle (C) comprising:
(A) a particle core; and
(B) a modifier,
wherein the particle core includes (A1) a particle core core and (A2) a functional group-containing moiety present on the particle core,
the modifier includes (B-1) a first functional group capable of binding to the particle core and capable of forming a chemical bond between molecules, and (B-2) a second functional group that imparts properties desired in the sensor for analysis, where the functional group of (B-1) and the binding group of (B-2) are the same as or different from each other, and
the particle core and the modifier are integrated with the particle core by a chemical bonding between the molecules of the functional group-containing moiety and the first functional group.

2. The particle according to claim 1, wherein the particle is used for producing a substrate for producing a sensor for analysis.

3. The particle according to claim 1 or 2, wherein the modifier includes one or a plurality of modifiers.

4. The particle according to any one of claims 1 to 3, wherein the modifier contains a substituent capable of binding to a substrate.

5. The particle according to any one of claims 1 to 4, wherein the modifier contains at least one group selected from the group consisting of a reversible linking group and a polymerizable functional group.

6. The particle according to any one of claims 1 to 5, wherein the modifier contains a polymerizable functional group.

7. The particle according to any one of claims 1 to 6, wherein the modifier further includes at least one selected from the group consisting of a group for binding a signal substance, a signal substance, and a reversible linking group.

8. The particle according to any one of claims 1 to 7, wherein the modifier contains a group for binding a specific binding molecule to a target substance and a group for binding a signal substance; a group for binding a specific binding molecule to a target substance and a signal substance; or a reversible linking group.

9. The particle according to any one of claims 1 to 8, wherein the reversible linking group is a disulfide group, an imino binding group, an oxime group, a hydrazone binding group, a boronic acid cyclic ester bond, a carboxylic acid ester group, a carboxylic acid thioester group, or an avidin-biotin bond.

10. The particle according to any one of claims 1 to 9, wherein the group for binding a specific binding molecule to a target substance is an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group.

11. The particle according to any one of claims 1 to 10, wherein the group for binding a signal substance is an amino group, a carboxyl group, a boronyl group, a thiol group, a maleimide group, a carbonyl group, an aldehyde group, an aminooxy group, a hydroxyl group, a hydrazide group, a biotin group, a nickel-nitrilotriacetic acid-derived group, a thiol group, or a pyridyl disulfide group.

12. The particle according to any one of claims 1 to 11, wherein the signal substance is a fluorescent molecule, a radioactive element-containing substance, a magnetic substance, or an enzyme.

13. The particle according to any one of claims 1 to 12, wherein the chemical bonding between molecules is a non-covalent bonding.

14. The particle according to any one of claims 1 to 13, wherein the non-covalent bonding is an electrostatic interaction.

15. The particle according to any one of claims 1 to 14, wherein the particle core is formed of silica, polystyrene, polylactic acid, polymethyl methacrylate, a polylactic acid/glycolic acid copolymer, chitosan, iron oxide, gold, silver, platinum, aluminum oxide, copper oxide, titanium oxide, zinc oxide, zirconium oxide, cerium oxide, cobalt oxide, tin-doped indium oxide (ITO), antimony-doped tin oxide (ATO), graphene, graphene oxide, a carbon nanotube, nanodiamond, or hydroxyapatite, or any combination thereof.

16. The particle according to any one of claims 1 to 15, wherein the particle core is formed of silica or polystyrene.

17. A kit for producing a substrate for producing a sensor for analysis, the kit comprising:
the particles according to any one of claim 1 to 16; and
a substrate.

18. The kit for producing a substrate for producing a sensor for analysis according to claim 17, further comprising a raw material of a polymer matrix.

19. Use of the particles according to any one of claims 1 to 16 for producing a substrate for producing a sensor for analysis.

20. A method for producing the particles according to any one of claims 1 to 16, the method comprising:
A) a step of providing a particle core;
B) a step of providing a modifier capable of being integrated with the core by a separable intermolecular chemical bonding; and
C) a step of subjecting the particle core and the modifier to a condition under which the particle core and the modifier are integrated by a separable intermolecular chemical bonding.

21. A method for producing a substrate for producing a sensor for analysis, the method comprising:
A) a step of providing the particles according to any one of claims 1 to 16;
B) a step of disposing the particles on a substrate; and
C) a step of forming a scaffold by subjecting the substrate to a condition under which the particles are dissociated from the substrate.

22. The method according to claim 21, further comprising a step of providing a raw material of a polymer matrix to the substrate on which the particles are immobilized.

23. The method according to claim 22, further comprising a step of forming a substrate on which the polymer matrix is disposed by subjecting the substrate to a condition under which the polymer matrix is polymerized.

24. A method for producing a sensor for analysis, the method comprising:
A) a step of providing the particles according to any one of claims 1 to 16;
B) a step of disposing the particles on a substrate;
C) a step of forming a scaffold by subjecting the substrate to a condition under which the particles are dissociated from the substrate; and
D) a step of binding a substance required for measurement to the scaffold.

25. The method according to claim 24, further comprising a step of providing a raw material of a polymer matrix to the substrate on which the particles are disposed.

26. The method according to claim 25, further comprising a step of forming a substrate on which the polymer matrix is disposed by subjecting the substrate to a condition under which the polymer matrix is polymerized.

27. The method according to any one of claims 21 to 26, wherein in the step C, the condition under which the particles are dissociated from the substrate is a condition under which a reversible linking group is cleaved and a reversible binding group is created.

28. The method according to any one of claims 21 to 27, further comprising, after the step C, a step of converting the reversible binding group in the scaffold into a group for binding a specific binding molecule to a target substance or a group for binding a signal substance.

29. A substrate for producing a sensor for analysis, the substrate comprising:
A) a substrate;
B) a polymer matrix disposed on the substrate, the polymer matrix having a scaffold that at least partially fits into a molecule to be detected; and
C) a binding group disposed on the scaffold.

30. A sensor for analysis comprising:
A) a substrate;
B) a polymer matrix disposed on the substrate, the polymer matrix having a scaffold that at least partially fits into a molecule to be detected; and
C) a group for binding a specific binding molecule that binds to a molecule to be detected.

31. The substrate according to claim 29 or the sensor according to claim 30, wherein a cleanliness of the polymer matrix is 95% or more.

32. The substrate according to claim 29 or the sensor according to claim 30, wherein a standard value of the scaffold in the substrate is 80% or more.

33. The substrate according to claim 29 or the sensor according to any one of claims 30 to 32, wherein a substituent derived from one or a plurality of modifiers integrated with the core by a separable intermolecular chemical bonding is bonded onto the substrate.

34. The substrate according to claim 29 or the sensor according to any one of claims 30 to 33, wherein the substituent derived from a modifier is an amino group, an acidic group, a coordinate binding group, a carbonyl group, an aldehyde group, a ketone group, a boronyl group, a cis-diol group, a thiol group, a biotin group, a desthiobiotin group, a peptide ligand, or a reversible linking group.

35. The substrate according to claim 29 or the sensor according to any one of claims 30 to 34, wherein the amino group is a primary to quaternary amino group, an aliphatic amino group, an aromatic amino group, a hydrazide, a guanidine, or an amidine.

36. The substrate according to claim 29 or the sensor according to any one of claims 30 to 34, wherein the acidic group is a carboxy group, a sulfonic acid group, or a phosphate group.

37. The substrate according to claim 29 or the sensor according to any one of claims 30 to 34, wherein the coordinate binding group is an NTA group or a His-tag.

38. The substrate according to claim 29 or the sensor according to any one of claims 30 to 34, wherein the peptide ligand is a glutathione group or an RGD group.

39. The substrate according to claim 29 or the sensor according to any one of claims 30 to 34, wherein the reversible linking group is a disulfide, pyridyl disulfide, imine, oxime, hydrazone, boronic acid cyclic ester, carboxylic acid ester, carboxylic acid thioester, or silyl group.

40. A sensor for analysis comprising:
A) a substrate;
B) a polymer matrix disposed on the substrate, the polymer matrix having a scaffold that at least partially fits into a molecule to be detected;
C) a substituent derived from a modifier integrated with the core by a separable intermolecular chemical bonding, the modifier being disposed on the scaffold;
D) a group for binding a signal substance or a signal substance disposed on the scaffold; and
E) a group for binding a specific binding molecule that binds to a molecule to be detected.

41. A substrate for producing a sensor for direct substrate analysis, the substrate comprising:
A) a substrate; and
B) the particles according to any one of claims 1 to 16.

42. The substrate for producing a sensor for direct substrate analysis according to claim 41, further comprising a polymer matrix on which the particles are disposed, the polymer matrix being disposed on the substrate.

43. The substrate for producing a sensor for direct substrate analysis according to claim 41 or 42, further comprising a blocking agent present on the outermost layer.

44. A sensor for direct substrate analysis, the sensor comprising:
A) a substrate; and
B) the particles according to any one of claims 1 to 16.

45. The sensor for direct substrate analysis according to claim 44, further comprising a polymer matrix on which the particles are disposed, the polymer matrix being disposed on the substrate.

46. The sensor for direct substrate analysis according to claim 44 or 45, further comprising a blocking agent present on the outermost layer.

47. The sensor for direct substrate analysis according to any one of claims 44 to 46, further comprising a group for binding a specific binding molecule and a group for binding a signal substance or a signal substance present on the particle.

48. A method for producing a substrate for producing a sensor for direct analysis, the method comprising:
A) a step of providing the particles according to any one of claims 1 to 16; and
B) a step of disposing the particles on a substrate.

49. The method according to claim 48, further comprising a step of providing a raw material of a polymer matrix to the substrate on which the particles are immobilized.

50. The method according to claim 49, further comprising a step of forming a substrate on which the polymer matrix is disposed by subjecting the substrate to a condition under which the polymer matrix is polymerized.

51. A method for producing a sensor for direct analysis, the method comprising:
A) a step of providing the particles according to any one of claims 1 to 16; and
B) a step of disposing the particles on a substrate.

52. The method according to claim 51, further comprising a step of providing a raw material of a polymer matrix to the substrate on which the particles are immobilized.

53. The method according to claim 52, further comprising a step of forming a substrate on which the polymer matrix is disposed by subjecting the substrate to a condition under which the polymer matrix is polymerized.

54. The method according to claim 53, further comprising a step of binding a substance required for measurement to the particle.

55. The particle according to claim 1, wherein the (A2) functional group-containing moiety is an acidic group, and the (B-1) first functional group is a basic group.
